(19) Europäisches Patentamt
European Patent Office
Office européen des brevets

(11) EP 2 389 356 B1

(12) **FASCICULE DE BREVET EUROPEEN**

(45) Date de publication et mention de la délivrance du brevet:
**23.10.2013 Bulletin 2013/43**

(21) Numéro de dépôt: **10707581.4**

(22) Date de dépôt: **25.01.2010**

(51) Int Cl.:
***C07C 281/06*** *(2006.01)* ***A61K 47/16*** *(2006.01)*

(86) Numéro de dépôt international:
**PCT/FR2010/050107**

(87) Numéro de publication internationale:
**WO 2010/084292 (29.07.2010 Gazette 2010/30)**

(54) **NOUVEAUX COMPOSES DE TYPE AZAPEPTIDE OU AZAPEPTIDOMIMETRIQUE, INHIBITEURS DE BCRP ET/OU P-GP**

NEUE AZAPEPTIDVERBINDUNGEN ODER AZAPEPTIDOMIMETISCHE VERBINDUNGEN, DIE BCRP- UND/ODER P-GP INHIBIEREN

NOVEL AZAPEPTIDE OR AZAPEPTIDOMIMETIC COMPOUNDS INHIBITING BCRP AND/OR P-GP

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **26.01.2009 FR 0950450**

(43) Date de publication de la demande:
**30.11.2011 Bulletin 2011/48**

(73) Titulaires:
- **Université Claude Bernard Lyon I**
  **69622 Villeurbanne Cedex (FR)**
- **Centre National de la Recherche Scientifique CNRS**
  **75794 Paris Cedex 16 (FR)**

(72) Inventeurs:
- **PARIS, Joëlle**
  **F-69003 Lyon (FR)**
- **FALSON, Pierre**
  **F-07100 Annonay (FR)**
- **DI PIETRO, Atillio**
  **F-01570 Manziat (FR)**
- **ARNAUD, Ophélie**
  **F-69006 Lyon (FR)**
- **MILLION, Marie-Emmanuelle**
  **F-38300 Cullin (FR)**

(74) Mandataire: **Sarlin, Laure V. et al**
**Cabinet Beau de Loménie**
**51, avenue Jean-Jaurès**
**BP 7073**
**69301 Lyon Cedex 07 (FR)**

(56) Documents cités:
**EP-A- 1 591 112**
**EP-A- 1 591 117**
**WO-A-2006/009765**
**WO-A-2007/148835**
**WO-A-2008/086008**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 389 356 B1

Printed by Jouve, 75001 PARIS (FR)

## Description

Le domaine de l'invention est de façon générale celui de la chimie thérapeutique, appliquée plus particulièrement à l'amélioration de l'efficacité de traitements chimiothérapeutiques, et notamment de traitements en cancérologie. Plus précisément, la présente invention a pour objet des composés de type azapeptide ou azapeptidomimétique se comportant comme des inhibiteurs de certaines protéines d'efflux telles que BCRP (de l'anglais *«breast cancer resistance protein*», également nommée ABCG2), ainsi que P-gp (de l'anglais « *Pleiotropic glycoprotein* », également nommée ABCB1).

Un problème important auquel se heurte le traitement chimiothérapeutique des cancers et des infections d'origine virale, bactérienne, fongique ou parasitaire, est la résistance intrinsèque ou acquise des cellules malignes ou des bactéries, champignons, virus ou parasites. Parmi les phénomènes de résistances acquises la résistance à de multiples drogues médicamenteuses (de l'anglais « *MultiDrug Resistance*») se traduit par une diminution de la concentration intracellulaire de l'agent chimiothérapeutique du fait de la surexpression de protéines de transport de type ABC (de l'anglais « ATP-Binding Cassette ») qui expulsent l'agent chimiothérapeutique hors des cellules cibles, tumorales ou infectées par un agent infectieux (virus, bactérie, champignon, parasite, ...). Ces protéines sont aussi exprimées de manière physiologique, notamment au niveau du tractus gastro-intestinal et de la barrière hémato-encéphalique.

La notion de résistance multiple tient au fait que les cellules deviennent résistantes non seulement aux agents chimiothérapeutiques administrés, mais également à un grand nombre de molécules structuralement non apparentées. Cette élimination survient principalement par l'action de pompes d'efflux, dont trois principales sont identifiées chez l'Homme. Ces protéines d'efflux sont ABCB1, ABCC1 et ABCG2, aussi appelées respectivement P-gp, découverte par Juliano et Ling en 1976 (Biochim Biophys Acta, 1976, 455, 152-162), MRP1 (*multidrug resistance protein 1*), découverte par Cole et Deeley 16 ans plus tard dans les cancers du poumon (Science, 1992, 258, 1650-1654) et BCRP ou MXR *(mitoxantrone resistance)* découverte plus récemment par Ross et al. d'une part (J Natl Cancer Inst, 1999, 91, 429-433) et Bates et al. d'autre part (J Cell Sci, 2000, 113 (Pt 11), 2011-2021). Ces protéines d'efflux sont insérées dans la membrane plasmique par un domaine transmembranaire lié à un Idomaine intracellulaire de grande taille. Ce dernier est constitué de deux sites de fixation de nucléotides *(Nucleotide-Binding Domain,* NBD). Un petit domaine est présent sur la face extracellulaire de ces protéines. La structure de ces protéines est probablement proche de celle des deux protéines d'efflux procaryotiques, Sav1866 (Dawson & Locher, Nature 2006 443 (7108) 180-5) et MsbA (Ward et al., , Proc. Natl. Acad. Sci. USA 2007,104 (48) 19005-10). Elles permettent l'efflux ATP-dépendant hors de la cellule d'agents chimiothérapeutiques variés.

La modulation de l'activité de ces protéines de transport peut permettre de restaurer un niveau de concentration efficace en médicament et limiter les échecs de la chimiothérapie dus au phénomène MDR. Dans ce contexte, de nombreuses études ont été réalisées pour obtenir des inhibiteurs bloquant la fonction d'efflux de ces protéines ; les agents présentant ce type d'activité sont dénommés agents chimiosensibilisateurs. Cependant, aucun n'a répondu à la fois aux critères d'efficacité, de non toxicité, de sélectivité, ce dernier visant à éviter de perturber les fonctions physiologiques des autres protéines de transport. La plupart sont aussi des inhibiteurs compétitifs, ie entrant en compétition avec le substrat au site de transport ; ils sont donc des pseudosubstrats qui du coup peuvent devenir le substrat d'un transporteur du même type, du fait de la capacité d'adaptation de ces transporteurs à différents substrats. Dans ce contexte, il est donc important de sélectionner des inhibiteurs non compétitifs.

Initialement découvertes lors de résistance aux anticancéreux, ces protéines d'efflux ont aussi récemment été associées aux développements de résistances aux traitements antiviraux. Ainsi, ABCB1 module la biodisponibilité orale des inhibiteurs de protéases et leur pénétration dans le système nerveux central (Kim, Top HIV Med, 2003, 11, 136-139) ; de même ABCG2 efflue la zidovudine/AZT, la lamivudine/Epivir et l'abacavir/Ziagen (Wang et al., Mol. Pharmacol. 2003, 63, 65-72 ; Weiss et al., J. Antimicrob. Chemother., 2007 dkl474.). Leur spectre de transport est très large, ce qui fait qu'elles sont capables d'effluer un grand nombre de composés, de structure chimique variée, et utilisés par exemple pour traiter différentes pathologies, dont le cancer.

Ces protéines d'efflux sont naturellement exprimées dans des tissus normaux afin d'expulser les toxines et les déchets produits par les cellules (*e.g.*, poumon, rein et foie). Elles sont aussi produites au niveau de la barrière hémato encéphalique pour empêcher les toxines de pénétrer dans le cerveau. Une des stratégies potentielles pour inverser le phénotype MDR consiste à co-administrer l'agent chimiothérapeutique et un inhibiteur des protéines d'efflux. Plusieurs molécules ont été développées dans ce but. Les trois générations d'inhibiteurs développés pour bloquer l'activité d'efflux de ces protéines sont présentées dans le **TABLEAU 1**. ,

**TABLEAU 1a et 1b**. les symboles indiquent soit que l'inhibiteur est actif (+) soit qu'il est inactif (-) sur un transporteur spécifié, ou que l'effet n'est pas connu (?). Tiré de Current Opinion in Pharmacology, 2006, 6, 350-354.

Tableau 1a

| Transporteur | Seconde génération d'inhibiteurs | |
|---|---|---|
| | PSC833 (valspodar) | VX-710 (biricodar) |
| ABCB1 | + | + |
| ABCC1 | + | + |
| ABCG2 | - | + |

Tableau 1b

| Transporteur | Troisième génération d'inhibiteurs | | | | |
|---|---|---|---|---|---|
| | GF 120918 (elacridar) | R101933 (laniquidar) | XR9576 (tariquidar) | Ly335979 (zosuquidar) | ONT-093 (ontogen) |
| ABCB1 | + | + | + | + | + |
| ABCC1 | - | ? | - | - | - |
| ABCG2 | + | ? | + | - | ? |

Certains inhibiteurs, comme le tariquidar, interagissent avec plusieurs protéines. Notamment, ils inhibent de façon significative, en plus de ABCB1 et/ou ABCG2, ABCC1. Ceci n'est pas nécessairement un avantage puisque, idéalement, un agent réversant ne devrait cibler qu'une seule protéine d'efflux, afin de préserver les fonctions physiologiques des autres dans les cellules saines. Par ailleurs, d'autres agents, comme le zosuquidar, ont été récemment stoppés en phase III à cause d'effets indésirables. Ces exemples démontrent qu'il est difficile de trouver de nouveaux types d'agents réversibles, à la fois puissants, spécifiques et ayant un minimum d'effets indésirables.

WO 2008/086008 décrit un composé de type Amonafide pour utilisation comme adjuvant d'un médicament anticancereux.

La présente invention se propose de fournir de nouveaux composés du type azapeptides ou azapeptidomimétiques qui se comportent comme des inhibiteurs d'ABCG2 et/ou d'ABCB1. De plus, un autre objectif de l'invention est de fournir des inhibiteurs d'ABCG2 et/ou d'ABCB1 qui ne présentent pas une inhibition significative de ABCC1. Certains des composés selon l'invention présentent également une grande affinité et, fréquemment, une sélectivité pour ABCG2 ou ABCB1 et/ou se comportent comme des inhibiteurs non compétitifs.

Un autre objectif de l'invention est de proposer de nouveaux composés qui soient non cytotoxiques.

Un autre objectif de l'invention est de proposer de nouveaux composés qui soient relativement faciles à préparer et d'un prix de revient raisonnable.

Dans ce contexte, la présente invention a pour objet les composés de type azapeptide ou azapeptidomimétique de formule (I) :

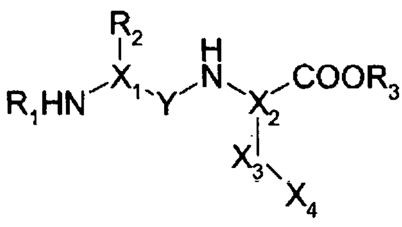

(I)

dans laquelle :

- $R_1$ représente un groupe protecteur d'un groupement amino, de préférence un groupe- C (O) OR'$_1$ avec R'$_1$ qui représente un groupe alkyle de 1 à 12 atomes de carbone ou un groupe- $(CH_2)_{m1}R''_1$ avec $R''_1$ qui représente un groupe aryle (par exemple phényle), cycloalkyle ou fluorényle et m1 qui est égal à 0, 1, 2 ou 3,
- $X_1$ et $X_2$ sont identiques ou différents et représente -N- ou -CH-, étant entendu que l'un au moins représente -N-,
- $R_2$ représente une chaine latérale éventuellement sous forme protégée d'un acide aminé ou d'un analogue d'acide

aminé, ou un groupe aryle (par exemple phényle) éventuellement substitué ou un groupe hétéroaryle éventuellement substitué,

- Y représente $-CH_2-$ ou -C(O)-,
- - $X_3$- $X_4$ représente soit un groupe- $(CH_2)_n$- $NHR_4$ avec n égal à 3, 4, 5 ou 6, soit un groupe choisi parmi :

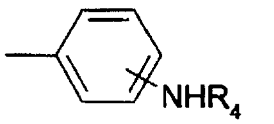 et 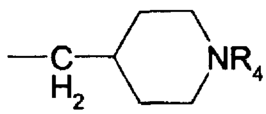

avec $R_4$ qui représente un groupe protecteur d'un groupement amino, de préférence un groupe- C (O) $OR'_4$ avec $R'_4$ qui représente un groupe alkyle de 1 à 12 atomes de carbone ou un groupe- $(CH_2)_{m4}R''_4$ avec $R''_4$ qui représente un groupe aryle (par exemple phényle) ou cycloalkyle et m4 qui est égal à 0, 1, 2 ou 3,

- $R_3$ représente un groupe alkyle de 1 à 12 atomes de carbone, ou un groupe- $(CH_2)_{m3}R''_3$ avec $R''_3$ qui représente un groupe cycloalkyle ou aryle (par exemple phényle), les dits groupes cycloalkyle et aryle pouvant être non-substitués ou substitués par un ou plusieurs groupes choisis parmi :- $CH_3$, $-CF_3$, -COOH, $-NO_2$, -Cl et $NH_2$ et m3 qui est égal à 0, 1, 2 ou 3,

sous la forme d'isomère optique pur ou de mélange d'isomères optiques, ainsi que leurs sels, solvats ou hydrates.

Selon une variante de réalisation, les composés de formule (I) tels que précédemment définis ont comme substituant $R_2$ un groupe- L- $COOR'_2$ avec L qui représente un groupe aryle, de préférence phényle, ou une chaine- $(CH_2)$ $m_2$- avec $m_2$ qui est égal à 1, 2 ou 3 et $R'_2$ qui représente un groupe alkyle de 1 à 12 atomes de carbone ou un groupe- $(CH_2)_{m'2}R''_2$ avec $R''_2$ qui représente un groupe aryle (par exemple phényle) ou cycloalkyle, éventuellement substitué et m'2 qui est égal à 0, 1, 2 ou 3.

Un sous groupe des composés selon l'invention est composé des composés de formule (IA), sous la forme d'isomère pur ou de mélange d'isomères, ainsi que leurs sels, solvats ou hydrates :

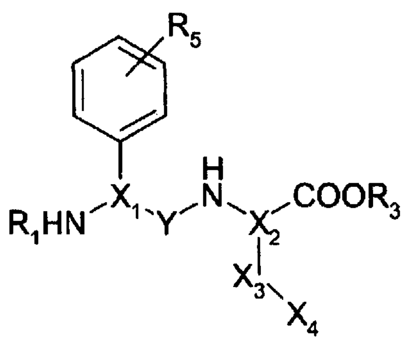

(IA)

dans laquelle $R_5$ représente un groupe- $NH_2$, $-NO_2$, -OH, -O- alkyle de 1 à 8 atomes de carbone, O- $(CH_2)_{m5}R'_5$ avec $R'_5$ qui représente un groupe aryle (par exemple phényle) et m5 qui est égal à 0 ou 1 ou bien $R_5$ représente un groupe- $COOR''_5$ avec $R''_5$ qui représente un groupe alkyle de 1 à 12 atomes de carbone ou un groupe- $(CH_2)_{m'5}R'''_5$ avec $R'''_5$ qui représente un groupe aryle (par exemple phényle) ou cycloalkyl et m'5 qui est égal à 0, 1, 2 ou 3, $R_1$, $X_1$, Y, $X_2$, $X_3$, $X_4$ et $R_3$ étant tels que définis pour (I) .

De façon avantageuse, les composés de formule (IA) présentent l'une ou l'autre des caractéristiques ci-dessous ou une combinaison de certaines ou de toutes ces caractéristiques, lorsqu'elles ne s'excluent pas l'une l'autre :

- $R_5$ représente un groupe- $COOR''_5$ avec $R''_5$ qui représente un groupe éthyle, benzyle ou- CH $[CH(CH_3)_2]_2$,
- $R_5$ est positionné en méta, dans ce cas on privilégie la sélectivité vis-à-vis de BCRP,
- le groupe $R_5$ est positionné en para, dans ce cas on privilégie la sélectivité vis-à-vis de P-gp,
- $X_1$ représente -N- et $X_2$ représente -CH-,
- $X_1$ et $X_2$ représentent N,
- Y représente -C(O)-,
- - $X_3$- $X_4$ représente un groupe- $(CH_2)_n$- $NHR_4$ avec n égal à 4 ou 6 et $R_4$ tel que défini pour (I) .

Un sous groupe des composés selon l'invention est composé des composés de formule (IB), sous la forme d'isomère pur ou de mélange d'isomères, ainsi que leurs sels, solvats ou hydrates :

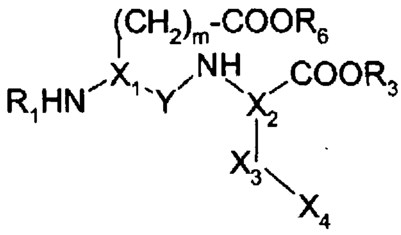

(IB)

dans laquelle m est égal à 1, 2 ou 3, $R_6$ représente un groupe alkyle de 1 à 12 atomes de carbone, ou un groupe-$(CH_2)_{m6}R'_6$ avec $R'_6$ qui représente un groupe cycloalkyle ou aryle (par exemple phényle), les dits groupes cycloalkyle et aryle (par exemple phényle) pouvant être non- substitués ou substitués par un ou plusieurs groupes choisis parmi :-$CH_3$, -$CF_3$, -COOH, -$NO_2$, -Cl et $NH_2$ et m6 qui est égal à 0, 1, 2 ou 3, $R_1$, $X_1$, Y, $X_2$, $X_3$, $X_4$ et $R_3$ étant tels que définis pour (I).

De façon avantageuse, les composés de formule (IB) présentent l'une ou l'autre des caractéristiques ci-dessous ou une combinaison de certaines ou de toutes ces caractéristiques, lorsqu'elles ne s'excluent pas l'une l'autre :

- m est égal à 1 ou 2,
- $R_6$ représente un groupe éthyle, benzyle, ou- CH [CH $(CH_3)_2]_2$,
- $X_1$ représente -CH- et $X_2$ représente -N-,
- $X_1$ représente -N- et $X_2$ représente -CH-,
- - $X_3$- $X_4$ représente un groupe- $(CH_2)_n$- $NHR_4$ avec n égal à 6 et $R_4$ tel que défini pour (I).

Sont également préférés, les composés de formule (I), (IA) ou (IB) qui présentent une, deux ou trois des caractéristiques suivantes :

- $R_1$ représente un groupe -COOR'$_1$ et R'$_1$ représente un groupe *tert*-butyle ou benzyle,
- $R_3$ représente un groupe tert-butyle ou benzyle,
- $R_4$ représente- C (O) OR'$_4$ avec R'$_4$ qui représente un groupe benzyle ou *tert*- butyle.

A titre d'exemples spécifiques des composés selon l'invention, on peut citer les composés de type azapeptide ou azapeptidomimétique choisis parmi :

- Ester *tert*- butylique de l'acide (2S)- 6- (benzyloxycarbonylamino)- 2- [1- (2'- (benzyloxycarbonyl) phényl)- 2- (*tert*-butoxycarbonyl)- hydrazinocarbonylamino]- hexanoïque (IA. 1)
- Ester *tert*- butylique de l'acide (2S)- 6- (benzyloxycarbonylamino)- 2- [1- (3'- (benzyloxycarbonyl) phényl)- 2- (*tert*-butoxycarbonyl)- hydrazinocarbonylamino]- hexanoïque (IA. 2)
- Ester *tert*- butylique de l'acide (2S)- 6- (benzyloxycarbonylamino)- 2- [1- (4'- (benzyloxycarbonyl) phényl)- 2- (*tert*-butoxycarbonyl)- hydrazinocarbonylamino]- hexanoïque (IA. 3)
- Ester benzylique de l'acide (2R)- 6- (benzyloxycarbonylamino)- 2- [1- (3'- (benzyloxycarbonyl) phényl)- 2- ( *tert*-butoxycarbonyl)- hydrazinocarbonylamino]- hexanoïque (IA. 4)
- Ester benzylique de l'acide (2S)- 6- (benzyloxycarbonylamino)- 2- [1- (3'- (benzyloxycarbonyl) phényl)- 2- (*tert*-butoxycarbonyl)- hydrazinocarbonylamino]- hexanoïque (IA. 5)
- Ester tert- butylique de l'acide (2S)- 6- (benzyloxycarbonylamino)- 2- [1- (3'- (phényloxycarbonyl) phényl)- 2- (*tert*-butoxycarbonyl)- hydrazinocarbonylamino]- hexanoïque (I A. 6)
- Ester benzylique de l'acide 2- [1- (3'- (benzyloxycarbonyl) phényl)- 2- (*tert*- butoxycarbonyl)- hydrazinocarbonyl]- 1-(6- benzyloxycarbonylamino- hexyl)- hydrazinocarboxylique (IA. 7)
- Ester benzylique de l'acide 2- [1- (3'- (benzyloxycarbonyl) phényl)- 2- (*tert*- butoxycarbonyl)- hydrazinocarbonyl]- 1-(6- *tert*- butyloxycarbonylamino- hexyl)- hydrazinocarboxylique (IA. 8)
- Ester benzylique de l'acide (3S)- 4- [2- (6- benzyloxycarbonylamino- hexyl)- 2- *tert*- butoxycarbonyl- hydrazino]- 3-*tert*- butoxycarbonylamino- butyrique (IB. 1)
- Ester benzylique de l'acide (3S)- 4- [2- (3- benzyloxycarbonylamino- propyl)- 2- *tert*- butoxycarbonyl- hydrazino]- 3-*tert*- butoxycarbonylamino- 4- oxo- butyrique (IB. 2)
- Ester benzylique de l'acide (3S)- 4- [2- (4- benzyloxycarbonylamino- phényl)- 2- *tert*- butoxycarbonyl- hydrazino]-3- *tert*- butoxycarbonylamino- 4- oxo- butyrique (IB. 3)
- Ester benzylique de l'acide (4S)- 5- [2- (4- benzyloxycarbonylamino- phényl)- 2- *tert*- butoxycarbonyl- hydrazino]-4- *tert*- butoxycarbonylamino- 5- oxo- pentanoïque (IB. 4)
- Ester benzylique de l'acide (3S)- 4- [2- (5- benzyloxycarbonylamino- pentyl)- 2- *tert*- butoxycarbonyl- hydrazino]- 3-*tert*- butoxycarbonylamino- 4- oxo- butyrique (IB. 5)

- Ester benzylique de l'acide 4- [2- ((2S)- 3- benzyloxycarbonyl- 2- *tert*- butoxycarbonylamino- propyl)- 1- *tert*- butoxycarbonyl- hydrazinométhyl]- pipéridine- 1- carboxylique (IB. 6)
- Ester benzylique de l'acide 4- [2- ((2S)- 3- benzyloxycarbonyl- 2- *tert*- butoxycarbonylamino- propionyl)- 1- *tert*-butoxycarbonyl- hydrazinométhyl]- pipéridine- 1- carboxylique (IB. 7)
- Ester benzylique de l'acide (3S)- 4- [2- (5- benzyloxycarbonylamino- pentyl)- 2- *tert*- butoxycarbonyl- hydrazino]- 3-*tert*- butoxycarbonylamino- butyrique (IB. 8)
- Ester benzylique de l'acide (3S)- 4- [2- (6- benzyloxycarbonylamino- hexyl)- 2- *tert*- butoxycarbonyl- hydrazino]- 3-*tert*- butoxycarbonylamino- 4- oxo- butyrique (IB. 9)
- Ester benzylique de l'acide (3S)- 3- *tert*- butoxycarbonylamino- 4- [2- *tert*- butoxycarbonyl- 2- (6- *tert*- butoxycarbonylamino- hexyl)- hydrazino]- butyrique (IB. 10)
- Ester benzylique de l'acide (3S)- 4- [2- benzyloxycarbonyl- 2- (6- benzyloxycarbonylamino- hexyl)- hydrazino]- 3-*tert*- butoxycarbonylaminobutyrique (IB. 11)
- Ester benzylique de l'acide (3S)- 3- benzyloxycarbonylamino- 4- [2- benzyloxycarbonyl- 2- (6- benzyloxycarbonylamino- hexyl)- hydrazino]- butyrique (IB. 12)
- Ester benzylique de l'acide (3S)- 4- [2- benzyloxycarbonyl- 2- (6- *tert*- butoxycarbonylamino- hexyl)- hydrazino]- 3-*tert*- butoxycarbonylaminobutyrique (IB. 13)
- Ester *tert*- butylique de l'acide (2S)- 6- (benzyloxycarbonylamino)- 2- [1- (benzyloxycarbonylméthyl)- 2- (*tert*- butoxycarbonyl)- hydrazinocarbonylamino]- hexanoïque (IB. 14),

ainsi que leurs sels, solvats et/ou hydrates, et notamment ceux pharmaceutiquement acceptables.

Les composés selon l'invention, de type azadipeptide ou azapeptidomimétique synthétique, présentent des propriétés inhibitrices de BCRP/ABCG2 et/ou de la P-gp/ABCB1. Les composés selon l'invention présentent une valeur faible de $IC_{50}$ pouvant atteindre 2 $\mu$M, voire 0,3 $\mu$M ou moins, vis-à-vis de l'une au moins de ces deux protéines. L'$IC_{50}$ est définie comme la demi concentration de composé bloquant 50% de l'efflux d'agent thérapeutique. Les tests utilisés pour déterminer l'activité des composés sont décrits, en détails, dans les exemples dans la partie « activité biologique ». Les composés selon l'invention ne présentent pas une inhibition significative de ABCC1. Notamment, la plupart des composés selon l'invention présentent un % d'efficacité à 10 $\mu$M, vis-à-vis de cette protéine, inférieure à 15%. Certains composés, comme les composés (IA.2) et (IA.3), se sont également montrés sélectifs vis-à-vis de l'une au l'autre des protéines BCRP ou P-gp. Certains tels que les composés (IA.2) et (IB.11) se sont également montrés non compétitifs.

Les composés selon l'invention sont des azadipeptides ou azapeptidomimétiques hydrophobes dans lesquels un atome d'azote a été introduit à la place du $CH\alpha$ , soit dans l'acide aminé *N*-terminal (N-aza), soit dans l'acide aminé *C*-terminal (C-aza), dans le but, d'une part d'augmenter leur stabilité et biodisponibilité, et d'autre part de favoriser/stabiliser la conformation active de la molécule la plus efficace. Les composés selon l'invention permettent de bloquer la fonction d'efflux de transporteurs ABC qui se trouve amplifiée lors des traitements chimiothérapeutiques. Les ABC modulent, *via* leur transport/efflux/rejet, la pharmacocinétique des médicaments, qu'ils soient anticancéreux, antiviraux, antibactériens, antifongiques, antiparasitaires, ou plus largement utilisés dans le cadre d'une pathologie donnée.

Un autre objet de l'invention est donc relatif aux composés précédemment définis en tant qu'adjuvant d'un traitement chimiothérapeutique administré à un patient, pour lequel l'organisme du patient traité développe une résistance, destiné à restaurer l'activité du traitement thérapeutique. Les composés selon l'invention peuvent, notamment, être utilisés en tant qu'adjuvant d'un médicament anti-cancéreux ou anti-infectieux.

De façon préférée, les composés selon l'invention peuvent être définis comme des adjuvants :

- d'un médicament choisi parmi les anthracyclines, les inhibiteurs de topoisomérase, les antimétaboliques (antifolates), les inhibiteurs de tyrosine kinase, les antiviraux (inhibiteurs de reverse transcriptase), les antiparasitaires, les antifongiques,
- ou d'un médicament choisi parmi la daunorubicine, la doxorubicine, la mitoxantrone, la camptotécine et ses dérivés, l'irinotécan, le topotécan, les indolocarbazoles, le méthotrexate, l'imatinib, le gefitinib, la zidovudine, la lamivudine, l'abacavir, l'ivermectine, l'albendazole, l'oxfendazole, le kétoconazole.

Selon un autre de ses aspects, la présente invention concerne l'utilisation d'un composé de formule (I), (IA), (IB), tel que défini ci-dessus, éventuellement sous forme hydratée ou solvatée, ou sous la forme d'un sel pharmaceutiquement acceptable, pour la fabrication d'un médicament destiné à améliorer l'effet d'un traitement chimiothérapeutique, notamment en cancérologie.

L'invention concerne un médicament comprenant séparément ou ensemble (i) un composé selon l'invention et (ii) un agent chimiothérapeutique efficace contre le cancer ou l'affection que l'on souhaite traiter. Selon un mode de réalisation particulier de l'invention, le médicament se présente sous la forme d'une composition pharmaceutique unique combinant, dans une même formulation, (i) le composé selon l'invention et (ii) l'agent chimiothérapeutique efficace contre le cancer ou l'affection que l'on souhaite traiter.

Aussi, l'invention a également pour objet les compositions pharmaceutiques comprenant un composé de formule (I), (IA) ou (IB) tels que définis précédemment, éventuellement sous la forme d'un sel, solvat ou hydrate pharmaceutiquement acceptable, destinées au traitement d'un cancer ou d'un traitement anti- infectieux, en association avec un agent chimiothérapeutique efficace contre le cancer ou l'affection que l'on souhaite traiter.

La description ci-après permet de mieux comprendre l'invention. En préliminaire, un certain nombre de définitions est rappelé.

Par groupe alkyle, on entend une chaîne hydrocarbonée, saturée, linéaire ou ramifiée. A titre d'exemple de groupe alkyle comprenant de 1 à 12 atomes de carbone et de préférence de 1 à 7 atomes de carbone, on peut citer les groupes méthyle, éthyle, n- propyle, iso- propyle, n- butyle, *tert*- butyle, sec- butyle, n- pentyle, n- hexyle, n- heptyle, $-CH [CH (CH_3)_2]_2$.

Par groupe cycloalkyle, on désigne une chaîne hydrocarbonée saturée, cyclique, comprenant de 3 à 7 atomes de carbone. A titre d'exemple de groupe cycloalkyle, on peut citer les groupes cyclopropyle, cyclobutyle, cyclopentyle, cyclohexyle et cycloheptyle.

Par groupe aryle, on entend un carbocycle mono-, bi- ou polycyclique, comportant de préférence de 6 à 12 atomes de carbone, comprenant au moins un groupe aromatique, par exemple, un radical phényle, cinnamyle ou naphtyle. Le phényle est le groupe aryle particulièrement préféré.

Par groupe hétéroaryle, on entend un groupe aryle interrompu par un ou plusieurs hétéroatomes choisis parmi les atomes d'azote, oxygène et soufre, par exemple choisis parmi les groupes pyridyle, furyle, thiényle, isoxazolyle, oxadiazolyle, oxazolyle, benzimidazole, indolyle, benzofurane.

Par groupe aryle ou hétéroaryle substitué, on entend, par exemple, un tel groupe mono ou poly- substitué, notamment di- substitué, par un atome d'halogène, un groupe- $CF_3$, $-NH_2$, -OH, -COOH, un groupe alkyle ayant de 1 à 12 atomes de carbone, un groupe O- alkyle ayant de 1 à 8 atomes de carbone, un groupe- $(CH_2)_pR'$ ou- O- $(CH_2)_pR'$ avec R' qui représente un groupe aryle ou cycloalkyle et p qui est égal à 0 ou 1, une fonction nitro, un groupe- COOR avec R qui représente un groupe alkyle de 1 à 12 atomes de carbone ou un groupe- $(CH_2)_pR'$ avec R' qui représente un groupe aryle ou cycloalkyle et p qui est égal à 0, 1, 2 ou 3.

Le terme « amino acide » correspond aux composés organiques ayant la structure suivante.

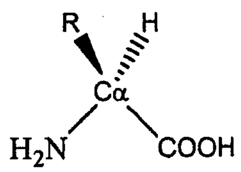

L-α- amino acide

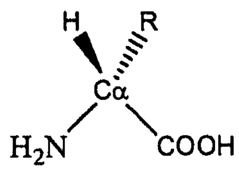

D-α-amino acide

Par chaine latérale des amino acides, on entend donc, notamment, le groupement R des aminoacides suivants : glycine, thréonine, sérine, acide aspartique, asparagine, cystéine, alanine, proline, valine, isoleucine, histidine, leucine, méthionine, phénylalanine, acide glutamique, glutamine, lysine, arginine, tryptophane, tyrosine. Les fonctions acide, amine ou alcool, notamment, seront de référence sous forme protégée. Les groupes protecteurs des chaines latérales des amino acides sont bien connus de l'Homme du métier et sont notamment décrits dans la publication de Chem. Rev. 2009, 109, 2455-2504.

D'une manière générale, les groupes protecteurs d'un groupement amino sont bien connus de l'Homme du métier et sont notamment décrits dans cette même publication, ou encore dans Protective Groups in Organic Synthesis, Greene T.W. et Wuts P.G.M., ed John Wiley et Sons, 2006 et dans Protecting Groups, Kocienski P.J., 1994, Georg Thieme Verlag. A titre d'exemple de tels groupements, on peut citer les groupes Boc (*tert*- butyloxycarbonyle), Fmoc (9- fluorénylméthoxycarbonyl) et Z benzyloxycarbonyl) . La présence d'un tel groupement au niveau du substituant R1 permet d'augmenter le caractère hydrophobe de la molécule.

Les analogues d'acides aminés utilisés dans la présente invention inclut, sans être limités, l'acide homo glutamique et l'homosérine.

Par azapeptide ou azapeptidomimétique, on entend un peptide ou un peptidomimétique dans lequel le CHα est remplacé par un N. Le terme peptidomimétique signifie le remplacement de la liaison peptidique, c'est-à-dire le lien entre deux acides aminés formé par élimination d'eau entre le groupement amine d'un acide aminé et le groupement acide carboxylique de l'autre acide aminé, par une liaison isostère, par exemple méthyle-amino $-CH_2-NH-$. Le terme « cancer » désigne toute condition pathologique qui est typiquement caractérisée par une croissance déréglée des cellules. A titre d'exemple de cancer, on peut citer les carcinomes, lymphomes, blastomes, sarcomes et leucémies, et plus précisément le cancer pulmonaire épidermoïde, l'adénocarcinome du colon, le mésothéliome, le gliome, l'adénocarcinome du sein, le mélanome, le cancer du rein à cellules claires, le cancer de la prostate, l'hépatocarcinome et le

myélome multiple.

Le terme «traitement» désigne toute mesure thérapeutique prophylactique ou suppressive d'une maladie ou désordre conduisant à un effet clinique souhaitable ou à tout effet bénéfique, incluant notamment la suppression ou la diminution de un ou plusieurs symptômes, la régression, le ralentissement ou la cessation de la progression du cancer ou du désordre qui y est associé.

Par « quantité thérapeutiquement efficace », on désigne toute quantité d'une composition qui améliore un ou plusieurs des paramètres caractéristiques du cancer.

La synthèse des composés de formule (I) est détaillée ci-après.

Les composés de formule (I) dans lesquels $X_1$=N et Y=C(O) sont obtenus par réaction de couplage entre les composés de formule (II) et les composés de formule (III) comme présenté par le **SCHEMA 1**, dans lequel $R_2$, $R_3$, $X_2$, $X_3$ et $X_4$ sont tels que définis pour (I) et $R_{1P}$ représente soit directement le groupe $R_1$ dans le cas où $R_2$ est un groupe aryle ou hétéroaryle éventuellement substitué, soit un atome d'hydrogène dans le cas où $R_2$ représente une chaine latérale éventuellement sous forme protégée d'un acide aminé ou d'un analogue d'acide aminé.

**SCHEMA 1**

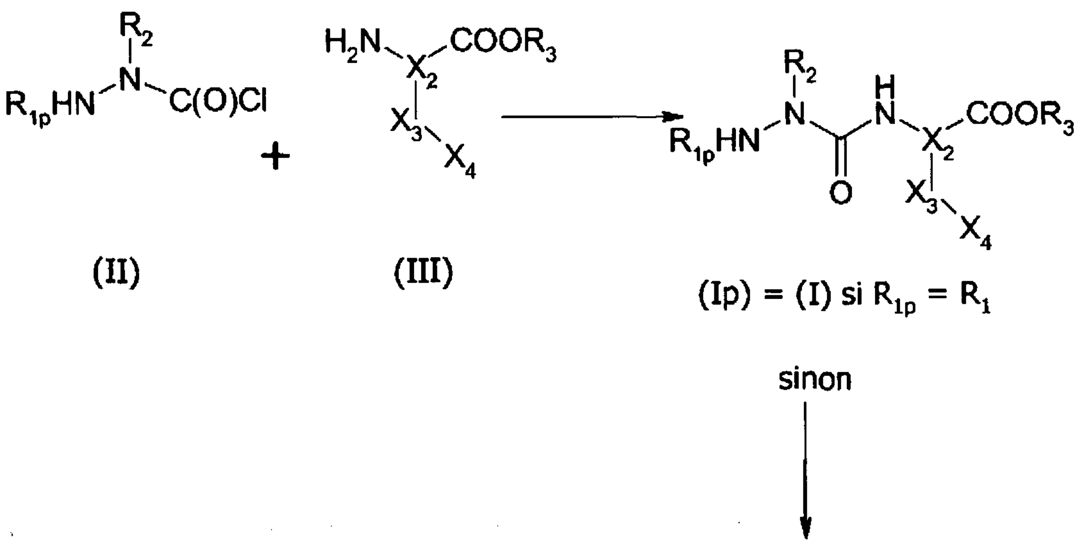


(II) (III) (Ip) = (I) si $R_{1p}$ = $R_1$

(I) avec $X_1$ = N et Y = C(O)

Le chlorure d'acide (II) est généré *in situ* par action de triphosgéne et est couplé, selon une méthode décrite dans la littérature (Journal of Peptide Science, 1997, 3, 429-441), avec un composé (III) qui correspond soit, dans le cas où $X_2$=CH, à un acide aminé D ou L dont la fonction acide (-$COOR_3$) et la fonction amine éventuellement présente sur la chaine latérale (-$X_3$-$X_4$) sont protégées, soit, dans le cas où $X_2$=$_N$, à un aza amino acide dont la fonction acide (-$COOR_3$) et la fonction amine éventuellement présente sur la chaine latérale (-$X_3$-$X_4$) sont protégées.

Les composés de formule (II), dans le cas où $R_2$ est un groupe aryle ou hétéroaryle éventuellement substitué, sont préparés selon des techniques bien connues de l'Homme de l'art.

Le **SCHEMA 2** illustre leur synthèse dans le cas de la préparation des composés de formule (IA), lorsque $R_2$ est un phényle substitué par un groupe -COOR"$_5$. Dans le **SCHEMA 2** $R_1$ et $R_5$ sont tels que définis pour (I) et $R_{5p}$ représente $R_5$ ou un groupe précurseur de $R_5$, par exemple -COOH.

**SCHEMA 2**

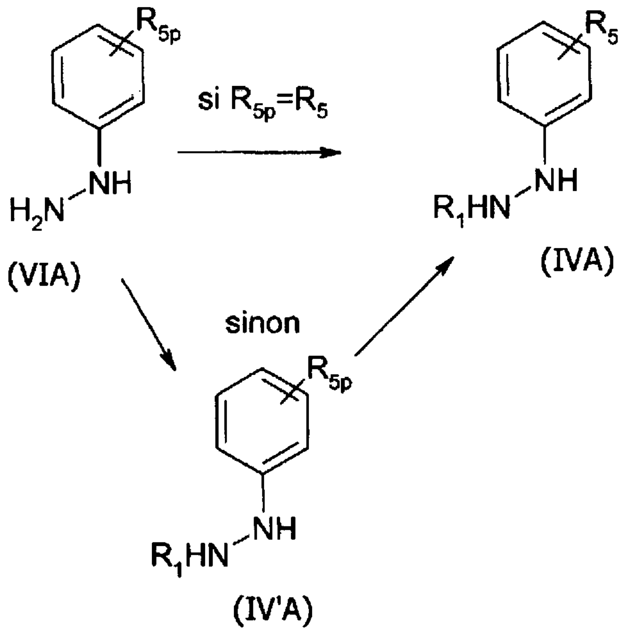


(VIA) (IVA) (IV'A)

On procède tout d'abord à une acylation régiosélective de l'acide hydrazinobenzoïque (VIA) (J Med Chem, 1996, 39, 1172-1188). En fonction de la nature du groupement $R_5$, et notamment dans le cas où $R_5$ = -COOR"$_5$ (avec R"$_5$ tel que défini pour les composés de formule (IA)), on formera intermédiairement un composé (IV'A) dans lequel $R_{5p}$ = -COOH qui sera ensuite estérifié en -COOR"$_5$, par exemple par la méthode de Wang (J. Org. Chem. 1977, 42, 1286-1290). Le chlorure d'acide (II) est ensuite généré *in situ* par action de triphosgène, avant couplage avec le composé (III).

Les composés de formule (II), dans le cas où $R_2$ représente une chaine latérale, éventuellement sous forme protégée, d'un acide aminé ou d'un analogue d'acide aminé, sont également préparés selon des techniques bien connues de l'Homme de l'art à partir des composés de formule (IVB), illustrés dans le cas où $R_2$ représente un groupe- $(CH_2)_m$-$COOR_6$, avec m et $R_6$ tels que définis pour les composés de formule (IB) :

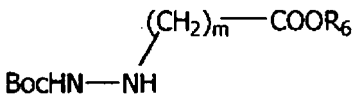

(IVB)

**Le SCHEMA 3** illustre leur synthèse dans le cas de la préparation des composés de formule (IB), lorsque $R_2$ est un -$CH_2COOBn$.

**SCHEMA 3**

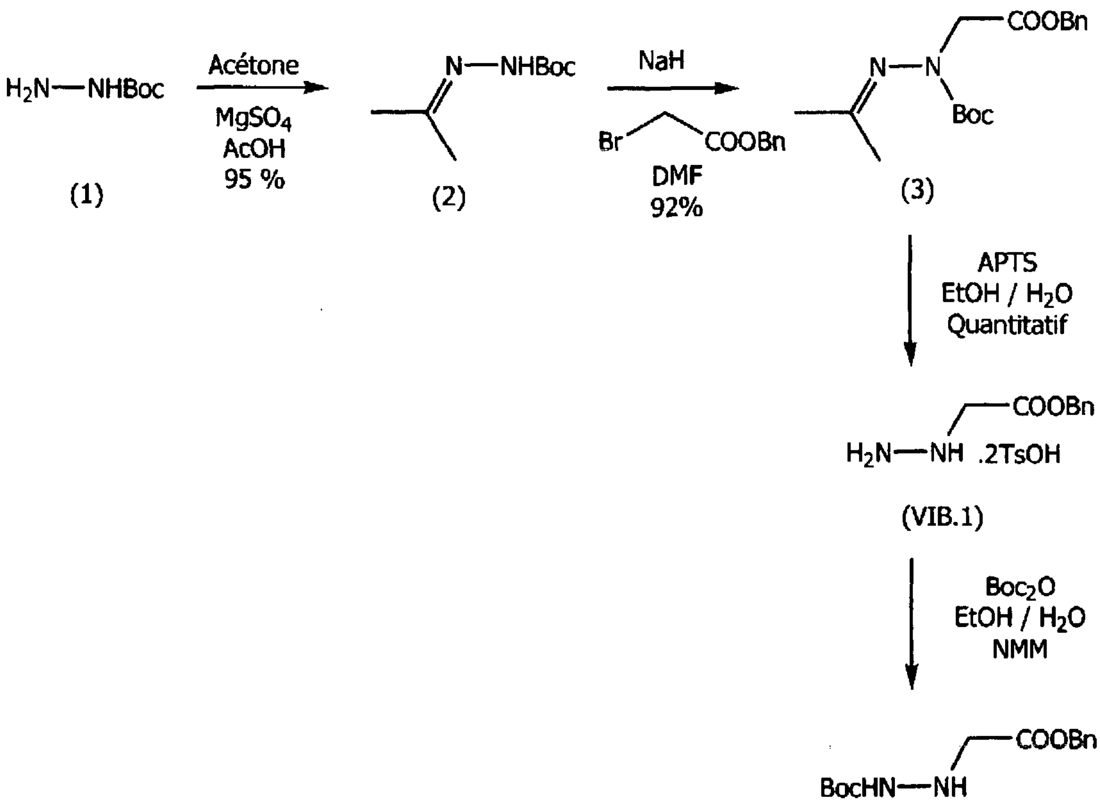


(1) (2) (3) (VIB.1) (IVB.1)

Les Nβ Boc hydrazino acétates (IVB. 1) sont préparés à partir de la Boc hydrazine (1) . Dans un premier temps, l'amine primaire de la Boc- hydrazine (1) est protégée par l'acétone pour former l'imine (2) . Puis, selon une méthode adaptée de celle de Meyer (K. Meyer Synlett, 2004, 2355- 2356), une réaction d'alkylation à l'aide d'un bromo acétate conduit au composé alkylé (3) . Afin de déprotéger simultanément les deux amines, l'acide paratoluène sulfonique est avantageusement utilisé pour conduire au composé (VIB. 1) . La protection de l'amine primaire pour donner (IVB. 1) puis la transformation en chlorure d'acide et le couplage avec les composés (III) sont réalisés selon la même méthode que dans le cas des composés (IVA) .

Les composés de formule (III), quant à eux, peuvent être préparés, notamment :

- soit *via* une réaction de Buchwald (Org. Lett., 2001, 3, 3803-3805) entre une iodo aniline convenablement protégée (5) obtenue à partir de la iodoaniline (4), selon une methode adaptée de celle de Pati (Synthetic communications, 2004, 34, 933-40), et de la Boc-hydrazine, pour conduire au composé (III) correspondant. Le **SCHEMA 4** ci-après illustre ce mode de préparation dans le cas du composé (III.1) où $X_2$=N, $R_3$ = *tert*-butyl
  et

$X_3$-$X_4$ = 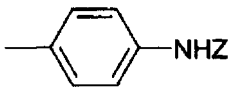 .

**SCHEMA 4**

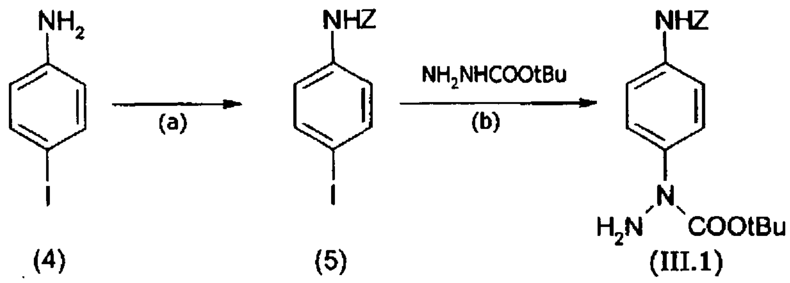


(4) (5) (III.1)

(a) chloroformiate de benzyle, en présence de triéthylamine

(b) CuI 1% mol, 1,10-phénanthroline 10% mol Cs2CO3, DMF, sous argon, 80°C, 21h, 10%

- soit *via* une réaction de Mitsunobu entre des hydrazines protégées en β par un groupement phtaloyl et en α par un groupement carbamate et des amino alcools protégés, par les groupements préférés, sur la fonction amine selon un procédé décrit dans la littérature (Tetrahedron, 1989, 45, 6319- 6330 et J Org Chem, 2001, 66, 2869- 2873) . Les amino alcools protégés sur la fonction amine sont préparés selon la méthode décrite dans la littérature (Tetrahedron 1989, 45, 6319- 6330 et Tetrahedron Asymmetry 2003, 14, 139- 143) . Le **SCHEMA 5** ci- après illustre ce mode de préparation dans le cas où $X_2$=N, -$COOR_3$=Boc et $X_3$- $X_4$ =- $(CH_2)_6NHZ$.

**SCHEMA 5**

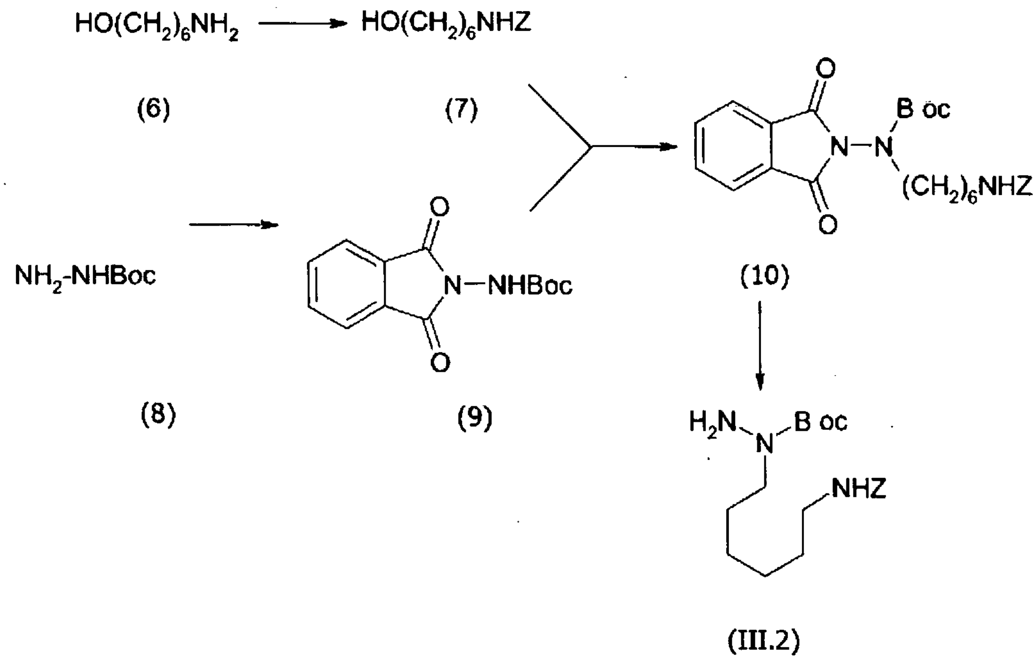

(6) (7) (10) (8) (9) (III.2)

L'amine (6) est protégée en aminoalcool (7). En parallèle, la Boc-hydrazine (8) est protégée par un groupement phtaloyl pour conduire au composé (9). La réaction de Mitsunobu entre l'hydrazine protégée (9) et l'aminoalcool (7) est suivie de la déprotection de l'aza-aminoacide pour conduire au composé (III.2).

Les composés de formule (I) dans lesquels $X_1$=CH, $X_2$=N et Y=$CH_2$ sont préparés *via* une amination réductrice selon la méthode de Martinez *et al.* (J. Med. Chem. **1985**, *28*, 273-278) entre un amino aldéhyde (V) convenablement protégé, et un composé (III) tel que précédemment décrit, comme illustré sur le **SCHEMA 6** ci-après.

**SCHEMA 6** dans lequel $R_1$, $R_2$, $R_3$, $X_3$ et $X_4$ sont tels que définis pour (I)

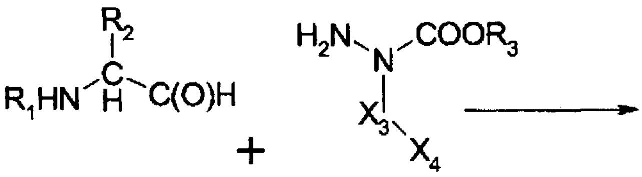

(I) avec $X_1$ = CH, $X_2$ = N et Y = $CH_2$

(V) (III)

avec $X_2$ = N

L'amino aldéhyde (V) peut être préparé selon la méthode de Z. Guo et al. (Bioorg. Med. Chem. 2001, 9, 99-106) . Le **SCHEMA 7** illustre la préparation de ces composés dans le cas où $R_1$ = Boc et $R_2$ =- $CH_2$- $CO_2Bn$ : le composé (V. 1) est obtenu par réduction de l'aminoacide (11) en aminoalcool (12) qui est ensuite oxydé.

**SCHEMA 7**

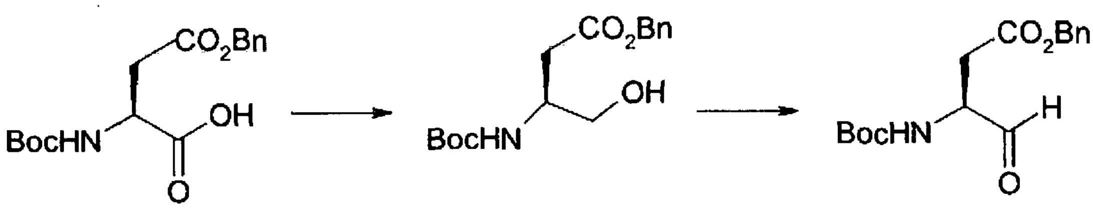

(11) (12) (V.1)

Les composés de formule (I) dans lesquels $X_1$ = CH, X2=N et Y = CO sont préparés *via* une réaction de couplage peptidique selon la méthode de Bouillon et al. (Tetrahedron 2007, 63, 223-2234) entre un aminoacide (VII) convenablement protégé et un composé (III) tel que précédemment décrit, comme illustré sur le **SCHEMA 8** ci-après.

**SCHEMA 8** dans lequel $R_1$, $R_2$, $R_3$, $X_3$ et $X_4$ sont tels que définis pour (I)

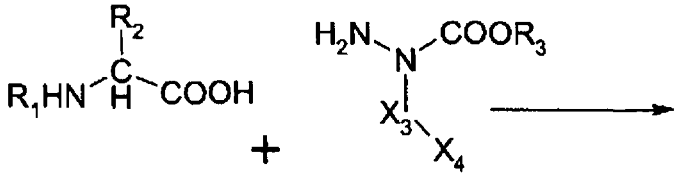

(I) avec $X_1$ = CH, $X_2$ = N et Y = $CH_2$

(VII) (III)

avec $X_2$ = N

Les sels des composés selon l'invention sont préparés selon des techniques bien connues de l'Homme de l'art. Les sels des composés de formule (I) selon la présente invention comprennent ceux avec des acides ou des bases, minéraux ou organiques qui permettent une séparation ou une cristallisation convenable des composés de formule (I), ainsi que des sels pharmaceutiquement acceptables. En tant qu'acide approprié, on peut citer : l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate, le mésylate, le bésylate, l'isotionate. En tant que base appropriée, on peut citer : la lysine, l'arginine, la méglumine, la bénéthamine, la benzathine et celles qui forment des sels physiologiquement acceptables, tels que des sels de sodium, de potassium, de calcium.

Comme composé sous forme hydratée, on peut citer, à titre d'exemple, les semihydrates et monohydrates.

Lorsqu'un composé selon l'invention présente un ou plusieurs carbones asymétriques, les isomères optiques de ce composé font partie intégrante de l'invention. La présente invention comprend les composés de formule (I) sous forme d'isomères purs mais également sous forme de mélange d'isomères en proportion quelconque. Les composés (I) sont isolés sous forme d'isomères purs par les techniques classiques de séparation : on pourra utiliser, par exemple des recristallisations fractionnées d'un sel du racémique avec un acide ou une base optiquement active dont le principe est bien connu ou les techniques classiques de chromatographies sur phase chirale ou non chirale.

Les composés de formule (I) ci-dessus comprennent également ceux dans lesquels un ou plusieurs atomes d'hydrogène, de carbone ou d'halogène, notamment de chlore ou de fluor ont été remplacés par leur isotope radioactif par exemple le tritium ou le carbone-14. De tels composés marqués sont utiles dans des travaux de recherche, de métabolisme ou de pharmacocinétique, dans des essais biochimiques.

Les groupes fonctionnels éventuellement présents dans la molécule des composés de formule (I) et dans les intermédiaires réactionnels peuvent être protégés, soit sous forme permanente soit sous forme temporaire, par des groupes protecteurs qui assurent une synthèse univoque des composés attendus. Les réactions de protection et déprotection sont effectuées selon des techniques bien connues de l'Homme de l'art. Par groupe protecteur temporaire ou permanents des amines, alcools ou des acides carboxyliques on entend les groupes protecteurs tels que ceux décrits dans Protective Groups in Organic Synthesis, Greene T.W. et Wuts P.G.M., ed John Wiley et Sons, 2006 et dans Protecting Groups, Kocienski P.J., 1994, Georg Thieme Verlag.

Les composés de formule (I) décrits précédemment présentent une activité inhibitrice d'une ou plusieurs des protéines d'efflux P-gp et/ou BCRP, activité mise en évidence selon les tests décrits ci-après. De plus, aucun signe de toxicité n'est observé avec ces composés aux doses pharmacologiquement actives et leur toxicité est donc compatible avec leur utilisation comme médicaments.

Ils peuvent donc être utilisés pour potentialiser l'effet, c'est-à-dire augmenter l'effet, d'agents chimiothérapetiques, notamment d'agents anti-infectieux ou anticancéreux, devenus non actifs vis-à-vis de souches résistantes *via* un tel mécanisme d'efflux. Par potentialisation, on entend qu'en associant un composé de formule (I) et un agent anti-infectieux ou anti-cancéreux, on obtient un effet thérapeutique supérieur à celui obtenu avec l'un ou l'autre des composés et même supérieur à la somme des effets obtenus séparément.

Aussi, les composés de formule (I), (IA) et (IB), ainsi que leurs sels, solvats ou hydrates selon l'invention peuvent être utilisés comme adjuvants d'agents anticancéreux, anti- infectieux ou tout autre traitement chimiothérapeutique en médecine humaine et/ou vétérinaire, pour lequel l'organisme traité développe une résistance de type MDR, afin de restaurer leur activité.

Les deux traitements, celui avec le composé de formule (I) et celui avec l'agent de chimiothérapie, peuvent être simultanés, séquencés, successifs, étalés dans le temps. Les deux principes actifs peuvent être administrés, séparément, chacun dans une composition pharmaceutique distincte, l'administration pouvant être simultanée ou étalée dans le temps, ou administrés conjointement dans une composition pharmaceutique unique, l'administration étant alors simultanée.

Différents ordres d'administration ou de traitement peuvent être prévus dans le cadre d'une administration étalée dans le temps. Le composé de formule (I) peut être administré avant (par exemple, 5 minutes, 15 minutes, 30 minutes, 45 minutes, 1 heure, 2 heures, 4 heures, 6 heures, 12 heures, 24 heures, 48 heures, 72 heures, 96 heures, 1 semaine, 2 semaines, 3 semaines, 4 semaines, 5 semaines, 6 semaines, 8 semaines, ou 12 semaines avant), de manière concomitante, ou après (par exemple, 5 minutes, 15 minutes, 30 minutes, 45 minutes, 1 heure, 2 heures, 4 heures, 6 heures, 12 heures, 24 heures, 48 heures, 72 heures, 96 heures, 1 semaine, 2 semaines, 3 semaines, 4 semaines, 5 semaines, 6 semaines, 8 semaines, ou 12 semaines après) l'administration de l'agent de chimiothérapie.

La présente invention a donc également pour objet les composés de formule (I), ainsi que leurs sels pharmaceutiquement compatibles, ou éventuellement solvats ou hydrates, en tant que médicaments, des compositions administrables aux animaux (y compris l'Homme), contenant une dose efficace d'un composé selon l'invention ou d'un sel, d'un solvat ou d'un hydrate acceptable de celui-ci, et des excipients convenables.

La présente invention a également pour objet les compositions pharmaceutiques contenant avec des excipients convenables, séparément ou sous une formulation unique, une dose efficace d'un composé de formule (I), (IA) ou (IB), éventuellement sous la forme d'un sel, solvat ou hydrate pharmaceutiquement acceptable, et d'un agent de chimiothérapie, de préférence choisi parmi les anthracyclines, les inhibiteurs de topoisomérase, les antimétaboliques (antifolates), les inhibiteurs de tyrosine kinase, les antiviraux (inhibiteurs de reverse transcriptase), les antiparasitaires, les antifongiques.

Lesdits excipients sont choisis selon la forme pharmaceutique et le mode d'administration souhaité.

Dans les compositions pharmaceutiques de la présente invention pour l'administration orale, sublinguale, sous-cutanée, intramusculaire, intraveineuse, topique, intratrachéale, intranasale, transdermique, rectale ou intraoculaire, les principes actifs de formule (I) ci-dessus, ou leurs sels, solvats et hydrates éventuels, peuvent être administrés sous formes unitaires d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades, lotions ou collyres.

Afin d'obtenir l'effet, la dose de principe actif varie, de préférence, entre 1 et 100 mg par kg de poids du corps et par jour. Le composé (I) et l'agent anti-infectieux ou anti-cancéreux dont l'effet est à potentialiser sont avantageusement administrés dans un rapport de 4 à 1.

Lorsqu'on prépare une composition solide sous forme de comprimés, on mélange l'ingrédient actif principal avec un véhicule pharmaceutique, tel que la gélatine, l'amidon, le lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

Une préparation sous forme de sirop ou d'élixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié. Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs de goût.

Ainsi, la présente invention a également pour objet des compositions pharmaceutiques contenant plusieurs principes actifs en association dont l'un est un composé (I) et l'autre un agent anti- infectieux ou anti- cancéreux tel que précédemment défini.

Par ailleurs, d'une façon générale, les mêmes préférences que celles indiquées précédemment pour les composés (I) et compositions sont applicables *mutatis mutandis* aux médicaments et utilisations mettant en oeuvre ces composés.

Les exemples ci-après regroupés dans les **TABLEAUX 2 à 5** illustrent l'invention mais n'ont aucun caractère limitatif.

Les abréviations suivantes sont utilisées :

Et = éthyle ; Ac = acétyle ; Bn = benzyle ; Z =- C (O) $OCH_2Ph$ ; Boc = *tert*- butyloxycarbonyle ; tBu = *tert*- butyle ; Ph = phényle ; AcOEt : acétate d'éthyle ; DCM : dichlorométhane ; DMF : diméthylformamide ; DMSO : diméthylsulfoxyde ; E.P. : éther de pétrole ; NMM : N- méthylmorpholine ; éq : équivalent (s) ; IR : infra rouge ; Rf : rapport frontal ; DBAD : di- *tert*- butylazidodicarboxylate ; DEAD : diéthylazidodicarboxylate ; DIAD : diisopropylazidodicarboxylate ; Asp : acide aspartique ; Glu : acide glutamique ; Lys : lysine ; EDCI : N- (3- diméthylaminopropyl)- N'- éthylcarbodiimide ; HOBt : 1- hydroxybenzotriazole ; DCC : dicydohexylcarbodiimide ; DMAP : 4- diméthylaminopyridine.

**TABLEAU 2**

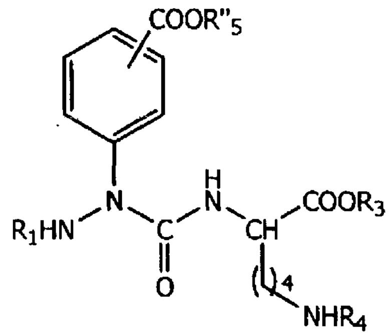

(IA) avec $X_1$ = N, Y=-C(O)-, $X_2$ = -CH-, $R_5$ = -COOR"$_5$, -$X_3$-$X_4$- = -$(CH_2)_4$-$NHR_4$

| Ex. | $R_1$ | COOR"$_5$ | $R_3$ | $R_4$ | Stéréochimie |
|---|---|---|---|---|---|
| **IA.1** | Boc | o-COOBn | tBu | Z | L |
| **IA.2** | Boc | m-COOBn | tBu | Z | L |
| **IA.3** | Boc | p-COOBn | tBu | Z | L |
| **IA.4** | Boc | m-COOBn | Bn | Z | D |
| **IA.5** | Boc | m-COOBn | Bn | Z | L |
| **IA.6** | Boc | m-$COOC_6H_5$ | tBu | Z | L |

**TABLEAU 3**

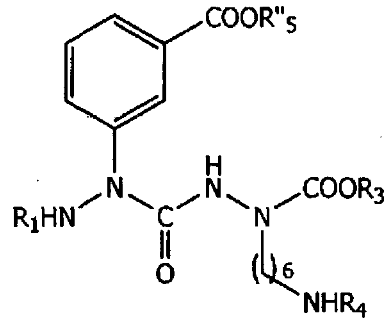

(IA) avec $X_1$ = N, Y=-C(O)-, $X_2$ = N, $R_5$ = -COOR"$_5$, -$X_3$-$X_4$- = -$(CH_2)_6$-$NHR_4$

| Ex. | $R_1$ | COOR"$_5$ | $R_3$ | $R_4$ |
|---|---|---|---|---|
| **IA.7** | Boc | m-COOBn | Bn | Z |
| **IA.8** | Boc | m-COOBn | Bn | Boc |

**TABLEAU 4**

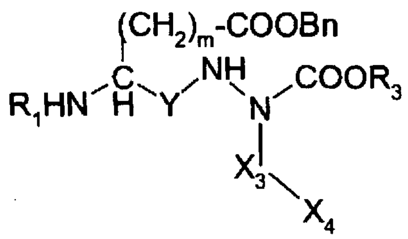

(IB) avec $X_1$ = CH, $X_2$ = N et $R_6$=Bn, stéréochimie L

(suite)

| Ex. | Y | $R_1$ | m | $R_3$ | $-X_3-X_4$ |
|---|---|---|---|---|---|
| **IB.1** | $CH_2$ | Boc | 1 | tBu | $-(CH_2)_6$-NHZ |
| **IB.2** | CO | Boc | 1 | tBu | $-(CH_2)_3$-NHZ |
| **IB.3** | CO | Boc | 1 | tBu | 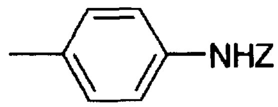 |
| **IB.4** | CO | Boc | 2 | tBu | 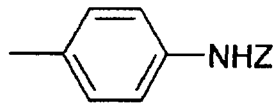 |
| **IB.5** | CO | Boc | 1 | tBu | $-(CH_2)_5$-NHZ |
| **IB.6** | $CH_2$ | Boc | 1 | tBu | 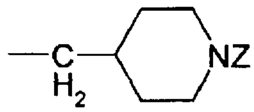 |
| **IB.7** | CO | Boc | 1 | tBu | 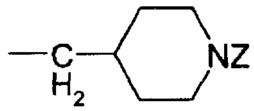 |
| **IB.8** | $CH_2$ | Boc | 1 | tBu | $-(CH_2)_5$-NHZ |
| **IB.9** | CO | Boc | 1 | tBu | $-(CH_2)_6$-NHZ |
| **IB.10** | $CH_2$ | Boc | 1 | tBu | $-(CH_2)_6$-NHBoc |
| **IB.11** | $CH_2$ | Boc | 1 | Bn | $-(CH_2)_6$-NHZ |
| **IB.12** | $CH_2$ | Z | 1 | Bn | $-(CH_2)_6$-NHZ |
| **IB.13** | $CH_2$ | Boc | 1 | Bn | $-(CH_2)_6$-NHBoc |

**TABLEAU 5**

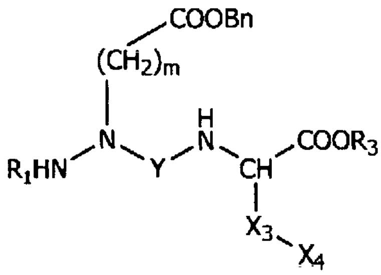

(IB) avec $X_1$ = N, $X_2$ = CH et et $R_6$=Bn

| Ex. | Y | $R_1$ | m | $R_3$ | $-X_3-X_4$ | Stéréochimie |
|---|---|---|---|---|---|---|
| **IB.14** | CO | Boc | 1 | tBu | $-(CH_2)_4$-NHZ | L |

**I. PARTIE EXPERIMENTALE**

**Exemples de synthèse de composés**

**Méthodes générales**

- Les chromatographies analytiques sur couche mince sont réalisées sur des plaques de silice 60 F254 Merck sur feuille d'aluminium. La détection se fait sous UV (254 nm) et la révélation par vaporisation d'une solution de ninhydrine dans l'éthanol ou d'une solution acide d'anisaldhéhyde.
- Les purifications par chromatographie flash sont effectuées sur gel de silice 60Å (40-63 $\mu$m) de marque Merck.
- Les points de fusion sont déterminés sur un appareil à point de fusion à bloc chauffant de marque Electrothermal 9200.
- Les pouvoirs rotatoires sont mesurés à l'aide d'un polarimètre ADP 220 de marque Bellingham+Stanley Ltd ou d'un polarimètre JASCO P-1010.
- Les spectres infra-rouge sont réalisés en film de pastille de chlorure de sodium pour les huiles ou après pastillage dans du bromure de potassium pour les solides. L'appareil de mesure est un spectromètre Perkin-Elmer Spectrum One FT-IR.
- Les spectres de résonance magnétique nucléaire (RMN) du proton sont réalisés sur un appareil Brüker ALS 300 (300 MHz) dans le solvant spécifié entre parenthèses. Ceux du carbone sont enregistrés sur un appareil Brüker DRX 300 (75MHz). Les déplacements chimiques ($\delta$) sont exprimés en ppm (partie par million). Les multiplicités sont : singulet (s), doublet (d), triplet (t), quadruplet (q) et multiplet (m).
- Les spectres de masse ont été réalisés en mode électrospray sur un appareil Thermo-Finnigan LCQ-Advantage en utilisant le dichlorométhane comme solvant.

**EXEMPLE 1**

**Ester *tert*-butylique de l'acide (2S)-6-(benzyloxycarbonylamino)-2-[1-(2'-(benzyloxycarbonyl)phényl)-2-(*tert*-butoxycarbonyl)-hydrazinocarbonylamino]-hexanoïque (IA.1)**

**a) Acide 2-[2-(*tert*-butoxycarbonyl)hydrazino]benzoïque (IV'A.1)**

À une solution de 3 g de chlorhydrate de l'acide 2-hydrazinobenzoïque (16 mmoles, 1 éq) dans le dioxane (1 mL/mmole) est additionnée une solution de 3.81 g de $Boc_2O$ (17.5 mmoles, 1.1 éq) dans le dioxane (0.4 mL/mmole). La solution est portée à 0°C et une solution de $Na_2CO_3$ 5% aqueuse est ajoutée goutte à goutte jusqu'à pH=8-9. La solution est alors ramenée à température ambiante et l'agitation est maintenue durant 24 heures. Le dioxane est évaporé. Le milieu est repris par de l'eau puis acidifié avec une solution d'HCl 1N jusqu'à pH=1. Le mélange est extrait trois fois par de l'acétate d'éthyle. L'ensemble de la phase organique est séchée sur sulfate de sodium, filtrée et évaporée à sec. La poudre obtenue est triturée à l'éther de pétrole puis séchée sous vide pour conduire à 3.75 g de composé **(IV'A.1)** **(rendement** 93%).

**RMN $^1$H** 300 MHz (DMSO) : $\delta$ (ppm) = 1.41 (s, 9H, C $(CH_3)_3$) ; 6.75 (t, J=7.5Hz, 1H, $H_3$) ; 6.86 (d, J=8.1Hz, 1H, $H_5$) ; 7.43 (t, J=7.8Hz, 1H, $H_4$) ; 7.81 (d, J=8.1Hz, 1H, $H_6$) ; 8.88 (s, 1H, NH) ; 9.05 (s, 1H, NH) ; 13 (s, 1H, COOH) .

**IR** (KBr) : $\nu_{NH}$ = 3381.6 $cm^{-1}$ ; $\nu_{Csp3-H}$ = 2971.7 $cm^{-1}$ ; $\nu_{C=O}$ = 1697.6 $cm^{-1}$ et 1676.7 $cm^{-1}$ ; $\nu_{C=C}$ = 1609.1 $cm^{-1}$ et 1587.2 $cm^{-1}$.

**Point de fusion** = 146°C.

**b) Ester benzylique de l'acide 2-[2-(*tert*-butoxycarbonyl)hydrazino]benzoïque (IVA.1)**

1, 5 g d'acide $N_\beta$- Boc- hydrazinobenzoïque (**IV'A. 1**) (5.94 mmoles, 1 éq) sont dissous dans du méthanol (4.2 mL/ mmole) et de l'eau (0, 42 mL/ mmole) . Le pH est amené à pH=7 par ajout d'une solution de $CsCO_3$ 20 % aqueuse. Le solvant est évaporé et le résidu repris deux fois par du DMF (1 mol/ 100 mg d'acide) puis réévaporé. Le sel de césium ainsi isolé est dilué dans du DMF (2 mL/ 100 mg d'acide) et 0, 78 mL de bromure de benzyle (6, 54 mmoles, 1.1 éq) sont ajoutés. Le mélange réactionnel est maintenu sous agitation durant 4 heures à température ambiante. Le DMF est évaporé et le mélange obtenu repris dans un grand volume d'eau puis extrait à l'acétate d'éthyle. La phase organique est lavée avec de l'eau puis avec une solution de NaCl saturée, séchée sur sulfate de sodium, filtrée et évaporée à sec. Le composé brut est purifié par chromatographie flash sur gel de silice avec comme éluant un mélange DCM/E.P. (3: 1 en volume) pour conduire à 1.88 g d'une huile jaune pâle (**rendement** 96%) .

**RMN $^1$H** 300MHz (DMSO) : $\delta$ (ppm) = 1.41 (s, 9H, C $(CH_3)_3$) ; 5.34 (s, 2H, $CH_2$) ; 6.75- 7.89 (m, 9H, $H_{arom}$) ; 8.73 (s, 1H, NH) ; 9.10 (s, 1H, NH) .

**IR** (NaCl) : $\nu_{NH}$ = 3346.2 $cm^{-1}$ ; $\nu_{Csp2-H}$ = 3066.3 $cm^{-1}$ et 3034.8 $cm^{-1}$; $\nu_{Csp3-H}$ = 2978.4 $cm^{-1}$ et 2934.6 $cm^{-1}$ ; $\nu_{C=O}$ = 1722.9 $cm^{-1}$ et 1692 $cm^{-1}$; $\nu_{C=C}$ = 1606.4 $cm^{-1}$ et 1585.6 $cm^{-1}$.

**c**) Á une solution de 250 mg d'hydrazinoester (**IVA. 1**) (0.73 mmole, 1 éq) dans le DCM distillé (4 mL/ mmole) sont additionnés 0.35 éq de triphosgène. La température du milieu réactionnel est portée à- 10 °C puis une solution de NMM (1.08 éq) dans le DCM distillé (0.5 mL/ mmole) est introduite. Le milieu réactionnel est agité à- 10 °C pendant 1 heure. 270 mg de H- Lys (Z)- OtBu (0.73 mmole, 1 éq) puis une solution de NMM (1.08 éq) dans le DCM distillé (0.5 mL/ mmole) sont alors ajoutés. La température est maintenue 2 heures à- 10°C puis ramenée progressivement à

température ambiante. Après 20 heures à température ambiante, le milieu est dilué par ajout de DCM (30 mL/ mmole d'hydrazinoester) puis filtré. La phase organique est lavée avec une solution 0.5M de $KHSO_4$ puis avec de l'eau, séchée sur sulfate de sodium, filtrée et évaporée. La purification du produit brut est réalisée par chromatographie flash sur gel de silice avec comme éluant un mélange AcOEt/E.P. (1: 2 en volume) . 250 mg d'une huile jaune sont obtenus (**rendement** 45 %) .

**RMN $^1$H** 300MHz ($CDCl_3$) : $\delta$ (ppm) = 0.87 (m, 2H, $\underline{CH_2}$- CH$\alpha$) ; 1.44 (s, 9H, C $(CH_3)_3$) ; 1.48 (s, 9H, C $(CH_3)_3$) : 1.7 (m, 4H, $CH_2$- $\underline{(CH_2)_2}$- $CH_2$) ; 3.18 (m, 2H, $\underline{CH_2}$- NHZ) ; 4.46 (m, 1H, CH$\alpha$) ; 5.02 (s, large, 1H, NH) ; 5.08 (s, 2H, $\underline{CH_2}$- $C_6H_5$) ; 5.36 (s, 2H, $\underline{CH_2}$- $C_6H_5$) ; 6.12 (s, large, 1H, NH) ; 7.12 (s, large, 1H, NH) ; 7.38- 8.2 (m, 14H, $H_{arom}$) .

**RMN $^{13}$C** 75MHz ($CDCl_3$) : $\delta$ (ppm) = 11.37 ; 14.61 ; 21.45 ; 22.39 ; 28.42 ; 29.6 ; 30.1 ; 33.1 ; 41.19 ; 53.92 ; 60.78 ; 66.83 ; 67.58 ; 77.1 ; 77.53 ; 77.73 ; 77.95 ; 82.27 ; 127.6 ; 128.36 ; 128.44 ; 128.65 ; 128.75 ; 128.85 ; 129.1 ; 132.01 ; 133.98 ; 135.8 ; 137.18 ; 141.64 ; 155.08 ; 156.61 ; 156.86 ; 166.13 ; 171.53 ; 172.13.

**IR** (NaCl) : $\nu_{NH}$ = 3349.2 $cm^{-1}$; $\nu_{Csp2-H}$= 3066.1 $cm^{-1}$ et 3033.8 $cm^{-1}$; $\nu_{Csp3-H}$ = 2978.3 $cm^{-1}$ et 2934.1 $cm^{-1}$; $\nu_{C}$=o = 1725.6 $cm^{-1}$, 1713.6 $cm^{-1}$, 1700.9 $cm^{-1}$ et 1681.7 $cm^{-1}$; $\nu_{C=C}$ = 1599.8 $cm^{-1}$ et 1578.2 $cm^{-1}$.

[$\alpha$] $D^{25°C}$ = +1.68° (16.8 $g.L^{-1}$, DCM) .

HRMS- ESI (m/z) : [M+Na] calc. pour $C_{38}H_{48}N_4O_9$, 727.3319 ; exp., 727.3324.

**EXEMPLE 2**

**Ester *tert*-butylique de l'acide (2S)-6-(benzyloxycarbonylamino)-2-[1-(3'-(benzyloxycarbonyl)phényl)-2-(tert-butoxycarbonyl)-hydrazinocarbonylamino]-hexanoïque (IA.2)**

**a) Acide 3-[2-(*tert*-butoxycarbonyl)hydrazino]benzoïque (IV'A.2)**

Selon un mode opératoire identique à celui décrit pour le composé (**IV'A. 1**), 2.4 g d'acide 3- hydrazinobenzoïque (16 mmoles, 1 éq) sont mis en réaction avec 3.8g de $Boc_2O$ (17 mmoles, 1.1 éq) pour conduire à 3.96g d'acide (**IV'A. 2**) (**rendement** 98%) .

**RMN $^1$H** 300 MHz (DMSO) : $\delta$ (ppm) = 1.42 (s, 9H, C $(CH_3)_3$) ; 6.88 (m, 1H, $H_4$) ; 7.27 (m, 3H, $H_2$- $H_5$- $H_6$) ; 7.83 (s, 1H, NH) ; 8.86 (s, 1H, NH) ; 12.78 (s, 1H, COOH) .

**IR** (KBr) : $\nu_{NH}$ = 3315.6 $cm^{-1}$ ; $\nu_{Csp3-H}$ = 2982.0 $cm^{-1}$ ; $\nu_{C=O}$ = 1706.4 $cm^{-1}$ et 1689.2 $cm^{-1}$; $\nu_{C=C}$ = 1611.2 $cm^{-1}$ et 1594.4 $cm^{-1}$.

**Point de fusion = 150°C**

**b) Ester benzylique de l'acide 3-[2-(*tert*-butoxycarbonyl)hydrazino]benzoïque (IVA.2)**

Selon un mode opératoire identique à celui décrit pour le composé (**IVA.1**), 406 mg de composé (**IV'A.2**) (1.61 mmoles, 1 éq) et 0.211 mL de bromure de benzyle (1.77 mmoles, 1.1 éq) conduisent, après purification par chromatographie flash sur gel de silice avec comme éluant un mélange AcOEt/E.P. (1:5) en volume, à 283 mg d'une huile jaune (**rendement** 51%).

**RMN $^1$H** 300MHz (DMSO) : $\delta$ (ppm) = 1.40 (s, 9H, C $(CH_3)_3$) ; 5.32 (s, 2H, $CH_2$) ; 7.25- 7.50 (m, 9H, $H_{arom}$) ; 7.90 (s, 1H, NH) ; 8.88 (s, 1H, NH) .

**IR** (NaCl) : $\nu_{NH}$ = 3334.5 $cm^{-1}$ ; $\nu_{Csp2-H}$ = 3066.1 $cm^{-1}$ et 3034.5 $cm^{-1}$ ; $\nu_{Csp3-H}$ = 2979.2 $cm^{-1}$ et 2934.7 $cm^{-1}$ ; $\nu_{C=O}$ = 1714.9 $cm^{-1}$ et 1699.1 $cm^{-1}$; $\nu_{C=C}$ = 1608.2 $cm^{-1}$ et 1595.1 $cm^{-1}$.

c) Selon un mode opératoire identique à celui décrit pour le composé (**IA. 1**), 270 mg d'hydrazinoester (**IVA. 2**) (0.79 mmole, 1 éq) et 295 mg de H- Lys (Z)- OtBu (0.79 mmole, 1 éq) conduisent, après purification par chromatographie flash sur gel de silice avec comme éluant un mélange AcOEt/E.P. (1: 3) puis (1: 2) en volume, à 220 mg d'une huile jaune (**rendement** 40 %) .

**RMN $^1$H** 300MHz ($CDCl_3$) : $\delta$ (ppm) = 1.18 (m, 2H, $CH_2$- CH$\alpha$) ; 1.35 (s, 9H, C $(CH_3)_3$) ; 1.38 (s, 9H, C $(CH_3)_3$) ; 1.40- 1.62 (m, 4H, $CH_2$- $(CH_2)_2$- $CH_2$) ; 3.10 (m, 2H, $\underline{CH_2}$- NHZ) ; 4.35 (m, 1H, CH$\alpha$) ; 5.02 (s, 2H, $\underline{CH_2}$- $C_6H_5$) 5.28 (s, 2H, $\underline{CH_2}$- $C_6H_5$) 5.85 (s, large, 1H, NH) ; 6.95 (s, large, 1H, NH) ; 7.2- 8.0 (m, 14H, $H_{arom}$) .

**RMN $^{13}$C** 75MHz ($CDCl_3$) : $\delta$ (ppm) = 22.14 ; 28.05 ; 28.09 ; 29.34 ; 32.49 ; 40.75 ; 53.71 ; 66.58 ; 66.92 ; 82.18 ; 82.56 ; 127.25 ; 128.07 ; 128.30 ; 128.52 ; 128.63 ; 129.00 ; 129.14 ; 130.99 ; 135.92 ; 136.72 ; 142.06 ; 154.75 ; 155.29 ; 156.57 ; 165.88 ; 171.93.

**IR** (NaCl) : $\nu_{NH}$ = 3334 $cm^{-1}$; $\nu_{Csp2-H}$ = 3066.1 $cm^{-1}$ et 3034.3 $cm^{-1}$ ; $\nu_{Csp3-H}$ = 2978.3 $cm^{-1}$ et 2927.4 $cm^{-1}$ ; $\nu_{C=O}$ = 1726.6 $cm^{-1}$, 1714.9 $cm^{-1}$, 1695.5 $cm^{-1}$, et 1679.1 $cm^{-1}$; $\nu_{C=C}$ = 1604.5 $cm^{-1}$ et 1587.8 $cm^{-1}$.

$[\alpha]_D^{25.7°C}$ = +4° (10 $g.L^{-1}$, DCM) .

HRMS- ESI (m/z) : [M+Na] calc. pour $C_{38}H_{48}N_4O_9$, 727.3319 ; exp., 727.3324.

## EXEMPLE 3

**Ester *tert*-butylique de l'acide (2S)-6-(benzyloxycarbonylamino)-2-[1-(4'-(benzyloxycarbonyl)phényl)-2-(*tert*-butoxycarbonyl)-hydrazinocarbonylamino]-hexanoïque (IA.3)**

**a)Acide 4-[2-(*tert*-butoxycarbonyl)hydrazino]benzoique (IVA'.3)**

Selon un mode opératoire identique à celui décrit pour le composé (**IV'A. 1**), 5 g d'acide 4- hydrazinobenzoïque (26 mmoles, 1 éq) sont mis en réaction avec 6.54 g de $Boc_2O$ (30 mmoles, 1.1 éq) pour conduire à 6.7 g d'acide (**IVA'. 3**) (**rendement** 100%) .

**RMN $^1H$** 300MHz (DMSO) : $\delta$ (ppm) = 1.42 (s, 9H, C $(CH_3)_3$) ; 6.65 (d, J=8.7Hz, 2H, , $H_3$, $H_5$) ; 7.74 (d, J=8.4Hz, 2H, $H_2$, $H_6$) ; 8.24 (s, 1H, NH) ; 8.92 (s, 1H, NH) ; 12.23 (s, 1H, COOH) .

**IR** (KBr) : $v_{NH}$ = 3401.4 $cm^{-1}$; $v_{Csp3-H}$ = 2981.5 $cm^{-1}$; $v_{C=O}$ = 1700.9 $cm^{-1}$ et 1683.2 $cm^{-1}$ ; $v_{C=C}$ = 1606.2 $cm^{-1}$.

**Point de fusion** = 144°C.

**b) Ester benzylique de l'acide 4-[2-(*tert*-butoxycarbonyl)hydrazino]benzoique (IVA.3)**

Selon un mode opératoire identique à celui décrit pour le composé (**IVA.1**), 1 g de composé (**IVA'.3**) (3.96 mmoles, 1 éq) et 0.51 mL de bromure de benzyle (4.36 mmoles, 1.1 éq) conduisent, après purification par chromatographie flash sur gel de silice avec comme éluant un mélange AcOEt/E.P. (1:2 en volume), à 800 mg d'une huile jaune (**rendement** 58 %).

**RMN $^1H$** 300MHz (DMSO) $\delta$ (ppm) = 1.42 (s, 9H, C $(CH_3)_3$) ; 5.28 (s, 2H, $CH_2$) ; 6.67- 7.84 (m, 9H, $H_{arom}$) ; 8.35 (s, 1H, NH) ; 9 (s, 1H, NH) .

**IR** (NaCl) : $v_{NN}$ = 3313.9 $cm^{-1}$ ; $v_{Csp2-H}$ = 3067 $cm^{-1}$ et 3034.5 $cm^{-1}$ ; $v_{Csp3-N}$ = 2983 $cm^{-1}$ et 2934.6 $cm^{-1}$ ; $v_{C=O}$ = 1709.1 $cm^{-1}$ et 1688.9 $cm^{-1}$ ; $v_{C=C}$ = 1609.4 $cm^{-1}$.

**c)** Selon un mode opératoire identique à celui décrit pour le composé (**IA. 1**), 244 mg d'hydrazinoester (**IVA. 3**) (0.71 mmole, 1 éq) et 260 mg de H- Lys (Z)- OtBu (0.71 mmole, 1 éq) conduisent, après purification par chromatographie flash sur gel de silice avec comme éluant un mélange AcOEt/E.P. (1: 2 en volume), à 150 mg d'une poudre beige (**rendement** 30 %) .

**RMN $^1H$** 300MHz ($CDCl_3$) : $\delta$ (ppm) = 0.89 (m, 2H, $\underline{CH_2}$- CH$\alpha$) ; 1.48 (s, 18H, C $(CH_3)_3$, C $(CH_3)_3$) ; 1.68 (m, 4H, $CH_2$- $(\underline{CH_2})_2$- $CH_2$) ; 3.18 (m, 2H, $\underline{CH_2}$- NHZ) ; 4.46 (m, 1H, CH$\alpha$) ; 5.0 (s, large, 1H, NH) ; 5.09 (s, 2H, $\underline{CH_2}$- $C_6H_5$) ; 5.36 (s, 2H, $\underline{CH_2}$- $C_6H_5$) ; 6.1 (s, large, 1H, NH) ; 7.08 (s, large, 1H, NH) ; 7.8- 8.1 (m, 14H, $H_{arom}$) .

**$R_{MN}$ $^{13}C$** 75MHz ($CDCl_3$) : $\delta$ (ppm) = 14.60 ; 21.46 ; 22.44 ; 28.21 ; 28.40 ; 28.45 ; 29.69 ; 30.09 ; 31.84 ; 32.69 ; 36.89 ; 41.06 ; 54.05 ; 60.81 ; 66.92 ; 66.99 ; 77.10 ; 77.53 ; 77.73 ; 77.95 ; 82.58 ; 82.92 ; 121.85 ; 126.53 ; 128.43 ; 128.53 ; 128.60 ; 128.88 ; 128.98 ; 130.86 ; 136.49 ; 137.03 ; 146.45 ; 154.97 ; 155.36 ; 156.96 ; 163.05 ; 166.3 ; 172.28.

**IR** (KBr) : $v_{NH}$ = 3389.9 $cm^{-1}$; $v_{Csp2-H}$ = 3068.2 $cm^{-1}$ et 3035.4 $cm^{-1}$ ; $v_{Csp3-H}$ = 2978.1 $cm^{-1}$ et 2932.6 $cm^{-1}$; $v_{C=O}$ = 1719.3 $cm^{-1}$, 1705.5 $cm^{-1}$, 1672.2 $cm^{-1}$ et 1665.2 $cm^{-1}$ ; $v_{C=C}$ = 1607.4 $cm^{-1}$.

$[\alpha]_D^{25°C}$ = +2.15° (13.9 g.$L^{-1}$, DCM) .

**Point de fusion** = 106°C.

HRMS- ESI (m/z) : [M+Na] calc. pour $C_{38}H_{48}N_4O_9$, 727.3319 ; exp., 727.3315.

## EXEMPLE 4

**Ester benzylique de l'acide (2R)-6-(benzyloxycarbonylamino)-2-[1-(3'-(benzyloxycarbonyl)phényl)-2-(*tert*-butoxycarbonyl)-hydrazinocarbonylamino]-hexanoïque (IA.4)**

Á une solution de 500 mg d'hydrazinoester (**IVA. 2**) (1.46 mmoles, 1 éq) dans le DCM distillé (4 mL/ mmole) sont additionnés 0.35 éq de triphosgène. La température du milieu réactionnel est portée à- 10 °C puis une solution de NMM (2 éq) dans le DCM distillé (0.5 mL/ mmole) est introduite. Le milieu réactionnel est agité à- 10 °C pendant 1 heure. 600 mg de HC) .H- D- Lys (Z)- OBn (1.46 mmoles, 1 éq) puis une solution de NMM (2 éq) dans le DCM distillé (0.5 mL/ mmole) sont alors ajoutés. La température est maintenue 2 heures à- 10°C puis ramenée progressivement à température ambiante. Après 20 heures à température ambiante, le milieu est dilué par ajout de DCM (30 mL/ mmole d'hydrazinoester) puis filtré. La phase organique est lavée avec une solution 0.5M de $KHSO_4$ puis avec de l'eau, séchée sur sulfate de sodium, filtrée et évaporée. La purification du produit brut est réalisée par chromatographie flash sur gel de silice avec comme éluant un mélange AcOEt/ Cyclohexane (3: 7 en volume) . 362 mg d'une pâte jaune sont obtenus (**rendement** 34%) .

**RMN $^1H$** 300MHz (DMSO) : $\delta$ (ppm) = 0.88- 1.20 (m, 2H, $\underline{CH_2}$- CH$\alpha$) ; 1.40 (s, 9H, COO $(\underline{CH_3})_3$) ; 1.60- 1.93 (m, 4H, $CH_2$- $(\underline{CH_2})_2$- CHa) ; 3.07- 3.14 (m, 2H, $CH_2$- NHZ) ; 4.55- 4.63 (m, 1H, CH$\alpha$) ; 5.06 (s, 2H, $CH_2$- Ph) ; 5.17

(s, 2H, $CH_2$- Ph) ; 5.33 (s, 2H, $CH_2$- Ph) ; 5.35 (s, 2H, $CH_2Ph$) ; 7.12 (s large, 1H, NH) ; 7.30- 7.43 (m, 20H, $H_{arom}$) ; 7.70 (s large, 1H, NH) .

**RMN $^{13}$C** 75MHz (DMSO) : δ (ppm) = 28.73 ; 29.80 ; 30.48 ; 31.11 ; 40.94 ; 54.24 ; 65.97 ; 66.66 ; 67.14 ; 81.28 ; 125.55 ; 127.48 ; 128.57 ; 128.81 ; 128.88 ; 128.93 ; 129.09 ; 129.18 ; 129.26 ; 129.36 ; 129.38 ; 129.54 ; 130.56 ; 136.91 ; 138.13 ; 143.79 ; 150.01 ; 155.32 ; 156.44 ; 156.91 ; 156.25 ; 173.29.

**IR** (KBr) : $\nu_{NH}$ = 3344 $cm^{-1}$; $\nu_{Csp2\ Harom}$ = 3065 $cm^{-1}$ et 3033 $cm^{-1}$ $\nu_{C=O}$ = 1715 $cm^{-1}$ ; $\nu_{C-O}$ ester = 1248 $cm^{-1}$ et 1158 $cm^{-1}$.

$[\alpha]_D^{25°C}$ = +3° (5 g.$L^{-1}$, DCM) .

HRMS- ESI (m/z) : [M+Na] calc. pour $C_{41}H_{46}N_4O_9$, 761.3162 ; exp., 761.3163.

## EXEMPLE 5

**Ester benzylique de l'acide (2S)-6-(benzyloxycarbonylamino)-2-[1-(3'-(benzyloxycarbonyl)phényl)-2-(tert-butoxycarbonyl)-hydrazinocarbonylamino]-hexanoïque (IA.5)**

Selon un mode opératoire identique à celui décrit pour le composé (**IA. 4**), 500 mg d'hydrazinoester (**IVA. 2**) (1.46 mmoles, 1 éq) et 610 mg de HCI.H- Lys (Z)- OBn (1.46 mmoles, 1 éq) conduisent, après purification par chromatographie flash sur gel de silice avec comme éluant un mélange AcOEt/ Cyclohexane (3: 7 en volume), à 320 mg d'une pâte jaune (**rendement 30%**) .

**RMN $^1$H** 300MHz (DMSO) : δ (ppm) = 1.31 (m, 2H, <u>$CH_2$</u>- CHα) ; 1.38 (s, 9H, COO (<u>$CH_3$</u>)$_3$) ; 1.61- 1.77 (m, 4H, $CH_2$- (<u>$CH_2$</u>)$_2$- CHα) ; 2.94- 2.96 (m, 2H, $CH_2$- NHZ) ; 4.19- 4.21 (m, 1H, CHα) ; 4.99 (s, 2H, $CH_2$- Ph) ; 5.13 (s, 2H, $CH_2$- Ph) ; 5.35 (s, 2H, $CH_2Ph$) ; 7.21- 7.72 (m, 19H, $H_{arom}$) .

**RMN $^{13}$C** 75MHz (DMSO) : δ (ppm) = 27.21 ; 28.13 ; 28.73 ; 29.80 ; 30.48 ; 31.10 ; 40.93 ; 54.23 ; 65.97 ; 66.66 ; 67.14 ; 81.27 ; 128.57 ; 128.81 ; 128.88 ; 128.93 ; 129.00 ; 129.18 ; 129.23 ; 129.26 ; 129.38 ; 130.27 ; 130.56 ; 136.79 ; 136.91 ; 138.13 ; 143.79 ; 155.32 ; 156.45 ; 156.91 ; 166.25 ; 173.04.

**IR** (KBr) : $\nu_{NH}$ = 3354 $cm^{-1}$; $\nu_{Csp2\ Harom}$ = 3066 $cm^{-1}$ et 3034 $cm^{-1}$ ; $\nu_{C=O}$ = 1723 $cm^{-1}$; $\nu_{C-O}$ ester = 1248 $cm^{-1}$ et 1156 $cm^{-1}$

$[\alpha]_D^{25°C}$ =- 3° (5 g.$L^{-1}$, DCM) .

HRMS- ESI (m/z) : [M+Na] calc. pour $C_{41}H_{46}N_4O_9$, 761.3162 ; exp., 761.3160.

## EXEMPLE 6

**Ester *tert*-butylique de l'acide (2S)-6-(benzyloxycarbonylamino)-2-[1-(3'-(phényloxycarbonyl)phényl)-2-(*tert*-butoxycarbonyl)-hydrazinocarbonylamino]-hexanoique (IA.6)**

**a) Ester phénylique de l'acide 3-(2-*tert*-butoxycarbonyl-hydrazino)-benzoïque (IVA.6)**

A une solution de 780 mg (3 mmoles, 1éq) de composé (**IV'A.2**) dans le DCM (3 mL/mmole) et le DMF (quantité minimale à dissolution), sont additionnés 618 mg (3 mmoles, 1 éq) de DCC et 873 mg (9 mmoles, 3 éq) de phénol. Après agitation pendant 15 minutes à température ambiante, 18 mg (0.15 mmole, 0.05 éq) de DMAP sont introduits. Le mélange réactionnel est agité à température ambiante pendant 4 heures puis filtré. Le filtrat est évaporé à sec puis repris dans 30 mL d'AcOEt. La phase organique est lavée 2 fois avec une solution d'HCl 0.1 N, avec une solution de $NaHCO_3$ 0.1 N et avec une solution saturée de NaCl, séchée sur sulfate de sodium, filtrée et évaporée. La purification du produit brut est réalisée par chromatographie flash sur gel de silice avec comme éluant un mélange AcOEt/Cyclohexane (1:9 en volume).

**RMN $^1$H** 300MHz (DMSO) : δ (ppm) = 1.41 (s, 9H, C ($CH_3$)$_3$) ; 6.98- 7.50 (m, 9H, $H_{arom}$) ; 7.97 (s, 1H, NH) ; 8.93 (s, 1H, NH) .

**b)** Selon un mode opératoire identique à celui décrit pour le composé (**IA. 1**), 300 mg d'hydrazinoester (**IVA. 6**) (0.9 mmole, 1 éq) et 340 mg de H- Lys (Z)- OtBu (0.9 mmole, 1 éq) conduisent, après purification par chromatographie flash sur gel de silice avec comme éluant un mélange AcOEt/ Cyclohexane (3: 7 en volume), à 130 mg d'un solide jaune.

**RMN $^1$H** 300MHz ($CDCl_3$) : δ (ppm) = 1.19 (m, 2H, <u>$CH_2$</u>- CHα) ; 1.39 (s, 9H, C ($CH_3$)$_3$) ; 1.41 (s, 9H, C ($CH_3$)$_3$) ; 1.50- 1.72 (m, 4H, $CH_2$- (<u>$CH_2$</u>)$_2$- $CH_2$) ; 2.97 (m, 2H, <u>$CH_2$</u>- NHZ) ; 4.04 (m, 1H, CHα) ; 4.99 (s, 2H, <u>$CH_2$</u>- $C_6H_5$) ; 7.23- 7.94 (m, 14H, $H_{arom}$) ; 8.14 (s, large, 1H, NH) .

**IR** (KBr) : $\nu_{NH}$ = 3378.6 $cm^{-1}$ **;** $\nu_{Csp2\ Harom}$ = 3066 $cm^{-1}$ et 3034 $cm^{-1}$; $\nu_{Csp3-H}$ = 2978.2 $cm^{-1}$ et 2931.7 $cm^{-1}$ ; $\nu_{C=O}$ = 1737.6 $cm^{-1}$ et 1700.9 $cm^{-1}$ ; $\nu_{C-O}$ ester = 1246.2 $cm^{-1}$ et 1158.4 $cm^{-1}$

$[\alpha]_D^{25°C}$ = 0.3° (5 g.$L^{-1}$, DCM) .

HRMS- ESI (m/z) : [M+Na] calc. pour $C_{37}H_{46}N_4O_9$, 713.3162 ; exp., 713.3162.

## EXEMPLE 7

**Ester benzylique de l'acide 2-[1-(3'-(benzyloxycarbonyl)phényl)-2-(*tert*-butoxycarbonyl)-hydrazinocarbonyl]-1-(6-benzyloxycarbonylamino-hexyl)-hydrazinocarboxylique (IA.7)**

**a)1-benzyloxycarbonyl-1-(6-benzyloxycarbonyl-aminohexyl)hydrazine (III.3)**

Le composé (**III. 3**) est obtenu selon un mode opératoire identique à celui suivi pour la synthèse de (**III. 2**) décrit ci- après en utilisant de la benzyloxycarbonyl- hydrazine et du 6- (benzyloxycarbonyl- amino) hexanol comme produits de départ.

**RMN $^1$H** 300MHz (DMSO) : δ (ppm) = 1.39- 1.34 (m, 6H, $(CH_2)_3$) ; 1.51- 1.46 (m, 2H, $CH_2$) ; 2.98- 2.92 (q, 2H, $CH_2$- NHZ) ; 3.31- 3.26 (t, 2H, $CH_2$- N- $NH_2$) ; 4.57 (s, 2H, $NH_2$) ; 5.00 (s, 2H, $CH_2$- Ph) ; 5.06 (s, 2H, $CH_2$- Ph) ; 7.37- 7.20 (m, 10H, $H_{arom}$) .

**IR** (KBr) : $\nu_{NH}$ = 3306 $cm^{-1}$ ; $\nu_{Csp2\ Harom}$ = 3061 $cm^{-1}$ et 3030 $cm^{-1}$ ; $\nu_{C=O}$ = 1676 $cm^{-1}$; $\nu_{C=C}$ = 1535 $cm^{-1}$; $\nu_{C\text{-}O\ ester}$ = 1257 $cm^{-1}$ et 1190 $cm^{-1}$

**b)** Selon un mode opératoire identique à celui décrit pour le composé (**IA. 4**), 850 mg d'hydrazinoester (**IVA. 2**) (2.5 mmoles, 1 éq) et 990 mg de composé (**III. 3**) (2.5 mmoles, 1 éq) conduisent, après purification par chromatographie flash sur gel de silice avec comme éluant un mélange AcOEt/ Cyclohexane (3: 7 en volume), à 244 mg d'une huile.

**RMN $^1$H** 300MHz (DMSO) : δ (ppm) = 1.19- 1.30 (m, 6H, $(CH_2)_3$) ; 1.38 (s, 9H, COO $(CH_3)_3$) ; 1.49 (m, 2H, $CH_2$) 2.98- 2.92 (q, 2H, $CH_2$- NHZ) ; 3.41 (t, 2H, $CH_2$- N- $NH_2$) ; 4.99 (s, 2H, $CH_2$- Ph) ; 5.10 (s, 2H, $CH_2$- Ph) ; 5.35 (s, 2H, $CH_2Ph$) ; 7.33- 7.50 (m, 19H, $H_{arom}$) .

**IR** (NaCl) : $\nu_{NH}$ = 3308.0 $cm^{-1}$ ; $\nu_{Csp2\ Harom}$ = 3065.9 $cm^{-1}$ et 3034.0 $cm^{-1}$ ; $\nu_{Csp3\text{-}H}$ = 2978.2 $cm^{-1}$ et 2932.8 $cm^{-1}$; $\nu_{C=O}$ = 1715.3 $cm^{-1}$ ; $\nu_{C\text{-}O}$ ester = 1251.3 $cm^{-1}$ et 1161.1 $cm^{-1}$.

HRMS- ESI (m/z) : [M+Na] calc. pour $C_{42}H_{49}N_5O_9$, 790.3428 ; exp., 790.3429.

## EXEMPLE 8

**Ester benzylique de l'acide 2-[1-(3'-(benzyloxycarbonyl)phényl)-2-(*tert*-butoxycarbonyl)-hydrazinocarbonyl]-1-(6-*tert*-butyloxycarbonylamino-hexyl)-hydrazinocarboxylique (IA.8)**

**a) 1-benzyloxycarbonyl-1-(6-*tert*-butoxycarbonyl-aminohexyl)hydrazine (III.4)**

Le composé (**III. 4**) est obtenu selon un mode opératoire identique à celui suivi pour la synthèse de (**III. 2**) décrit ci- après en utilisant de la benzyloxycarbonyl- hydrazine et du 6- (tert- butoxycarbonyl- amino) hexanol comme produits de départ et en subtituant le DIAD par du DBAD au cours de la réaction de Mitsunobu.

**RMN $^1$H** 300MHz (DMSO) : δ (ppm) = 1.35 (s, 9H, COO $(CH_3)_3$) ; 1.20- 1.50 (m, 8H, $CH_2$- $(CH_2)_4$- $CH_2$) ; 2.87 (q, 2H, $CH_2$- NHZ) ; 3.28 (t, 2H, $CH_2$- N- $NH_2$) ; 4.57 (s, 2H, $NH_2$) ; 5.06 (s, 2H, $CH_2$- Ph) ; 6.75 (s, 1H, NH) ; 7.33 (m, 5H, $H_{arom}$) .

**IR** (KBr) : $\nu_{NH}$ = 3345 $cm^{-1}$ ; $\nu_{Csp2\ Harom}$ = 3065 $cm^{-1}$ et 3033 $cm^{-1}$ ; $\nu_{Csp3}$ = 2975 $cm^{-1}$ et 2933 $cm^{-1}$ ; $\nu_{C=O}$ = 1696 $cm^{-1}$; $\nu_{C=C}$ = 1523 $cm^{-1}$; $\nu_{C\text{-}O}$ ester = 1250 $cm^{-1}$ et 1174 $cm^{-1}$.

**b)** Selon un mode opératoire identique à celui décrit pour le composé (**IA. 4**), 850 mg d'hydrazinoester (**IVA. 2**) (2.5 mmoles, 1 éq) et 913 mg de composé (**III. 4**) (2.5 mmoles, 1 éq) conduisent, après purification par chromatographie flash sur gel de silice avec comme éluant un mélange AcOEt/ Cyclohexane (3: 7 en volume), à 50 mg d'une pâte marron.

**RMN $^1$H** 300MHz (DMSO) : δ (ppm) = 1.23- 1.30 (m, 6H, $(CH_2)_3$) ; 1.36 (s, 9H, COO $(CH_3)_3$) ; 1.39 (s, 9H, COO $(CH_3)_3$) ; 1.48 (m, 2H, $CH_2$) ; 2.86 (q, 2H, $CH_2$- NHZ) ; 3.41 (t, 2H, $CH_2$- N- $NH_2$) ; 5.09 (s, 2H, $CH_2$- Ph) ; 5.35 (s, 2H, $CH_2$- Ph) ; 7.28- 7.47 (m, 14H, $H_{arom}$) .

**IR** (NaCl) : $\nu_{NH}$ = 3325.0 $cm^{-1}$ ; $\nu_{Csp2\ Harom}$ = 3065.9 $cm^{-1}$ et 3027.4 $cm^{-1}$ ; $\nu_{Csp3\text{-}H}$=2920.8 $cm^{-1}$; $\nu_{C=O}$ = 1704.9 $cm^{-1}$ et 1684.5 $cm^{-1}$

HRMS- ESI (m/z) : [M+Na] calc. pour $C_{39}H_{51}N_5O_9$, 756.3584 ; exp., 756.3585.

## EXEMPLE 9

**Ester benzylique de l'acide (3S)-4-[2-(6-benzyloxycarbonylamino-hexyl)-2-tert-butoxycarbonyl-hydrazino]-3-*tert*-butoxycarbonylamino-butyrique (IB.1)**

**a) 6-benzyloxycarbonylaminohexan-1-ol (7)**

1 g de 6-aminohexanol (**6**) (8.53 mmoles, 1 éq) et 466 mg $Na_2CO_3$ (4.4 mmoles, 2.2 éq) sont dissous dans 8

mL d'eau et 4 mL de THF. Le milieu est porté à 5°C. 1.33mL de chloroformate de benzyle (9.4 mmoles, 1.1 éq), en solution dans 2.5 mL de THF, sont alors ajoutés goutte à goutte. La réaction est laissée sous agitation durant 2h45 à température ambiante. Le THF est évaporé. Le solide apparu est filtré sous vide, lavé à l'eau, puis séché sous vide pour conduire à 1.848 g de solide blanc (**rendement** 86%)

**RMN $^1$H** 300MHz ($CDCl_3$) : δ (ppm) = 1.20- 1.50 (m, 8H, $CH_2$- ($C\underline{H}_2)_4$- $CH_2$) ; 3.14 (m, 2H, $C\underline{H}_2$- NH) ; 3.55 (m, 2H, $C\underline{H}_2$- OH) ; 4.70 (s, large, 1H, NH) ; 5.05 (s, 2H, $C\underline{H}_2$Ph) ; 7.30 (m, 5H, aromatiques) .

**IR** (KBr) : $\nu_{OH}$ = 3384 $cm^{-1}$ ; $\nu_{NH}$ = 3335 $cm^{-1}$; $\nu_{Csp2\ Harom}$ = 3061 $cm^{-1}$ et 3030 $cm^{-1}$; $\nu_{Csp3}$ = 2942 $cm^{-1}$ et 2859 $cm^{-1}$; $\nu_{C=O}$ = 1685 $cm^{-1}$ ; $\nu_{C=C}$ = 1530 $cm^{-1}$ et 1464 $cm^{-1}$ ; $\nu_{C-O}$ ester = 1290 $cm^{-1}$ et 1253 $cm^{-1}$.

**Point de fusion** = 86°C

**b) N-(*tert*-butoxycarbonyl-amino)phtalimide (9)**

Dans un ballon monocol équipé d'un Dean Stark, sont introduits l'anhydride phtalique (3.36 g, 22.7 mmoles, 1 éq) et le *tert*-butyl carbazate (3 g, 22.7 mmoles, 1 éq) dans 65 mL de toluène. La solution est chauffée au reflux. Après 2h30, le milieu réactionnel est refroidi à température ambiante, puis à 0°C. Le produit précipite et est filtré sous vide. Il est ensuite recristallisé dans de l'acétate d'éthyle pour conduire à 5.6 g d'un solide cristallin blanc translucide (**rendement** 94 %).

**RMN $^1$H** 300MHz ($CDCl_3$) : δ (ppm) = 1.52 (s, 9H, COOC $(CH_3)_3$) ; 6.57 (s, 1H, NH) ; 7.87 (m, 4H, aromatiques) .

**IR** (KBr) : $\nu_{NH}$ = 3323 $cm^{-1}$ ; $\nu_{Csp2\ Harom}$ = 3092 $cm^{-1}$ ; $\nu_{Csp3}$ = 2984 $cm^{-1}$ ; $\nu_{C=O}$ = 1798 $cm^{-1}$ et 1736 $cm^{-1}$; $\nu_{C=C}$ = 1530 $cm^{-1}$.

**Point de fusion** = 188-190°C (litt. : 184-186°C)

**c) N-[(6-benzyloxycarbonyl-aminohexyl)-(tert-butoxycarbonyl)-aminolphtalimide (10)**

Le N- (*tert*- butoxycarbonyl- amino) phtalimide (**9**) (1.86 g, 7.10 mmoles, 1éq), l'aminoalcool protégé (**7**) (1.78 g, 7.10 mmoles, 1éq) et la triphénylphosphine (1.5 éq) sont mis en solution dans le THF anhydre (10mL/ mmol de phtalimide) à 0°C, sous argon. Le 1.5 éq de DIAD sont ajoutés. L'agitation est maintenue à 0°C durant 30 minutes, puis à température ambiante. Le THF est alors évaporé. Le résidu est purifié par chromatographie flash sur gel de silice (AcOEt/EP: 1/1 en volume) pour conduire à 2.71 g d'une huile orange (**rendement** 77 %) .

**RMN $^1$H** 300MHz ($CDCl_3$) : δ (ppm) = 1.10- 1.30 (m, 8H, $CH_2$- ($C\underline{H}_2)_4$- $CH_2$) ; 1.40 (s, 9H, COOC $(C\underline{H}_3)_3$), 3.10 (m, 2H, $C\underline{H}_2$- NH) ; 3.55 (m, 2H, $C\underline{H}_2$- N- N) ; 4.05 (m, 1H, NH) ; 5.05 (s, 2H, $C\underline{H}_2$- Ph) ; 6.30 (s, large, 1H, NH) ; 7.30 (m, 5H, phényl) ; 7.75 (m, 4H, aromatiques phtalyl),

**IR** (NaCl) : $\nu_{NH}$ = 3347 $cm^{-1}$ ; $\nu_{Csp2\ Harom}$ = 3065 $cm^{-1}$ et 3032 $cm^{-1}$ ; $\nu_{Csp3}$= 2980 $cm^{-1}$ et 2936 $cm^{-1}$ ; $\nu_{C=O}$ = 1796 $cm^{-1}$ et 1737 $cm^{-1}$ ; $\nu_{C=C}$ = 1530 $cm^{-1}$ $\nu_{C-O\ ester}$ = 1246 $cm^{-1}$ et 1157 $cm^{-1}$.

**d)1-*tert*-butyloxycarbonyl-1-(6-benzyloxycarbonyl-aminohexyl)hydrazine (III.2)**

Le dérivé phtaloylé (**10**) (1.5 g, 3.03 mmoles, 1éq) est dissous dans de l'EtOH (1mL pour 60 mg). Le milieu est porté à -20°C. Le monohydrate d'hydrazine (1.5 éq) est alors ajouté. Le milieu est laissé sous agitation à - 20°C pendant 30 minutes, puis à température ambiante durant 3h. Le solide blanc apparu est filtré. Le filtrat est évaporé puis purifié par chromatographie flash sur gel de silice (AcOEt/EP: 4/6 en volume) pour conduire à 734 mg d'une huile jaune (**rendement** 67 %).

**RMN $^1$H** 300MHz ($CDCl_3$) : δ (ppm) = 1.15- 1.50 (m, 18H, $CH_2$- ($C\underline{H}_2)_4$- $CH_2$ et COOC $(C\underline{H}_3)_3$) ; 3.10 (q, J= 6.8Hz et J= 6.4Hz, 2H, $C\underline{H}_2$- NHZ) ; 3.28 (t, J= 6.8Hz, 2H, $C\underline{H}_2$- N- $NH_2$) ; 3.85 (s, large, 2H, $N\underline{H}_2$) ; 4.72 (s, large, 1H, N$\underline{H}$Z) ; 5.03 (s, 2H, $C\underline{H}_2$Ph) ; 7.30 (m, 5H, aromatiques) .

**IR** (NaCl) : $\nu_{NH}$ = 3335 $cm^{-1}$; $\nu_{Csp2\ Harom}$ = 3065 $cm^{-1}$ et 3033 $cm^{-1}$ ; $\nu_{Csp3}$ = 2975 $cm^{-1}$ et 2933 $cm^{-1}$; $\nu_{C=O}$ = 1694 $cm^{-1}$; $\nu_{C=C}$ = 1532 $cm^{-1}$ et 1455 $cm^{-1}$; $\nu_{C-O\ ester}$ = 1255 $cm^{-1}$, 1167 $cm^{-1}$ et 1141 $cm^{-1}$.

**e) Ester benzylique de l'acide 3-*tert*-butoxycarbonylamino-4-hydroxybutanoïque (12)**

2 g de Boc- Asp (OBn)- OH (**11**) (6.2 mmoles, 1 éq) sont dissous dans 100 mL de THF anhydre. Le milieu est placé sous argon et porté à- 15°C. 3.5 mL de triéthylamine (24.8 mmoles, 4 éq) et 854 μL de chloroformate d'isobutyle (6.2 mmoles, 1 éq) sont alors ajoutés. Après 20 minutes sous agitation, le milieu réactionnel est coulé goutte à goutte dans une solution de $NaBH_4$ (400 mg, 10.54 mmoles, 1.7 éq) dissous dans 40 mL de THF et 10 mL de MeOH sous argon à- 78°C. Le milieu est laissé sous agitation pendant 2 heures. 150 mL d'une solution d'HCl 1M sont alors ajoutés. La phase aqueuse est extraite 3 fois avec de l'AcOEt. La phase organique est lavée 3 fois avec une solution saturée de $Na_2CO_3$, séchée sur sulfate de sodium anhydre, évaporée puis purifiée par chromatographie flash sur gel de silice (AcOEt/ EP : gradient d'élution 1/3 à 1/1 en volume) pour conduire à 1.043 g d'une huile orange/ marron (**rendement**

56%) .

**RMN $^1$H** 300MHz ($CDCl_3$) : δ (ppm) = 1.38 (s, 9H, COOC ($C\underline{H}_3$)$_3$) ; 2.44 (s, large, 1H, OH) ; 2.62 (d, J= 6.4 Hz, 2H, $C\underline{H}_2$COO) ; 3.62 (s, 2H, $C\underline{H}_2$OH) 3.95 (m, 1H, Chα) ; 5.05 (s, 2H, $C\underline{H}_2$Ph) ; 5.15 (s, large, 1H, NH) ; 7.28 (m, 5H, aromatiques) .

**IR** (NaCl) : $\nu_{NH\ et\ OH}$ = 3380 $cm^{-1}$ ; $\nu_{Csp2\ Harom}$ = 3066 $cm^{-1}$ et 3034 $cm^{-1}$ ; $\nu_{Csp3}$ = 2977 $cm^{-1}$ et 2933 $cm^{-1}$ ; $\nu_{C=O}$ = 1713 $cm^{-1}$; $\nu_{C=C}$ = 1515 $cm^{-1}$ et 1456 $cm^{-1}$ ; $\nu_{C\text{-}O}$ ester = 1249 $cm^{-1}$ et 1166 $cm^{-1}$.

**f) Ester benzylique de l'acide 3-*tert*-b utoxycarbonylamino-4-oxo-butanoïque (V.1)**

A une solution de 1.03 mL de chlorure d'oxalyle (11.75 mmoles, 2.5 éq) dans 9 mL de DCM fraichement distillé, sous argon à -78°C, sont ajoutés goutte à goutte 1.67 mL de DMSO (23.5 mmoles, 5éq). Le milieu est laissé 15 minutes sous agitation dans ces conditions. 1.45 g de composé (**12**) (4.70 mmoles, 1éq), dissous dans 20 mL de DCM fraichement distillé, sont ajoutés. L'agitation est maintenue 1 heure dans les mêmes conditions. 5.9 mL de triéthylamine fraichement distillée (42.3 mmoles, 9 éq) sont ensuite ajoutés goutte à goutte. L'agitation est maintenue durant 30 minutes à -78°C puis 15 minutes à température ambiante. Le milieu est dilué par ajout de DCM et d'$H_2O$. La phase aqueuse est extraite 2 fois avec du DCM. Les phases organiques sont réunies, séchées sur sulfate de sodium anhydre, évaporées et purifiées par chromatographie flash sur gel de silice (AcOEt/EP : 3/7 + 0,1% v/v de triéthylamine) pour conduire à 1.3 g d'une huile jaune pâle (**rendement** 85%).

**RMN $^1$H** 300MHz ($CDCl_3$) : δ (ppm) = 1.45 (s, 9H, COOC ($CH_3$)$_3$) ; 2.90 (dd, J=4.9Hz et J=17.6Hz, 1H, $C\underline{H}_2$COO) ; 3.10 (dd, J=4.9Hz et J=17.7Hz, 1H, $C\underline{H}_2$COO) ; 4.40 (m, 1H, CαH) ; 5.15 (s, 2H, $C\underline{H}_2$Ph) ; 5.62 (d, J=8.3Hz, 1H, NH) ; 7.37 (m, 5H, aromatiques) ; 9, 70 (s, 1H, CHO) .

**IR** (NaCl) : $\nu_{NH}$ = 3376 $cm^{-1}$ ; $\nu_{Csp2\ Harom}$ = 3066 $cm^{-1}$ et 3034 $cm^{-1}$ ; $\nu_{Csp3}$ = 2978 $cm^{-1}$ et 2933 $cm^{-1}$ ; $\nu_{C=O}$ = 1731 $cm^{-1}$ ; $\nu_{C=C}$ = 1499 $cm^{-1}$ et 1455 $cm^{-1}$ ; $\nu_{C=O}$ ester = 1253 $cm^{-1}$ et 1165 $cm^{-1}$.

**[α]$_D$$^{26,6°C}$**=- 3, 88° (0.1 g/L, DCM) .

**g)** 304 mg (0.99 mmole, 1.67 éq) d'aldéhyde (V.**I**) et 216 mg (0.59 mmole, 1 éq) d'aza amino acide (**III. 2**) sont mis en solution dans un mélange MeOH/ AcOH : 99/1 (1mL/ 40mg d'aldéhyde) . 1, 33 éq de cyanoborohydrure de sodium sont ajoutés à cette solution par portions de 5- 6 mg durant 45 minutes. Le milieu est alors laissé sous agitation toute une nuit à température ambiante. Le ballon est ensuite plongé dans un bain de glace et une solution de $NaHCO_3$ saturée (1mL/ 10mg d'aldéhyde) est ajoutée lentement. La phase aqueuse est extraite 3 fois par AcOEt. La phase organique est lavée 3 fois avec une solution saturée de $NaHCO_3$, séchée sur sulfate de sodium anhydre, évaporée puis purifiée par chromatographie flash sur gel de silice (AcOEt/ EP : 3/7 en volume) pour conduire à 265 mg d'une huile jaune (**rendement** 41 %) .

**RMN $^1$H** 300MHz ($CDCl_3$) : δ (ppm) = 1.44 (s, 9H, COOC ($C\underline{H}_3$)$_3$) ; 1.56- 1.46 (m, 17H, $CH_2$- ($C\underline{H}_2$)$_4$- $CH_2$ et COOC ($CH_3$)$_3$) ; 2.65 (m, 2H, $C\underline{H}_2$COOBn) ; 2.95 (m, 2H, $C\underline{H}_2$- NH) ; 3.18 (m, 2H, $C\underline{H}_2$- N- N) ; 3.25 (m, 2H, $C\underline{H}_2$- N- N) ; 4.00 (m, 1H, CHα) ; 4.30 (s, large, 1H, NH) ; 4.85 (s, large, 1H, NH) ; 5.12 (d, J= 6.0Hz, 4H, $C\underline{H}_2$- Ph) ; 5.15 (m, 1H, NH) ; 7.45- 7.30 (m, 10H, aromatiques) . **RMN $^{13}$C** 75MHz (DMSO) : δ (ppm) = 26.23 ; 26.34 ; 28.41 ; 28.28 ; 29.89 ; 37.46 ; 40.98 ; 49.16 ; 53.47 ; 66.60 ; 66.50 ; 80.55 ; 79.55 ; 128.62 ; 128.53 ; 128.33 ; 128.28 ; 128.09 ; 136.74 ; 135.74 ; 155.38; 156.48 ; 156.30 ; 171.30.

**IR** (NaCl) : $\nu_{NH}$ = 3339 $cm^{-1}$ ; $\nu_{Csp2\ Harom}$ = 3065 $cm^{-1}$ et 3033 $cm^{-1}$; $\nu_{Csp3}$ = 2976 $cm^{-1}$ et 2933 $cm^{-1}$; $\nu_{C=O}$ = 1696 $cm^{-1}$; $\nu_{C=C}$ = 1523 $cm^{-1}$ et 1455 $cm^{-1}$; vc-o ester = 1248 $cm^{-1}$ et 1166 $cm^{-1}$.

**[α]$_D$**$^{25°C}$ =- 0.61° (18.9 g/L, DCM)

HRMS- ESI (m/z) : [M+Na] calc. pour $C_{35}H_{52}N_4O_8$, 679.3683 ; exp., 679.3688.

**EXEMPLE 10**

**Ester benzylique de l'acide (3S)-4-[2-(3-benzyloxycarbonylamino-propyl)-2-*tert*-butoxycarbonyl-hydrazino]-3-*tert*-butoxycarbonylamino-4-oxo-butyrique (IB.2)**

**a)1-*tert*-butoxycarbonyl-1-(3-benzyloxycarbonyl-aminopropyl)hydrazine (III.5)**

Le composé (**III. 5**) est obtenu selon un mode opératoire identique à celui suivi pour la synthèse de (**III. 2**) en utilisant de la *tert*- butoxycarbonyl- hydrazine et du 3- (benzyloxycarbonyl- amino) propanol comme produits de départ et en subtituant le DIAD par du DEAD au cours de la réaction de Mitsunobu.

**RMN $^1$H** 300MHz ($CDCl_3$) : δ (ppm) = 1.50 (s, 9H, COOC ($C\underline{H}_3$)$_3$) ; 1.80 (quint, J=19.2Hz, J=13.2Hz et J=6.4Hz, 2H, $CH_2$- $C\underline{H}_2$- $CH_2$) ; 3, 25 (q, J=6.4Hz et J=12.8Hz, 2H, $C\underline{H}_2$NH) ; 3.50 (t, J=6.4Hz, 2H, N- N- $C\underline{H}_2$) ; 5, 10 (s, 2H, $COOC\underline{H}_2$- Ph) ; 6.50 (s, large, 1H, NH) ; 7.40 (m, 5H, phényl) .

**IR** (NaCl) : $\nu_{NH}$ = 3335 $cm^{-1}$ ; $\nu_{Csp2\ Harom}$ = 3066 $cm^{-1}$ et 3035 $cm^{-1}$ ; $\nu_{Csp3}$ = 2977 $cm^{-1}$ et 2934 $cm^{-1}$ ; $\nu_{C=O}$ = 1694 $cm^{-1}$ ; $\nu_{C=O\ ester}$ = 1250 $cm^{-1}$ et 1143 $cm^1$.

**b)** A une solution d'acide aspartique (20 mg, 0.17 mmole, 1.1 éq) dans du DMF (2 mL/ 0.1 mmol d'acide aspartique) sont ajoutés 1.3 éq de HOBt puis 1.3 éq d'EDCI. L'aza- amino acide (**III. 5**) (50 mg, 0.15 mmole, 1 éq) est ensuite ajouté. La solution est agitée à température ambiante toute une nuit. La solution est diluée dans de l'acétate d'éthyle puis lavée trois fois avec une solution saturée de $NaHCO_3$, puis d'HCl 1N. La phase organique est séchée sur sulfate de sodium anhydre puis est évaporée. Le résidu est chromatographié sur gel de silice (MeOH/ DCM : 1/99 en volume) pour conduire à 20mg d'une huile jaune clair (**rendement** 20%) .

**RMN $^1$H** 300MHz ($CDCl_3$) : δ (ppm) = 1.40 (s, 9H, COOC ($C\underline{H}_3)_3$) ; 1.45 (s, 9H, COOC ($C\underline{H}_3)_3$) ; 1.70 (m, 2H, $CH_2$- $C\underline{H}_2$- $CH_2$) ; 2, 80 (dd, J=17.5Hz et J=4.5Hz, 1H, $C\underline{H}_2$COOBn) 3.00 (dd, J=17.5Hz et J=4.5Hz, 1H, $C\underline{H}_2$COOBn) ; 3.30 (m, 2H, $C\underline{H}_2$NH) ; 3.50 (m, 2H, N- N- $C\underline{H}_2$) ; 4.60 (m, 1H, CαH) ; 5.12 (s, 2H, $C\underline{H}_2$Ph) ; 5.15 (s, 2H, $C\underline{H}_2$Ph) ; 5.62 (d, 1H, NH) ; 7.40 (m, 10H, aromatiques) ; 8.40 (s, 1H, CONHN) .

**RMN $^{13}$C** 75MHz ($CDCl_3$) : 27.96 (1C, $CH_2$- $\underline{C}H_2$- $CH_2$- NHZ) ; 28.50 (3C, COOC ($\underline{C}H_3)_3$) ; 28.69 (3C, COOC ($\underline{C}H_3)_3$) ; 36.20 (1C, $\underline{C}H_2$ asp) ; 38, 39 (1C, $CH_2$- $CH_2$- $CH_2$NHZ) ; 47.00 (1C, N- N- $\underline{C}H_2$- $CH_2$- $CH_2$- NHZ) ; 49.73 (1C, CαH) ; 66.94 (1C, $\underline{C}H_2$- PH) ; 67.38 (1C, $\underline{C}H_2$- PH) ; 77.04 (1C, COO$\underline{C}$ ($CH_3)_3$) ; 77.88 (1C, COO$\underline{C}$ ($CH_3)_3$) ; 128.39 (2C, CHar) ; 128.48 (2C, CHar) ; 128.68 (1C, CHar) ; 128.86 (1C, CHar) ; 129.02 (2C, CHar) ; 129.14 (2C, CHar) ; 135.67 (1C, $C^{IV}$ar) ; 137.12 (1C, $C^{IV}$ar) ; 153.25 (NH$\underline{C}$OO) ; 155.98 (1C, NH$\underline{C}$OO) ; 157.04 (1C, $CH_2\underline{C}$OO) ; 169.80 (1C, $\underline{C}$ONHN) ; 171.88 (1C, $\underline{C}$ONNH) .

$[\alpha]_D^{22,6°C}$= +7.14° (2.8 g/L, DCM)

HRMS- ESI (m/z) : [M+Na] calc. pour $C_{32}H_{44}N_4O_9$, 651.3006 ; exp., 651.3010.

## EXEMPLE 11

### Ester benzylique de l'acide (35)-4-[2-(4-benzyloxycarbonylamino-phényl)-2-*tert*-butoxycarbonyl-hydrazino]-3-*tert*-butoxycarbonylamino-4-oxo-butyrique (IB.3)

### a) Ester benzylique de l'acide (4-iodo-phényl)-carbamique (5)

0.5 g de 4-iodoaniline (**4**) (2.28 mmoles, 1 éq) sont dissous dans 20 mL de DCM. On additionne 1 mL de triéthylamine distillée et on refroidit le mélange à 0°C. Sous agitation, on ajoute goutte à goutte une solution de chloroformiate de benzyle (1.95 mL, 13.7 mmoles, 6 éq) dans 20 mL de DCM. Une fois l'addition terminée, on laisse un quart d'heure à 0°C puis on remonte à température ambiante. Le milieu réactionnel est ensuite chauffé à reflux durant 20h. Après retour à température ambiante, le mélange réactionnel est jeté dans une solution 1M d'HCl. Après dilution par du DCM, la phase organique est récupérée, lavée avec une solution de $Na_2CO_3$ saturée, séchée sur $Na_2SO_4$, filtrée et évaporée à sec. Le produit brut obtenu est chromatographié sur gel de silice avec pour éluant un mélange DCM/E.P. (3 :2 en volume). On obtient une poudre blanche (m=460mg) (**rendement** 60%).

**RMN $^1$H** 300MHz (DMSO) : δ (ppm) = 5.14 (s, 2H, $\underline{CH_2}$- $C_6H_5$) ; 7.28- 7.69 (m, 9H, $H_{arom}$) ; 9.89 (s, 1H, NH) .

**IR** (KBr) : $\nu_{NH}$ = 3322 $cm^{-1}$ ; $\nu_{Csp2\ Harom}$ = 3034 $cm^{-1}$ ; $\nu_{C=O}$ = 1701 $cm^{-1}$ ; $\nu_{C=C}$ = 1528 $cm^{-1}$; $\nu_{C-O}$ ester = 1236 $cm^{-1}$.

**Point de fusion** = 130°C

### b) Ester *tert*-butytique de l'acide 1-(4-benzyloxycarbonylaminophényl)hydrazine carboxylique (III.1)

Dans un ballon, on introduit le DMF distillé (4.5 mL). Quatre cycles de « congélation-vide-décongélation » sont effectués. Après passage sous atmosphère d'argon, 1 g de composé (**5**) (2.83 mmoles, 1 éq), 54 mg de CuI (0.283 mmoles, 0.1 éq), 51 mg de 1,10-phénanthroline (0.283 mmoles, 0.1 éq), 1.29 g de $Cs_2CO_3$ (3.96 mmoles, 1.4éq) et 450 mg de *tert*-butylcarbazate (3.39 mmoles, 1.2 éq) sont introduits. Quatre cycles de « congélation-vide-décongélation » sont à nouveau effectués, le milieu est placé sous atmosphère d'argon et chauffé à 80°C. Le flux d'argon est arrêté au bout de 20 min. La réaction est laissée à 80°C durant 18h. Après retour à température ambiante le milieu est filtré sur un fritté à travers une couche d'un centimètre d'épaisseur de silice en effectuant des rinçages à l'acétate d'éthyle. L'acétate d'éthyle et le DMF sont évaporés pour donner un produit brut qui est chromatographié sur gel de silice avec un gradient d'éluant E.P/AcOEt de (2 :1) à (1 :1) en volume. 100 mg d'une poudre jaune paille sont obtenus (**rendement** 10%).

**RMN $^1$H** 300MHz ($CDCl_3$) : δ (ppm) = 1.43 (s, 9H, C ($CH_3)_3$) ; 5.00 (sl, 2H, $NH_2$) ; 5.14 (s, 2H, $\underline{CH_2}$- $C_6H_5$) ; 7.29- 7.55 (m, 9H, $H_{arom}$) ; 9.7 (s, 1H, NH) . **RMN $^{13}$C** 75MHz ($CDCl_3$) : δ (ppm) = 28.63 ; 28.72 ; 28.76 ; $\overline{68.36}$ ; 71.73 ; 113.21 ; 113.3 ; 117.43 ; 122.06 ; 122.13 ; 129.63 ; 129.74 ; 129.98 ; 131.16 ; 138.04 ; 138.94 ; 153.54 ; 155.92.

**IR** (KBr) : $\nu_{NH}$ = 3323 $cm^{-1}$ ; $\nu_{Csp3}$ = 2978 $cm^{-1}$ ; $\nu_{C=O}$ = 1699 $cm^{-1}$ et 1603 $cm^{-1}$ ; $\nu_{C=C}$ = 1538 $cm^{-1}$. **c)** Selon un mode opératoire identique à celui décrit pour le composé (**IB. 2**), 100 mg d'aza- amino acide (**III. 1**) (0.27 mmole, 1 éq) et 100 mg de Boc- Asp (OBn)- OH (0.31 mmole, 1.1 éq) conduisent, après purification par chromatographie flash sur gel de silice avec comme éluant un mélange AcOEt/E.P. (1: 1 en volume), à 120 mg d'une poudre jaune paille

(**rendement** 64%) .

**RMN $^1$H** 300MHz (DMSO) : δ (ppm) = 1.4 (s, 18H, C $(CH_3)_3$) ; 4.1 (m, 2H, $CH_2\beta$) ; 4.41 (m, 1H, CHα) ; 5.18 (s, 2H, $CH_2$- $C_6H_5$) ; 5.76 (s, 2H, $CH_2$- $C_6H_5$) ; 7.2- 7.6 (m, 14H, $H_{arom}$) ; 9.8 (s, 1H, NH) ; 10.6 (s, 1H, NH) .

**RMN $^{13}$C** 75MHz ($CDCl_3$) : δ (ppm) = 14.6 ;, 21.46 ;, 28.28 ; 28.50 ; 28.69 ; 28.75 ; 30.1 ; 36.24 ; 49.74 ; 60.84 ; 67.36 ; 77.10 ; 77.52 ; 77.95 ; 81.15 ; 82.73 ; 84.32 ; 119.19 ; 125.29 ; 128.69 ; 128.71 ; 128.79 ; 129.00 ; 135.73 ; 136.27 ; 136.50 ; 137.26 ; 150.52 ; 153.36 ; 153.82 ; 156.04 ; 169.54 ; 170.55 ; 171.66 ; 171.8.

**IR** (KBr) : $\nu_{NH}$ = 3347 $cm^{-1}$ ; $\nu_{Csp3}$ = 2980 $cm^{-1}$ $\nu_{C=O}$ = 1721 $cm^{-1}$, 1703 $cm^{-1}$ et 1676 $cm^{-1}$ ; $\nu_{C\text{-}O\ ester}$ = 1162 $cm^{-1}$.

**Point de fusion** = 109°C.

HRMS- ESI (m/z) : [M+Na] calc. pour $C_{35}H_{42}N_4O_9$, 685.2849 ; exp., 685.2854.

**EXEMPLE 12**

**Ester benzylique de l'acide (4S)-5-[2-(4-benzyloxycarbonylamino-phényl)-2-*tert*-butoxycarbonyl-hydrazino]-4-*tert*-butoxycarbonylamino-5-oxo-pentanoïque (IB.4)**

Selon un mode opératoire identique à celui décrit pour le composé (**IB. 2**), 100 mg d'aza- amino acide (**III. 1**) (0.27 mmole, 1 éq) et 105 mg de Boc- Glu (OBn)- OH (0.31mmole, 1.1 éq) conduisent, après purification par chromatographie flash sur gel de silice avec comme éluant un mélange AcOEt/E.P. (1: 1 en volume), à 60 mg d'une poudre jaune paille (**rendement** 32%) .

**RMN $^1$H** 300MHz ($CDCl_3$) : δ (ppm) = 1.46 (s, 9H, C $(CH_3)$ ) ; 1.48 (s, 9H, C $(CH_3)$ ) ; 1.7 (m, 2H, $CH_2$) ; 2.6 (m, 2H, $CH_2$) ; 4.3 (m, 1H, CHα) ; 5.15 (s, 2H, $CH_2$- $C_6H_5$) ; 5.21 (s, 2H, $CH_2$- $C_6H_5$) ; 6.7 (s, 1H, NH) ; 7.3- 7.5 (m, 14H, $H_{arom}$) ; 8.6 (s, 1H, NH) ._

**RMN $^{13}$C** 75MHz ($CDCl_3$) : δ (ppm) = 28.04 ; 28.53 ; 28.68 ; 30.11 ; 30.74 ; 52.26 ; 65.71 ;, 67.03 ; 67.44 ; 77.03 ; 77.45 ; 77.65 ; 77.87 ; 80.86 ; 82.83 ; 119.21 ; 125.48 ; 127.4 ; 128.67 ; 128.73 ; 128.76 ; 129.02 ; 136.06 ; 136.23 ; 136.40 ; 137.40 ; 138.14 ; 153.43 ; 153.77 ; 156.26 ; 171.28 ; 173.55.

**IR** (KBr) : $\nu_{NH}$ = 3350 $cm^{-1}$; $\nu_{Csp3}$ = 2981 $cm^{-1}$; $\nu_{C=O}$ = 1703 $cm^{-1}$ 1724 $cm^{-1}$ et 1676 $cm^{-1}$; $\nu_{C=C}$ = 1530 $cm^{-1}$ ; $\nu_{C\text{-}O}$ ester= 1164 $cm^{-1}$.

**Point de fusion** = 115°C.

HRMS- ESI (m/z) : [M+Na] calc. pour $C_{36}H_{44}N_4O_9$, 699.3006 ; exp., 699.3010.

**EXEMPLE 13**

**Ester benzylique de l'acide (3S)-4-[2-(5-benzyloxycarbonylamino-pentyl)-2-*tert*-butoxycarbonyl-hydrazino]-3-tert-butoxycarbonylamino-4-oxo-butyrique (IB.5)**

**a) Ester *tert*-butylique de l'acide 1-(5-Benzyloxycarbonylamino-pentyl)-hydrazinecarboxylique (III.6)**

Le composé (**III. 6**) est obtenu selon un mode opératoire identique à celui suivi pour la synthèse de (**III. 2**) en utilisant de la *tert*- butoxycarbonyl- hydrazine et du 5- (benzyloxycarbonyl- amino) pentanol comme produits de départ et en subtituant le DIAD par du DBAD au cours de la réaction de Mitsunobu.

**RMN $^1$H** 300MHz ($CDCl_3$) : δ (ppm) = 1.40- 1.70 m, 15H, $CH_2$- $(CH_2)_3$- $CH_2$ et COOC $(CH_3)_3$) ; 3.20 (q, J= 6.8Hz et J= 6.4Hz, 2H, $CH_2$- NHZ) ; 3.35 (t, J= 7, . 2Hz, 2H, $CH_2$- N- N) ; 4.00 (s, 2H, $NH_2$) ; 4.80 (s, large, 1H, NHZ) ; 5.10 (s, 2H, $CH_2Ph$) ; 7.36 (m, 5H, aromatiques) .

**IR** (NaCl) : $\nu_{NH}$ = 3340 $cm^{-1}$ ; $\nu_{Csp2\ Harom}$ = 3065 $cm^{-1}$ et 3033 $cm^{-1}$ ; $\nu_{Csp3}$ = 2975 $cm^{-1}$ et 2933 $cm^{-1}$; $\nu_{C=O}$ = 1694 $cm^{-1}$ ; $\nu_{C\text{-}O\ ester}$ = 1251 $cm^{-1}$ et 1143 $cm^1$.

**b)** Selon un mode opératoire identique à celui décrit pour le composé (**IB. 2**), 120 mg d'aza- amino acide (**III. 6**) (0.34 mmole, 1 éq) et 120 mg de Boc- Asp (OBn)- OH (0.37 mmole, 1.1 éq) conduisent, après purification par chromatographie flash sur gel de silice avec comme éluant un mélange AcOEt/E.P. (4: 6 en volume), à 136 mg d'une huile jaune (**rendement** 61%) . **RMN $^1$H** 300MHz ($CDCl_3$) : δ (ppm) = 1.10- 1.30 (m, 24H, $CH_2$- $(CH_2)_3$- $CH_2$ et COOC $(CH_3)_3$) ; 2.66- 2.94 (m, 2H, $CH_2COOBn$) ; 3.12 (m, 2H, $CH_2$- NHZ) ; 3.36 (t, J= 6.4Hz, 2H, $CH_2$- N- N) ; 4.50 (s, large, 1H, CHα) ; 4.85 (s, large, 1H, NH) ; 5.05 (d, J= 12.8Hz, 4H, $CH_2$- Ph) ; 5.60 (s, large, 1H, NH) ; 7.30 (m, 10H, aromatiques) ; 8.30 (s, 1H, CO- NH- N) .

**RMN $^{13}$C** 75MHz ($CDCl_3$) : δ (ppm) = 26, 19 ; 27.27 ; 28.20 ; 28.33 ; 29.73 ; 35.91 ; 40.83 ; 49.36 ; 66.60 ; 66.95 ; 80.76 ; 81.27 ; 128.08 ; 128.28 ; 128.44 ; 128.53 ; 128.64 ; 135.38 ; 136.75 ; 154.67 ; 155.59 ; 156.5 ; 171.51.

**IR** (NaCl) : $\nu_{NH}$ = 3323 $cm^{-1}$ ; $\nu_{Csp2\ Harom}$ = 3034 $cm^{-1}$ ; $\nu_{Csp3}$ = 2978 $cm^{-1}$ et 2934 $cm^{-1}$ ; $\nu_{C=O}$ = 1694 $cm^{-1}$ ; $\nu_{C=O\ ester}$ = 1250 $cm^{-1}$ et 1161 $cm^{-1}$.

$[\alpha]_D^{24,3°C}$ =- 13° (10g/L, DCM)

HRMS- ESI (m/z) : [M+Na] calc. pour $C_{34}H_{48}N_4O_9$, 679.3319 ; exp., 679.3329.

**EXEMPLE 14**

**Ester benzylique de l'acide 4-[2-((2S)-3-benzyloxycarbonyl-2-*tert*-butoxycarbonylamino-propyl)-1-*tert*-butoxycarbonyl-hydrazinométhyl]-pipéridine-1-carboxylique (IB.6)**

**a) Ester benzylique de l'acide 4-(1-*tert*-Butoxycarbonyl-hydrazinométhyl)-pipéridine-1-carboxylique (III.7)**

Le composé (**III. 7**) est obtenu selon un mode opératoire identique à celui suivi pour la synthèse de (**III. 2**) en utilisant de la *tert*- butoxycarbonyl- hydrazine et de l'ester benzylique de l'acide 4- hydroxyméthyl- pipéridine- 1- carboxylique comme produits de départ et en subtituant le DIAD par du DBAD au cours de la réaction de Mitsunobu.
**RMN $^1$H** 300MHz ($CDCl_3$) : δ (ppm) = 1.38 (s, 9H, COOC ($CH_3$)$_3$) ; 1.50- 1.65 (m, 4H, $CH_2$- $CH_2$- N) ; 1.82 (m, 1H, CHα) ; 2.70 (m, 2H, $CH_2$- N- $NH_2$) ; 3.18 (d, J= 7, 2Hz, 2H, $CH_2$- $CH_2$- N) ; 3.90 (s, 2H, $NH_2$) ; 4.10 (s, 2H, $CH_2$- $CH_2$- N) ; 5.05 (s, 2H, $CH_2$Ph) ; 7.30 (m, 5H, aromatiques) .
**IR** (NaCl) : $\nu_{NH}$ = 3500 $cm^{-1}$ et 3334 $cm^{-1}$ ; $\nu_{Csp2\ Harom}$ = 3065 $cm^{-1}$; $\nu_{Csp3}$ = 2974 $cm^{-1}$ et 2927 $cm^{-1}$; $\nu_{C=O}$ = 1694 $cm^{-1}$ et 1682 $cm^{-1}$; $\nu_{C\text{-}O\ ester}$ = 1278 $cm^{-1}$ et 1220 $cm^{-1}$.
**b)** Selon un mode opératoire identique à celui décrit pour le composé (**IB. 1**), 116 mg d'aza- amino acide (**III. 7**) (0.34 mmole, 1 éq) et 162 mg de composé (**V. 1**) (0.53 mmole, 1.5 éq) conduisent, après purification par chromatographie flash sur gel de silice avec comme éluant un mélange AcOEt/ Cyclohexane (3: 7 en volume), à 167 mg d'une huile jaune (**rendement** 80%) .
**RMN $^1$H** 300MHz ($CDCl_3$) : δ (ppm) = 1.30- 1.40 (m, 18H, COOC ($CH_3$)$_3$) ; 1.50- 1.70 (m, 4H, $CH_2$- $CH_2$- N) ; 1.78 (m, 1H, CHα) ; 2.55 (d, J= 5, 7Hz, 2H, $CH_2$- COOBn) ; 2.70 (m, 2H, $CH_2$- NH- N) ; 2.90 (m, 2H, $CH_2$- N- NH) ; 3.10 (m, 2H, $CH_2$- $CH_2$- N) ; 3.90 (s, large, 1H, NH) ; 4.08 (m, 2H, $CH_2$- $CH_2$- N) ; 5.05 (m, 4H, $CH_2$Ph) ; 5.10 (m, 1H, NH) ; 7.28 (m, 10H, aromatiques) .
**RMN $^{13}$C** 75MHz ($CDCl_3$) : δ (ppm 26.95 ; 28.27 ; 28.42 ; 29.68 ; 34.73 ; 37.47 ; 43.89 ; 46.70 ; 53.25 ; 54.34 ; 66.57 ; 67.07 ; 80.89 ; 127.89 ; 128.31 ; 128.40 ; 128.52 ; 128.65 ; 135.57 ; 136.93 ; 155.34 ; 155.41 ; 170.63 ; 171.25.
**IR** (NaCl) : $\nu_{NH}$ = 3334 $cm^{-1}$ ; $\nu_{Csp2\ Harom}$ = 3091 $cm^{-1}$ et 3033 $cm^{-1}$ ; $\nu_{Csp3}$ = 2976 $cm^{-1}$ et 2929 $cm^{-1}$ ; $\nu_{C=O}$ = 1695 $cm^{-1}$ ; $\nu_{C\text{-}O\ ester}$ = 1278 $cm^{-1}$ et 1249 $cm^{-1}$.
$[\alpha]_D^{25°C}$=- 0, 80° (7, 1 g/L, DCM) .
HRMS- ESI (m/z) : [M+Na] calc. pour $C_{35}H_{50}N_4O_8$, 677.3526 ; exp., 677.3532.

**EXEMPLE 15**

**Ester benzylique de l'acide 4-[2-((2S)-3-benzyloxycarbonyl-2-*tert*-butoxycarbonylamino-propionyl)-1-*tert*-butoxycarbonyl-hydrazinométhyl]-pipéridine-1-carboxylique (IB.7)**

Selon un mode opératoire identique à celui décrit pour le composé (**IB. 2**), 145 mg d'aza- amino acide (**III. 7**) (0.45 mmole, 1 éq) et 160 mg de Boc- Asp (OBn)- OH (0.49 mmole, 1.1 éq) conduisent, après purification par chromatographie flash sur gel de silice avec comme éluant un mélange AcOEt/ Cyclohexane (3: 7 en volume), à 210 mg d'une huile jaune (**rendement** 77%) .
**RMN $^1$H** 300MHz ($CDCl_3$) : δ (ppm) = 1.45 (m, 18H, COOC ($CH_3$)$_3$) ; 1.60- 1.80 (m, 5H, CHα et $CH_2$- $CH_2$- N) ; 2.70- 3.05 (m, 4H, $CH_2$- COOBn et $CH_2$- N- NH) ; 3.35 (m, 2H, $CH_2$- $CH_2$- N) ; 4.20 (m, 2H, $CH_2$- $CH_2$- N) ; 4.55 (s, large, 1H, CHα) ; 5.15 (d, J= 6.4Hz, 4H, $CH_2$Ph) ; 5.68 (s, large, 1H, NH) ; 7.38 (m, 10H, aromatiques) ; 8.30 (s, 1H, CONHN) .
**RMN $^{13}$C** 75MHz ($CDCl_3$) : δ (ppm) = 26.96 ; 28.18 ; 28.34 ; 29.69 ; 34.85 ; 43.85 ; 49.39 ; 55.00 ; 67.04 ; 80.87 ; 81.57 ; 127.88 ; 127.97 ; 128.26 ; 128.51 ; 128.66 ; 135.34 ; 136.96 ; 155.29 ; 155.66 ; 171.54.
**IR** (NaCl) : $\nu_{NH}$ = 3306 $cm^{-1}$ ; $\nu_{Csp2\ Harom}$ = 3034 $cm^{-1}$ ; $\nu_{Csp3}$ = 2922 $cm^{-1}$ et 2853 $cm^{-1}$ ; $\nu_{C=O}$ = 1701 $cm^{-1}$.
$[\alpha]_D^{25°C}$ =- 19, 31° (14, 1g/L, DCM)
HRMS- ESI (m/z) : [M+Na] calc. pour $C_{35}H_{48}N_4O_9$, 691.3319 ; exp., 691.3322.

**EXEMPLE 16**

**Ester benzylique de l'acide (3S)-4-[2-(5-benzyloxycarbonylamino-pentyl)-2-*tert*-butoxycarbonyl-hydrazino]-3-tert-butoxycarbonylamino-butyrique (IB.8)**

Selon un mode opératoire identique à celui décrit pour le composé (**IB. 1**), 192 mg d'aza- amino acide (**III. 6**) (0.54 mmole, 1 éq) et 280 mg de composé (**V. 1**) (0.91 mmole, 1.6 éq) conduisent, après purification par chromatographie flash sur gel de silice avec comme éluant un mélange AcOEt/E.P. (3: 7 en volume), à 295 mg d'une huile jaune (**rendement** 85%) . **RMN $^1$H** 300MHz ($CDCl_3$) : δ (ppm) = 1.32- 1.40 (m, 18H, COOC ($CH_3$)$_3$) ; 1.41- 1.52 (m, 6H, $CH_2$- ($CH_2$)$_3$-

$CH_2$) ; 2.60 (m, 2H, $CH_2COOBn$) ; 2.95 (m, 2H, $C\underline{H_2}$- NH) ; 3.12 (m, 2H, $C\underline{H_2}$- N- N) 3, 25 (m, 2H, $C\underline{H_2}$- N- N) ; 3.95 (m, 1H, CHα) ; 4.65 (s, large, 1H, NH) ; 4.80 (s, large, 1H, NH) ; 5.05 (d, J= 7, 9Hz, 4H, $C\underline{H_2}$- Ph) ; 5.15 (s, 1H, large, NH) ; 7.20- 7.35 (m, 10H, aromatiques) . **RMN $^{13}$C** 75MHz ($CDCl_3$) : δ (ppm) = 23.77 ; 28.29 ; 28.41 ; 28.45 ; 29.57 ; 37.42 ; 40.98 ; 49.08 ; 53.46 ; 66.51 ; 66.62 ; 79.56 ; 80.63 ; 128.10 ; 128.20 ; 128.29 ; 128.34 ; 128.53 ; 128.62 ; 135.74 ; 136.71 ; 155.38 ; 156.50 ; 171.14 ; 171.31.

**IR** (NaCl) : $\nu_{HH}$ = 3344 $cm^{-1}$ ; $\nu_{Csp2\ Harom}$ = 3065 $cm^{-1}$ et 3034 $cm^{-1}$ ; $\nu_{Csp3}$ = 2977 $cm^{-1}$ et 2934 $cm^{-1}$ ; $\nu_{C=O}$ = 1694 $cm^{-1}$ ; vc=c = 1520 $cm^{-1}$ et 1455 $cm^{-1}$ ; $\nu_{C\text{-}O\ ester}$ = 1250 $cm^{-1}$ et 1167 $cm^{-1}$.

**$[\alpha]_D$**$^{25°C}$ =- 1, 04° (18, 2g/L, DCM) .

HRMS- ESI (m/z) : [M+Na] calc. pour $C_{34}H_{50}N_4O_8$, 665.3526 ; exp., 665.3527.

## EXEMPLE 17

### Ester benzylique de l'acide (3S)-4-[2-(6-benzyloxycarbonylamino-hexyl)-2-*tert*-butoxycarbonyl-hydrazino]-3-*tert*-butoxycarbonylamino-4-oxo-butyrique (IB.9)

Selon un mode opératoire identique à celui décrit pour le composé (**IB. 2**), 120 mg d'aza- amino acide (**III. 2**) (0.55 mmole, **1** éq) et 196 mg de Boc- Asp (OBn)- OH (0.60 mmole, 1.1 éq) conduisent, après purification par chromatographie flash sur gel de silice avec comme éluant un mélange AcOEt/E.P. (4: 6 en volume), à 297 mg d'une huile jaune (**rendement** 80%) . **RMN $^1$H** 300MHz ($CDCl_3$) : δ (ppm) = 1.15- 1.30 (m, 26H, $CH_2$- $(CH_2)_4$- $CH_2$ et COOC $(C\underline{H_3})_3$) ; 2.60- 2.96 (m, 2H, $CH_2COOBn$) ; 3.10 (m, 2H, $C\underline{H_2}$- NHZ) ; 3.38 (m, 2H, $C\underline{H_2}$- N- N) ; 4.50 (s, large, 1H, CHα) ; 4.80 (s, large, 1H, NH) ; 5.05 (d, 4H, $CH_2Ph$) ; 5.65 (s, large, 1H, NH) ; 7.30 (m, 10H, aromatiques) ; 8.20 (s, 1H, CON$\underline{H}$N).

**RMN $^{13}$C** 75MHz ($CDCl_3$) : δ (ppm) = 23.43 ; 26.83 ; 28.20 ; 28.33 ; 29.44 ; 40.48 ; 49.44 ; 66.65 ; 66.95 ; 80.73 ; 81.31 ; 128.07 ; 128.13 ; 128.29 ; 128.43 ; 128.52 ; 128.64 ; 135.39 ; 136.71 ; 154.71 ; 156.63 ; 171.47.

**IR** (NaCl) : $v_{NH}$ = 3320 $cm^{-1}$ ; $v_{Csp2\ Harom}$ = 3034 $cm^{-1}$ ; $v_{Csp3}$ = 2977 $cm^{-1}$ et 2933 $cm^{-1}$ ; $v_{C=O}$ = 1691 $cm^{-1}$ ; $v_{C=C}$ = 1524 $cm^{-1}$ et 1455 $cm^{-1}$; $v_{C\text{-}O}$ ester = 1250 $cm^{-1}$ et 1159 $cm^{-1}$.

**$[\alpha]_D$**$^{24,9°C}$ =- 10° (10g/L, DCM) .

HRMS- ESI (m/z) : [M+Na] calc. pour $C_{35}H_{50}N_4O_9$, 693.3475 ; exp., 693.3669.

## EXEMPLE 18

### Ester benzylique de l'acide (3S)-3-*tert*-butoxycarbonylamino-4-[2-*tert*-butoxycarbonyl-2-(6-*tert*-butoxycarbonylamino-hexyl)-hydrazino]-butyrique (IB.10)

#### a) 1-*tert*-butoxycarbonyl-1-(6-*tert*-butoxycarbonyl-aminohexyl)hydrazine (III.8)

Le composé (**III. 8**) est obtenu selon un mode opératoire identique à celui suivi pour la synthèse de (**III. 2**) en utilisant de la *tert*- butoxycarbonyl- hydrazine et du 6- (Boc- amino) hexanol comme produits de départ et en subtituant le DIAD par du DBAD au cours de la réaction de Mitsunobu. **RMN $^1$H** 300MHz ($CDCl_3$) : δ (ppm) = 1.36 (s, 9H, COO $(\underline{CH_3})_3$) ; 1.39 (s, 9H, COO $(\underline{CH_3})_3$) ; 1.19- 1.48 (m, 8H, $CH_2$- $(\underline{CH_2})_4$- $CH_2$) ; 2.89 (q, 2H, $\underline{CH_2}$- NHBoc) ; 3.21 (t, 2H, $CH_2$- N- $NH_2$) ; 4.38 (s, 2H, $NH_2$) ; 6.76 (s, 1H, NH) .

**IR** (KBr) : $v_{NH}$ = 3342 $cm^{-1}$ ; $v_{Csp3}$ = 2976 $cm^{-1}$ et 2932 $cm^{-1}$ ; $v_{C=O}$ = 1690 $cm^{-1}$; $v_{C=C}$ = 1525 $cm^{-1}$; $v_{C=O\ ester}$ = 1250 $cm^{-1}$ et 1170 $cm^{-1}$.

**b)** Selon un mode opératoire identique à celui décrit pour le composé (**IB. 1**), 130 mg d'aza- amino acide (**III. 8**) (0.4 mmole, 1 éq) et 200 mg de composé (**V. 1**) (0.7 mmole, 1.67 éq) conduisent, après purification par chromatographie flash sur gel de silice avec comme éluant un mélange AcOEt/ Cyclohexane (3: 7 en volume), à 190 mg d'une huile incolore.

**RMN $^1$H** 300MHz ($CDCl_3$) : δ (ppm) = 1.35 (s, 9H, COO $(\underline{CH_3})_3$) ; 1.38 (s, 9H, COO $(\underline{CH_3})_3$) ; 1.39 (s, 9H, COO $(\underline{CH_3})_3$) ; 1.23- 1.45 (m, 8H, $CH_2$- $(\underline{CH_2})_4$- $CH_2$) ; 2.63- 2.83 (m, 4H, $\underline{CH_2}$- NHZ et $CH_2COOBn$) ; 2.88 (m, 2H, $CH_2$- NH- N) ; 3.18 (m, 2H, $CH_2$- N- NH) ; 3.87 (m, 1H, CHα) ; 5.05 (s, 2H, $CH_2Ph$) ; 6.75 (m, 1H, NH) ; 7.36- 7.30 (m, 5H, $H_{arom}$) .

**IR** (NaCl) : $v_{NH}$ = 3353 $cm^{-1}$ ; $v_{Csp2\ Harom}$ = 3065 $cm^{-1}$ et 3027 $cm^{-1}$ ; $v_{Csp3}$ = 2977 $cm^{-1}$ et 2932 $cm^{-1}$; $v_{C=O}$ = 1694 $cm^{-1}$ ; $v_{C\text{-}O}$ ester = 1250 $cm^{-1}$ et 1167 $cm^1$.

HRMS- ESI (m/z) : [M+Na] calc. pour $C_{32}H_{54}N_4O_8$, 645.3839 ; exp., 645.3834.

**EXEMPLE 19**

**Ester benzylique de l'acide (3S)-4-[2-benzyloxycarbonyl-2-(6-benzyloxycarbonylamino-hexyl)-hydrazino]-3-*tert*-butoxycarbonylamino-butyrique (IB.11)**

Selon un mode opératoire identique à celui décrit pour le composé (**IB.1**), 130 mg d'aza-amino acide (**III.3**) (0.4 mmole, 1 éq) et 200 mg de composé (**V.1**) (0.7 mmole, 1.67 éq) conduisent, après purification par chromatographie flash sur gel de silice avec comme éluant un mélange AcOEt/Cyclohexane (3:7 en volume), à 120 mg d'un solide blanc.
**RMN $^1$H** 300MHz ($CDCl_3$) : $\delta$ (ppm) = 1.35 (s, 9H, COO $(\underline{CH_3})_3$) ; 1.30- 1.46 (m, 8H, $CH_2$- $(\underline{CH_2})_4$- $CH_2$) ; 2.52- 2.83 (m, 4H, $\underline{CH_2}$- NHZ et $CH_2COOBn$) ; 2.94 (m, 2H, $CH_2$- NH- N) ; 3.25 (m, 2H, $CH_2$- N- NH) ; 3.87 (m, 1H, $CH\alpha$) ; 4.99 (s, 2H, $CH_2$- Ph) ; 5.04 (s, 2H, $CH_2$- Ph) ; 5.06 (s, 2H, $CH_2Ph$) ; 7.20- 7.38 (m, 15H, $H_{arom}$) .
HRMS- ESI (m/z) : [M+Na] calc. pour $C_{38}H_{50}N_4O_9$, 713.3526 ; exp., 713.3520.

**EXEMPLE 20**

**Ester benzylique de l'acide (3S)-3-benzyloxycarbonylamino-4-[2-benzyloxycarbonyl-2-(6-benzyloxycarbonylamino-hexyl)-hydrazino]-butyrique (IB.12)**

**a) Ester benzylique de l'acide 3-benzyloxycarbonylamino-4-oxo-butanoïque (V.2)**

Le composé (**V. 2**) est obtenu selon un mode opératoire identique à celui suivi pour la synthèse de (**V. 1**) en utilisant du Z- Asp (OBn)- OH comme produit de départ.
**IR** (NaCl) : $\nu_{NH}$ = 3354 $cm^{-1}$ ; $\nu_{Csp2\ Harom}$ = 3065 $cm^{-1}$ et 3034 $cm^{-1}$ ; $\nu_{Csp3}$ = 2931 $cm^{-1}$; $\nu_{C=O}$ = 1731 $cm^{-1}$, 1715 $cm^{-1}$ et 1693 $cm^{-1}$; $\nu_{C=C}$ = 1528 $cm^{-1}$ ; $\nu_{CO}$ ester = 12 59 $cm^{-1}$.
**b)** Selon un mode opératoire identique à celui décrit pour le composé (**IB. 1**), 28 mg d'aza- amino acide (**III. 3**) (0.07 mmole, 1 éq) et 45 mg de composé (**V. 2**) (0.13 mmole, 1.67 éq) conduisent, après purification par chromatographie flash sur gel de silice avec comme éluant un mélange AcOEt/ Cyclohexane (3: 7 en volume), à 20 mg d'une huile jaune.
**RMN $^1$H** 300MHz ($CDCl_3$) : $\delta$ (ppm) = 1.10- 1.46 (m, 8H, $CH_2$- $(\underline{CH_2})_4$- $CH_2$) ; 2.60- 2.70 (m, 4H, $\underline{CH_2}$- NHZ et $CH_2COOBn$) ; 2.94 (m, 2H, $CH_2$- NH- N) ; 3.25 (m, 2H, $CH_2$- N- NH) ; 3.95 (m, 1H, $CH\alpha$) ; 4.96- 5.12 (s, 8H, 4 x $CH_2$- Ph) ; 7.20- 7.40 (m, 20H, $H_{arom}$) .
**IR** (NaCl) : $\nu_{NH}$ = 3333 $cm^{-1}$ ; $\nu_{Csp2\ Harom}$ = 3065 $cm^{-1}$ et 3034 $cm^{-1}$ ; $\nu_{Csp3}$ = 2932 $cm^{-1}$ et 2857 $cm^{-1}$ ; $\nu_{C=O}$ = 1695 $cm^{-1}$ ; $\nu_{C=C}$ = 1531 $cm^{-1}$ ; $\nu_{C=O}$ ester = 1254 $cm^{-1}$ et 1174 $cm^{-1}$.
$[\alpha]_D^{25°C}$ =- 0.06° (5 g/L, DCM) .
HRMS- ESI (m/z) : [M+Na] calc. pour $C_{40}H_{48}N_4O_6$, 747.3370 ; exp., 747.3369.

**EXEMPLE 21**

**Ester benzylique de l'acide (3S)-4-[2-benzyloxycarbonyl-2-(6-*tert*-butoxycarbonylamino-hexyl)-hydrazino]-3-*tert*-butoxycarbonylamino-butyrique (IB.13)**

Selon un mode opératoire identique à celui décrit pour le composé (**IB.1**), 153 mg d'aza-amino acide (**III.4**) (0.4 mmole, 1 éq) et 200 mg de composé (**V.1**) (0.7 mmole, 1.67 éq) conduisent, après purification par chromatographie flash sur gel de silice avec comme éluant un mélange AcOEt/Cyclohexane (3:7 en volume), à 130 mg d'une huile jaune.
RMN $^1$**H** 300MHz (DMSO) : $\delta$ (ppm) = 1.35 (s, 18H, 2xCOO $(\underline{CH_3})_3$) ; 1.29- 1.49 (m, 8H, $CH_2$- $(\underline{CH_2})_4$- $CH_2$) ; 2.41- 2.86 (m, 4H, $\underline{CH_2}$- NHBoc et $CH_2COOBn$) ; 2.88 (m, 2H, $CH_2$- NH- N) ; 3.25 (m, 2H, $CH_2$- N- NH) ; 3.86 (m, 1H, $CH\alpha$) ; 5.05 (s, 2H, $CH_2$- Ph) ; 5.07 (s, 2H, $CH_2$- Ph) ; 7.29- 7.36 (m, 10H, $H_{arom}$) . **HRMS- ESI** (m/z) : [M+Na] calc. pour $C_{38}H_{50}N_4O_9$, 679.3683 ; exp., 679.3659.

**EXEMPLE 22**

**Ester *tert*-butylique de l'acide (2S)-6-(benzyloxycarbony)amino)-2-[1-(benzyloxycarbonylméthyl)-2-(*tert*-butoxycarbonyl)-hydrazinocarbonylamino]-hexanoique (IB.14)**

a) **Ester *tert*-butylique de l'acide 2-isopropylidène-hydrazinecarboxylique (2)**

A une solution de Boc-hydrazine (4 g, 30.3 mmoles, 1 éq) dans 55 mL d'acétone, sont ajoutés 1.35 g de $MgSO_4$ anhydre et 4 gouttes d'acide acétique. Le mélange est chauffé au reflux pendant 2 heures. Le sulfate de magnésium

est filtré puis le solvant évaporé pour conduire à 4.96 g d'un solide blanc (**rendement** 95%).

**RMN $^1$H** 300MHz ($CDCl_3$) : δ (ppm) = 1.53 (s, 9H, COOC ($C\underline{H}_3$)$_3$) ; 1.80 (s, 3H, $C\underline{H}_3$) ; 2.05 (s, 3H, $C\underline{H}_3$) ; 7.37 (s, 1H, NH) .

**IR** (KBr) : $\nu_{NH}$ = 3348 $cm^{-1}$ ; $\nu_{Csp3}$ = 2981 $cm^{-1}$ et 2920 $cm^{-1}$ ; $\nu_{C=O}$ = 1725 $cm^{-1}$; $\nu_{C=N}$ = 1648 $cm^{-1}$.

**Point de fusion :** 87°C.

**b)(1-*tert*-butoxycarbonyl-2-isopropylidène-hydrazino)-acétate de benzyle (3)**

4.9 g de composé (**2**) (28.5 mmoles, 1 éq) sont mis en solution dans 60 mL de DMF anhydre, sous $N_2$. 1.4 g de NaH (60% dans de l'huile minérale, 34 mmoles, 1.2 éq) so,t introduits par petites quantités. Un dégagement gazeux et une prise en masse sont observés. 60 mL de DMF anhydre puis 5 mL de bromoacétate de benzyle (31.4 mmoles, 1.1 éq) sont additionnés lentement. La solution devient orangé foncé. Après une heure, le NaH en excès est hydrolysé par ajout lent d'eau. La solution est extraite par deux fois 90 mL d'AcOEt. Les phases organiques sont lavées avec 90 mL d'eau puis 90 mL d'une solution de NaCl saturée, séchées sur $Na_2SO_4$ anhydre puis évaporées. Le résidu est chromatographié sur gel de silice avec comme éluant un mélange AcOEt/E.P. (1:2 en volume) pour conduire à 8.4 g d'une huile jaune (**rendement** 92%).

**RMN $^1$H** 300MHz ($CDCl_3$) : δ (ppm) = 1.45 (s, 9H, COOC ($C\underline{H}_3$)$_3$) ; 1.95 (s, 3H, $C\underline{H}_3$) ; 2.05 (s, 3H, $C\underline{H}_3$) ; 4.35 (s, 2H, N- $CH_2$) ; 5.20 (s, 2H, $C\underline{H}_2$) ; 7.37 (m, 5H, $H_{arom}$) .

**IR** (NaCl) : $\nu_{Csp2\ Harom}$ = 3066 $cm^{-1}$ et 3034 cm-$^1$ ; $\nu_{Csp3}$ = 2977 $cm^{-1}$ et 2932 $cm^{-1}$; $\nu_{C=O}$ = 1754 $cm^{-1}$ et 1710 $cm^{-1}$.

c)**Ditosylate d'hydrazinoacétate de benzyle (VIB.1)**

A une solution de 1g de composé (**3**) (3.13 mmoles, 1 éq) dans 10 mL d'éthanol et 100 μL d'eau sont ajoutés 1.19 g d'acide para-toluènesulfonique (6.25 mmoles, 2 éq). La solution est chauffée au reflux pendant 5h. Le solvant est évaporé pour conduire à 1.64 g d'une gélatine rose qui est utilisée sans aucune autre purification (**rendement** 100%).

**RMN $^1$H** 300MHz ($CDCl_3$) : δ (ppm) = 2.45 (s, 6H, 2 $PhC\underline{H}_3$) $\nu_{C=O}$ = 1724 $cm^1$2.45 (m, 2H, N- $CH_2$) ; 5.10 (s, 2H, $C\underline{H}_2Ph$) ; 7.10 (d, J=8.3Hz, 4H, $H_{arom}$ APTS) ; 7.40 (m, 5H, $H_{arom}$ benzyl) ; 7.70 (d, J=8.3Hz, 4H, $H_{arom}$ APTS) ; 8.60 (bs, 5H, $NH_2^+$et $NH_3^+$) .

**IR** (NaCl) : $\nu_{NH}$ = 3337 $cm^{-1}$ ; $\nu_{Csp2\ Harom}$ = 3066 $cm^{-1}$ et 3034 $cm^{-1}$; $\nu_{Csp3}$ = 2978 $cm^{-1}$ et 2935 $cm^{-1}$ ; $\nu_{C=O}$ = 1724 $cm^{-1}$ ; $\nu_{S=O}$ = 1052 $cm^{-1}$.

d)**(2-tert-butoxycarbonyl-hydrazino)-acétate de benzyle (IVB.1)**

A une solution de 330 mg de ditosylate d'hydrazinoacétate de benzyle (0.63 mmoles, 1 éq) dans 7 mL d'un mélange EtOH/$H_2O$ (1:1 en volume) à 0°C sont ajoutés 139 μL de NMM (1.26 mmoles, 2 éq). 151 mg de $(Boc)_2O$ (0.69 mmoles, 1.1 éq) sont introduits. La solution est agitée à 0°C pendant 15 min puis à température ambiante pendant deux heures. La somlution organique obtenue après ajout d'éther éthylique est lavée avec une solution de $KH_2PO_4$ saturée. La phase aqueuse est réextraite par de l'éther et l'ensemble des phases organiques est séché sur $Na_2SO_4$ anhydre puis évaporé pour conduire à 128 mg d'un liquide jaune (**rendement** 66%).

**RMN $^1$H** 300MHz ($CDCl_3$) : δ (ppm) = 1.35 (s, 9H, COOC $(CH_3)_3$) ; 4.10 (s, 2H, N- $CH_2$) ; 5.10 (s, 2H, $CH_2Ph$) ; 7.20 (m, 5H, $H_{arom}$) .

**IR** (NaCl) : $\nu_{NH}$ = 3349 $cm^{-1}$ $\nu_{Csp2\ Harom}$ = 3070 $cm^{-1}$ et 3035 $cm^{-1}$ ; $\nu_{Csp3}$ = 2980 cm-$^1$ et 2935 $cm^{-1}$; $\nu_{C=O}$ = 1749 $cm^{-1}$ et 1708 $cm^{-1}$.

e) Selon un mode opératoire identique à celui décrit pour le composé (**IA. 4**), 105 mg d'hydrazinoester (**IVB. 1**) (0.37 mmole, 1 éq) et 155 mg de HCl.H- Lys (Z)- OtBu (0.37 mmole, 1 éq) conduisent, après purification par chromatographie flash sur gel de silice avec comme éluant un mélange AcOEt/E.P. (1: 2 en volume), à 70 mg d'une huile jaune (**rendement** 30%) .

**RMN $^1$H** 300MHz ($CDCl_3$) : δ (ppm) = 1.20 (m, 2H, $CH_2$- $C\underline{H}_2CH_2$- $CH_2NHZ$) ; 1.35 (s, 9H, COOC ($C\underline{H}_3$)$_3$) ; 1.40 (s, 9H, COOC ($C\underline{H}_3$)$_3$) ; 1.50- 1.60 (m, 4H, $CH_2$- $C\underline{H}_2$- $C\underline{H}_2$- $CH_2NHZ$) ; 3.10 (q, J=7.5Hz et J=14.3Hz, 2H, $C\underline{H}_2NHZ$) ; 4.10 (s, 2H, N- N- $CH_2$) ; 4.30 (m, 1H, CHα) ; 5.00 (s, 2H, $C\underline{H}_2Ph$) ; 5.15 (s, 2H, $C\underline{H}_2Ph$) ; 6.30 (bs, 1H, NH) ; 6.60 (bs, 1H, NH) ; 7.35 (m, 10H, $H_{arom}$) .

HRMS- ESI (m/z) : [M+Na] calc. pour $C_{33}H_{46}N_4O_9$, 665.3162 ; exp., 665.3168.

**II. Activité biologique**

Il est fait référence dans cette partie relative à l'activité biologique des composés aux Figures annexées.

La **Figure 1** présente un schéma d'efflux de substrat provoqué par un transporteur ABC typique.

La **Figure 2** met en évidence le type d'inhibition du composé (I.A.2).
La **Figure 3** montre la chimiosensibilisation par le composé (IA.2) des cellules HEK-293 exprimant ABCG2.
La **Figure 4** montre l'innocuité du composé (IA.2) sur les cellules HEK-293.
La **Figure 5** montre la chimiosensibilisation par le composé (IA.1) des cellules HEK-293 exprimant ABCG2.
La **Figure 6** montre l'innocuité du composé (IA.1) sur les cellules HEK-293
La **Figure 7** montre la chimiosensibilisation par le composé (IB.1) des cellules HEK-293.
La **Figure 8** montre l'innocuité du composé (IB.1) sur les cellules HEK-293.
La **Figure 9** montre l'innocuité du composé (IB.1) sur les cellules NIH-3T3.
La **Figure 10** montre l'innocuité du composé (IB.11) sur les cellules HEK293.
La **Figure 11** met en évidence le type d'inhibition du composé (I.A.3).
La **Figure 12** montre que le composé (IA.2) réduit le niveau d'expression d'ABCG2.

<u>**Méthodes**</u>

**Cultures cellulaires.** La lignée de fibroblastes humains HEK-293 transfectée avec le vecteur pcDNA3.1 incluant ou pas le gène codant pour ABCG2 (Robey, R. W., Honjo, Y., Morisaki, K., Nadjem, T. A., Runge, S., Risbood, M., Poruchynsky, M. S., Bates, S. E. 2003, Br. J. Cancer 89(10) 1971-1978) a été utilisée pour tester l'efficacité des produits sur ABCG2. La lignée de cellules de rein de hamster nouveau-né BHK21 soit naturelle (contrôle négatif) soit transfectée avec le vecteur pNUT-MRP/His exprimant le gène codant pour ABCC1 (Chang, Xiu-Bao, Hou, Yue-Xian, Riordan, John R.1997, J. Biol. Chem. 272(49) 30962-30968) a été utilisée pour tester l'efficacité des produits sur ABCC1. La lignée cellulaire NIH3T3 naturelle ou transfectée par le vecteur pHaMDR1/A exprimant ABCB1 (Cardarelli, Carol O., Aksentijevich, Ivan, Pastan, Ira, Gottesman, Michael M. 1995 Cancer Res 55 1086-1091) été utilisée pour tester l'efficacité des composés sur ABCB1. Les différents types cellulaires sont cultivés selon les protocoles décrits précédemment (Robey, R. W., Honjo, Y., Morisaki, K., Nadjem, T. A., Runge, S., Risbood, M., Poruchynsky, M. S., Bates, S. E. 2003, Br. J. Cancer 89(10) 1971-1978 pour la lignée HEK293, Chang, Xiu-Bao, Hou, Yue-Xian, Riordan, John R.1997, J. Biol. Chem. 272(49) 30962-30968 pour la lignée BHK21 et Cardarelli, Carol O., Aksentijevich, Ivan, Pastan, Ira, Gottesman, Michael M. 1995 Cancer Res 55 1086-1091 pour la lignée NIH3T3).

**Estimation du niveau d'expression d'ABCG2**. Les cellules HEK293 pcDNA3.1 ou HEK293 pcDNA3. BCRP sont ensemencées dans 16 boites de pétri de 6 $cm^2$ et incubées pendant 24 h à 37 °C sous 5 % de $CO_2$. Après cette incubation, les différents puits sont additionnés de 30 $\mu$L soit de DMSO (contrôles) soit du composé IA. 2 en solution dans du DMSO, correspondant à une concentration finale après dilution de 5 $\mu$M. Après 3h, 24h, 48h ou 72h d'incubation, les cellules sont trypsinées et centrifugées à 200g pendant 5 min. La fraction protéique est extraite par un tampon de lyse hypotonique (10 mM Tris- Cl pH 7, 5, 10 mM NaCl, 1 mM $MgCl_2$, 1 mM DTT, inhibiteurs de protéases) et dosée par l'acide bicinchoninique (Sigma- aldrich) . Dix microgrammes de cette fraction sont déposés sur gel de polyacrylamide de tyep Laemmli (U. K. Laemmli, Nature 1970, 227, 680) pour séparer les protéines, qui sont ensuite transférées sur membrane de polyvinyl fluoride (pvdf) pendant 60 min à 100 mA dans un tampon de transfert 10 mM CAPS pH 11.1, 10% méthanol (Sigma- aldrich) . La membrane de pvdf est ensuite saturée 30 min dans une solution de lait écrémé en poudre à 1 % (w/v) dans du tampon TBST (Tris buffer saline 1X, Tween 20 0, 1 %) . Après un rinçage rapide au TBST, la membrane est incubée 1 h, sous agitation à température ambiante, avec un anticorps monoclonal anti- BCRP BXP-21 (Santa Cruz) ou un anticorps monoclonal anti- tubuline (Santa Cruz) dilué au 1/250$^{ème}$ dans du TBST 1 % lait. Après 3 lavages de 15 min avec du TBST, la membrane est incubée 1 h avec un anticorps secondaire monoclonal anti- souris couplé à la peroxydase (Santa Cruz) dilué au 1/5000$^{ème}$ dans du TBST 1 % lait. Puis la membrane est lavée 3 fois 15 min dans du TBST. La révélation est réalisée par le kit « immobilon western » (Millipore) avec le logiciel QuantityOne (Biorad) .

La **Figure 1** illustre schématiquement un efflux de substrat provoqué par un transporteur ABC typique. Les substrats **S**, tels que la mitoxantrone effluée par ABCG2 et ABCB1 ou la daunorubicine effluée par ABCC1, sont accumulés dans les cellules et sont expulsés par le transporteur **T**, conduisant à une diminution de la fluorescence intracellulaire. Dans le cas de l'accumulation du produit, typiquement par inhibition de la protéine d'efflux, la fluorescence intracellulaire augmente.

**Cytométrie en flux**. Les cellules HEK-293 et BHK-21 transfectées ou pas sont dans un premier temps mises en présence de substrat (5 $\mu$M mitoxantrone ou 1 $\mu$M daunorubicine suivant le transporteur). Le substrat est accumulé pendant 30 minutes à 37 °C en présence ou en absence de concentrations variées des molécules testées, à 0.5 % de DMSO final. Après lavage par du PBS, les cellules sont incubées dans du milieu contenant les mêmes concentrations de molécules testées que précédemment, pendant 1 h à 37 °C. La fluorescence intracellulaire de drogue est mesurée avec le FACscan flow cytometer (Becton Dickinson, Moutain, View, CA). Les substrats fluorescents sont excités à 488 nm ; l'émission de fluorescence de la mitoxantrone (MTX) est mesurée à 650 nm et celle de la daunorubicine est mesurée à 530 nm. L'efficacité des molécules testées est estimée par l'équation 1 :

$$\% \text{ efficacité} = 100 \times (F_A - F_{BG}) / (F_C - F_{BG}),$$

Où :

■ $F_A$ correspond au niveau intracellulaire de substrat mesuré dans les cellules surexprimant le transporteur T (correspondant au plus faible niveau de fluorescence en absence d'inhibiteur),

■ $F_C$ correspond à la fluorescence dans les cellules contrôles n'exprimant pas le transporteur testé. C'est donc l'accumulation maximale de substrat dans les cellules),

■ $F_{BG}$ correspond à la fluorescence mesurée en absence de substrat et en présence d'inhibiteur.

**Test de croissance cellulaire.**

Les cellules sont ensemencées (10 000 cellules/ puits) dans des plaques 96 puits et incubées pendant 24 heures à 37 °C sous 5 % de $CO_2$. Des concentrations variées de mitoxantrone (de 0 à 20 μM dans un volume final constant de 100 μL) sont ensuite additionnées avec un volume égal de chacun des composés testés. Les cellules sont ensuite laissées à diviser pendant 72 h. La cytotoxicité est évaluée colorimétriquement avec le 3- (4, 5- diméthylthiazol-2- yl)- 2, 5- diphényltetrazolium bromide (MTT) .

**A- EFFICACE D'INHIBITION DES PROTEINES D'EFFLUX HUMAINES ABC PAR LES AZADIPEPTIDES**

Les composés présentés dans les **TABLEAUX 2** à **5** ont été testés par cytométrie en flux sur des lignées cellulaires de mammifères exprimant sélectivement les protéines d'efflux ABCG2, ABCB1 ou ABCC1, telles que décrites précédemment.

Les **TABLEAU 6** et 6bis montrent l'efficacité d'inhibition des azadipeptides (IA.1), (IA.2), (IA.3), (IB.1) et (IB. 11) sur l'efflux des drogues anticancéreuses par les pompes ABC humaines.

**TABLEAU 6**

| Composé | BCRP / ABCG2 | | | P-gp / ABCB1 | | |
|---|---|---|---|---|---|---|
| | % efficacité | | $IC_{50}$, μM | % efficacité | | $IC_{50}$, μM |
| | 10 μM | 2 μM | | 10 μM | 2 μM | |
| (IA.3) | 38±14 | - | - | 98±14 | 90±7 | 0.33±0.09 |
| (IA.1) | 112±6 | 53±10 | 2.0±0.2 | 74±1 | 70±10 | ≤0.5 |
| (IA.2) | 112±26 | 86±14 | 1.06±0.05 | 43±8 | - | - |
| (IB.1) | 87±27 | 72±1 | 0.90±0.05 | 63±5 | 70±20 | 1.0±0.1 |
| (IB.11) | 104±10 | 92±2 | 0.5±0.05 | - | - | 0.7±0.1 |

**TABLEAU 6bis**

| Composé | MRP1 / ABCC1 % efficacité 10 µM |
|---|---|
| (IA.3) | - |
| (IA.1) | 14±3 |
| (IA.2) | 5±1 |
| (IB.1) | 5±1 |
| (IB.11) | - |

L'efflux de mitoxantrone induit par ABCG2 ou ABCB1 et l'efflux de daunorubicine induit par ABCC1 sont estimés par cytométrie en flux, comme décrit précédemment, à 10 µM de chaque composé testé, et ensuite à 2 µM pour les composés présentant au moins 80 % d'efficacité à 10 µM. La concentration induisant 50 % d'inhibition ($IC_{50}$) a été estimée pour les dérivés aza les plus efficaces. Les valeurs sont obtenues par 3 expérimentations indépendantes et ont été ajustées par le logiciel Sigmaplot.

Ce test met en évidence la capacité des produits à bloquer l'efflux de mitoxantrone (par ABCG2 ou ABCB1) ou de daunorubicine (par ABCC1).

Comme il est montré dans le **TABLEAU 6**, le composé (IA.3) est le plus efficace pour bloquer ABCB1 ($IC_{50}$ de 0.33 µM) tandis que son efficacité d'inhibition d'ABCG2 est faible (38 % à 10 µM). L'inverse est observé pour le composé (IA.2) qui a une $IC_{50}$ pour ABCG2 de 1 µM et une efficacité de blocage d'ABCB1 inférieure à 50 % à 10 µM. Le composé (IA.1) présente une meilleure affinité pour ABCB1, avec une IC50 de l'ordre de 0,5 µM tandis qu'elle est de 2 µM pour ABCG2. Les composés (IB.1 et IB.11) ont la même efficacité sur les 2 transporteurs avec une $IC_{50}$ micromolaire ou submicromolaire dans les 2 cas. Tous ces composés ne semblent pas avoir d'effet sur la protéine d'efflux ABCC1.

**B- LE COMPOSE (IA.2) EST UN PUISSANT INHIBITEUR NON COMPETITIF D'ABCG2**

La **Figure 2** met en évidence que le composé (IA.2) est un inhibiteur non compétitif d'ABCG2. L'efflux de mitoxantrone par ABCG2 a été mesuré à des concentrations de substrat variant de 0 à 20 µM (panneaux A-C) en présence ou en absence de concentrations fixes de composé (IA.2) dans une gamme de 0 à 20 µM (panneaux D-E). Dans la **Figure 2,** les résultats obtenus en faisant varier la concentration d'inhibiteur sont présentés en représentation, soit directe dans le panneau A, soit de Lineweaver & Burke dans le panneau B ou de Eadie-Hofstee dans le panneau C. Les résultats obtenus en faisant varier la concentration de substrat sont présentés en échelle des abscisses linéaire (panneau D) ou logarithmique (panneau E). Les courbes ont été ajustées par le logiciel Sigmaplot.

Les résultats présentés sur la **Figure 2A** par la représentation directe de l'efflux de mitoxantrone en fonction de sa concentration, montrent que le plateau atteint par chaque courbe obtenue est différent pour une concentration donnée de (IA.2) tandis que la concentration de demi-accumulation maximale de mitoxantrone reste la même quelle que soit la concentration de l'inhibiteur. Ces données sont caractéristiques d'une inhibition de type non compétitif. Ceci est confirmé par la représentation de Lineweaver & Burke et Eadie-Hofstee présentées respectivement dans la **Figure 2B** et la **Figure 2C**. Les représentations de ces 2 graphiques sont caractéristiques de ce type d'inhibition. La constante d'inhibition $K_i$ du composé (IA.2) est estimé autour de 1 µM à partir de l'ajustement des points de la **Figure 2D** ou de la **Figure 2E**.

**C- CHIMIOSENSIBILISATION DES CELLULES HEK-293 EXPRIMANT ABCG2 PAR LE COMPOSE (IA.2).**

Des concentrations croissantes de mitoxantrone (MTX) sont appliquées à des cellules HEK-293 exprimant ou n'exprimant pas ABCG2 et soumises ou non à un traitement avec 5 µM de composé (IA.2), et dont la vitalité résiduelle est évaluée après 72 h de traitement par un test MTT. Les résultats sont illustrés à la **Figure 3**, dans laquelle les cellules exprimant ABCG2 sont représentées par des cercles, celles ne l'exprimant pas par des carrés ; celles qui sont traitées avec 5 µM de (IA.2), soit 5 fois son $IC_{50}$, apparaissent en symboles vides, tandis que celles qui ne sont pas traitées avec (IA.2) apparaissent en symboles pleins. La **Figure 3** montre que l'incubation des cellules HEK-293 surexprimant ABCG2 avec le composé (IA.2) restaure une sensibilité à la mitoxantrone égale à celle observée dans les cellules

contrôle dans lesquelles ABCG2 n'est pas exprimé. Le composé (IA.2) apparait donc être un agent de chimiosensibilisation très efficace.

**D- ABSENCE DE CYTOTOXICITE DU COMPOSE (IA.2) SUR LA CROISSANCE CELLULAIRE DE CELLULES HUMAINES HEK-293**

Des concentrations croissantes de composé (IA.2) sont appliquées à des cellules HEK-293 exprimant ou n'exprimant pas ABCG2, et dont la vitalité résiduelle est évaluée après 72 h de traitement par un test MTT. Les résultats sont illustrés à la **Figure 4**, dans laquelle les cellules exprimant ABCG2 sont représentées par des cercles vides, tandis que celles ne l'exprimant pas par des cercles pleins. Les résultats montrent que le composé (IA.2) n'est absolument pas cytotoxique pour les cellules HEK-293, qu'elles expriment ou pas ABCG2. Cette absence de toxicité est observée même à une très forte concentration telle que 40 $\mu$M, qui correspond à 40 fois la valeur d'$IC_{50}$.

**E- CHIMIOSENSIBILISATION DES CELLULES HEK-293 EXPRIMANT ABCG2 PAR LE COMPOSE (IA.1).**

Des concentrations croissantes de mitoxantrone (MTX) sont appliquées à des cellules HEK-293 exprimant ou n'exprimant pas ABCG2 et soumises ou non à un traitement avec 5 $\mu$M de composé (IA.1), et dont la vitalité résiduelle est évaluée après 72 h de traitement par un test MTT. Les résultats sont illustrés à la **Figure 5**, dans laquelle les cellules exprimant ABCG2 sont représentées par des cercles, celles ne l'exprimant pas par des carrés ; celles qui sont traitées avec 5 $\mu$M de (IA.1), soit 2.5 fois son $IC_{50}$, apparaissent en symboles vides, tandis que celles qui ne sont pas traitées avec (IA.1) apparaissent en symboles pleins. Les résultats montrent que le composé (IA.1) restaure une sensibilité quasi complète à la mitoxantrone des cellules qui surexpriment ABCG2, du même niveau que celle observée dans les cellules n'exprimant pas le transporteur. La résistance résiduelle est attribuée au fait que la concentration de produit, 5 $\mu$M, qui ne correspond qu'à 2.5 fois l'$IC_{50}$, est donc probablement insuffisante pour conférer une sensibilisation complète. Le composé (IA.1) est donc, lui aussi, un agent chimiosensibilisant efficace.

**F- Absence de cytotoxicité du composé (IA.1) sur la croissance cellulaire**

Des concentrations croissantes de composé (IA.1) sont appliquées à des cellules HEK-293 exprimant ou n'exprimant pas ABCG2, et dont la vitalité résiduelle est évaluée après 72 h de traitement par un test MTT. Les résultats sont illustrés à la **Figure 6**, dans laquelle les cellules exprimant ABCG2 sont représentées par des cercles vides, tandis que celles ne l'exprimant pas par des cercles pleins.

Les résultats montrent que le composé (IA.1) n'a pas d'effet cytotoxique dans la gamme de concentrations testée, et cela même à plus de 10 fois son $IC_{50}$, qu'ABCG2 soit exprimé ou non.

**G- Chimiosensibilisation des cellules HEK-293 exprimant ABCG2 par le composé (IB.1)**

Des concentrations croissantes de mitoxantrone (MTX) sont appliquées à des cellules HEK-293 exprimant ou n'exprimant pas ABCG2 et soumises ou non à un traitement avec 5 $\mu$M de composé (IB.1), et dont la vitalité résiduelle est évaluée après 72 h de traitement par un test MTT. Les résultats sont illustrés à la **Figure 7**, dans laquelle les cellules exprimant ABCG2 sont représentées par des cercles, celles ne l'exprimant pas par des carrés ; celles qui sont traitées avec 5 $\mu$M de (IB.1), soit 5 fois son $IC_{50}$, apparaissent en symboles vides, tandis que celles qui ne sont pas traitées avec (IB.1) apparaissent en symboles pleins. Les résultats montrent que l'incubation des cellules HEK-293 surexprimant ABCG2 avec le composé (IB.1) conduit à une restauration quasi complète de la sensibilité à la mitoxantrone, d'un niveau proche de celui observé dans les cellules n'exprimant pas le transporteur. Le composé (IB.1) est donc, lui aussi, un agent chimiosensibilisant efficace.

**H- ABSENCE DE CYTOTOXICITE DU COMPOSE (IB.1) SUR LA CROISSANCE CELLULAIRE.**

Des concentrations croissantes de composé (IB.1) sont appliquées à des cellules HEK-293 exprimant ou n'exprimant pas ABCG2, et dont la vitalité résiduelle est évaluée après 72 h de traitement par un test MTT. Les résultats sont illustrés à la **Figure 8**, dans laquelle les cellules exprimant ABCG2 sont représentées par des cercles vides tandis que celles ne l'exprimant pas par des cercles pleins. Les résultats montrent que le composé (IB.1) n'a pas d'effet cytotoxique jusqu'à une concentration équivalente à 20 fois son $IC_{50}$, qu'ABCG2 soit exprimé ou non. A 40 fois l'$IC_{50}$, une cytotoxicité relative apparaît.

**I- CYTOTOXICITE LIMITEE DU COMPOSE (IB.1) SUR LA CROISSANCE DES CELLULES NIH-3T3**

Des concentrations croissantes de composé (IB.1) sont appliquées à des cellules NIH-3T3 exprimant ou n'exprimant pas ABCB1, et dont la vitalité résiduelle est évaluée après 72 h de traitement par un test MTT. Les résultats sont illustrés à la **Figure 9**, dans laquelle les cellules exprimant ABCB1 sont représentées par des cercles vides, tandis que celles ne l'exprimant pas par des cercles pleins. Les résultats montrent que jusqu'à 5 fois son $IC_{50}$ (1 $\mu$M), la toxicité du composé (IB.1) est négligeable. Elle augmente de 30-40 % au delà, mais reste toutefois limitée à cette valeur, jusqu'à une concentration équivalente à 40 fois son $IC_{50}$, qu'ABCB1 soit exprimé ou non.

**3- CYTOTOXICITE LIMITEE DU COMPOSE (IB.11) SUR LA CROISSANCE CELLULAIRE**

Des concentrations croissantes de composé (IB.11) sont appliquées à des cellules HEK-293 exprimant ou n'exprimant pas ABCG2, et dont la vitalité résiduelle est évaluée après 72 h de traitement par un test MTT. Les résultats sont illustrés à la **Figure 10**, dans laquelle les cellules exprimant ABCG2 sont représentées par des cercles vides, tandis que celles ne l'exprimant pas par des cercles pleins. Les résultats montrent que le composé (IB.11) n'a pas d'effet cytotoxique dans la gamme de concentrations testée, et cela même à plus de 80 fois son $IC_{50}$, qu'ABCG2 soit exprimé ou non.

**K- LE COMPOSE (IA.3) EST UN PUISSANT INHIBITEUR NON COMPETITIF D'ABCB1**

Les résultats sont présentés sur la **Figure 11** par la représentation directe de l'efflux de daunorubicine en fonction de sa concentration, montrent que le plateau atteint par chaque courbe obtenue est différent pour une concentration donnée de (IA.3) tandis que la concentration de demi-accumulation maximale de daunorubicine reste la même quelle que soit la concentration de l'inhibiteur. Ces données sont caractéristiques d'une inhibition de type non compétitif. Cela indique aussi que cette classe d'inhibiteurs est de façon générale non compétitive sur ABCB1 et ABCG2 (cf. Figure 2 et paragraphe B précédent).

**L- LE COMPOSE (IA.2) REDUIT LE NIVEAU D'EXPRESSION D'ABCG2, LA PROTEINE QU'IL INHIBE**

La **Figure 12** met en évidence la modulation du niveau d'expression d'ABCG2 par l'inhibiteur I.A2. Les cellules HEK293 exprimant ou pas BCRP, respectivement « HEK293 BCRP » et « HEK293 pcDNA3 », sont cultivées pendant le temps indiqué en présence du composé IA.2 ou de DMSO (contrôle négatif). Elles sont ensuite récoltées et fractionnées pour analyser leur contenu en ABCG2 et tubuline, cette dernière étant utilisée comme témoin, comme détaillé dans la partie Méthodes.

Les résultats présentés sur la **Figure 12** montrent qu'en plus de son action inhibitrice d'ABCG2, le composé IA.2 entraine aussi une diminution du niveau d'expression de la protéine, sans toucher à celle d'une protéine traditionnellement utilisée comme témoin, la tubuline. Le composé IA.2 permet donc non seulement de moduler l'activité d'ABCG2, mais aussi son niveau d'expression, ce qui potentialise d'autant son action.

## Revendications

**1.** Composés, de type azapeptide ou azapeptidomimétique, de formule (I) :

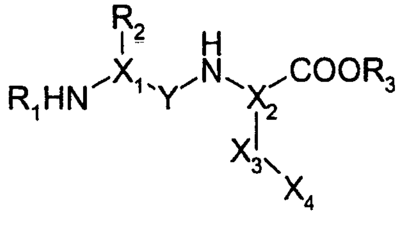

(I)

dans laquelle :

- $R_1$ représente un groupe- C (O) OR'$_1$ avec R'$_1$ qui représente un groupe alkyle de 1 à 12 atomes de carbone ou un groupe- $(CH_2)_{m1}R''_1$ avec $R''_1$ qui représente un groupe aryle, cycloalkyle ou fluorényle et m1 qui est égal

à 0, 1, 2 ou 3,
- $X_1$ et $X_2$ sont identiques ou différents et représente -N- ou -CH-, étant entendu que l'un au moins représente -N-,
- $R_2$ représente une chaine latérale éventuellement sous forme protégée d'un acide aminé ou d'un analogue d'acide aminé, ou un groupe aryle éventuellement substitué ou un groupe hétéroaryle éventuellement substitué,
- Y représente $-CH_2-$ ou -C(O)-,
-- $X_3$- $X_4$ représente soit un groupe- $(CH_2)_n$- $NHR_4$ avec n égal à 3, 4, 5 ou 6, soit un groupe choisi parmi :

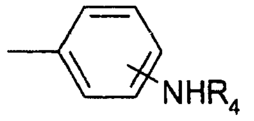

et

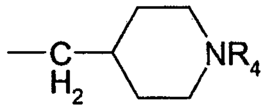

avec $R_4$ qui représente un groupe protecteur d'un groupement amino, de préférence un groupe- COOR'$_4$ avec R'$_4$ qui représente un groupe alkyle de 1 à 12 atomes de carbone ou un groupe- $(CH_2)_{m4}R''_4$ avec $R''_4$ qui représente un groupe aryle ou cycloalkyle et m4 qui est égal à 0, 1, 2 ou 3,
- $R_3$ représente un groupe alkyle de 1 à 12 atomes de carbone, ou un groupe- $(CH_2)_{m3}R''_3$ avec $R''_3$ qui représente un groupe cycloalkyle ou aryle, les dits groupes cycloalkyle et aryle pouvant être non- substitués ou substitués par un ou plusieurs groupes choisis parmi :- $CH_3$, $-CF_3$, -COOH, $-NO_2$, -Cl et $NH_2$ et m3 qui est égal à 0, 1, 2 ou 3,

sous la forme d'isomère optique pur ou de mélange d'isomères optiques, ainsi que leurs sels, solvats ou hydrates.

2. Composés de type azapeptide ou azapeptidomimétique selon la revendication 1 **caractérisés en ce que** $R_2$ représente un groupe- L- COOR'$_2$ avec L qui représente un groupe aryle, de préférence phényle, ou une chaine- $(CH_2)_{m_2}$- avec $m_2$ qui est égal à 1, 2 ou 3 et R'$_2$ qui représente un groupe alkyle de 1 à 12 atomes de carbone ou un groupe- $(CH_2)_{m'2}R''_2$ avec $R''_2$ qui représente un groupe aryle ou cycloalkyle, éventuellement substitué et m'2 qui est égal à 0, 1, 2 ou 3.

3. Composés de type azapeptide ou azapeptidomimétique selon la revendication 1 de formule (IA), sous la forme d'isomère pur ou de mélange d'isomères, ainsi que leurs sels solvats ou hydrates :

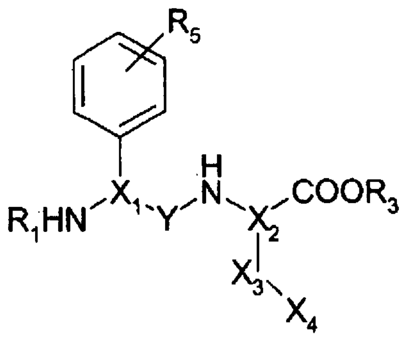

(IA)

dans laquelle $R_5$ représente un groupe- $NH_2$, $-NO_2$, -OH, -O- alkyle de 1 à 8 atomes de carbone, O- $(CH_2)_{m5}R'_5$ avec R'$_5$ qui représente un groupe aryle et m5 qui est égal à 0 ou 1 ou bien $R_5$ représente un groupe- COOR"$_5$ avec R"$_5$ qui représente un groupe alkyle de 1 à 12 atomes de carbone ou un groupe- $(CH_2)_{m'5}R'''_5$ avec $R'''_5$ qui représente un groupe aryle ou cycloalkyle et m'5 qui est égal à 0, 1, 2 ou 3, $R_1$, $X_1$, Y, $X_2$, $X_3$, $X_4$ et $R_3$ étant tels que définis pour (I) à la revendication 1.

4. Composés de type azapeptide ou azapeptidomimétique de formule (IA) selon la revendication 3, **caractérisés en ce que** $R_5$ représente un groupe- COOR"$_5$ avec R"$_5$ qui représente un groupe éthyle, benzyle ou- CH $[CH(CH_3)_2]_2$.

5. Composés de type azapeptide ou azapeptidomimétique de formule (IA) selon la revendication 3 ou 4, **caractérisés en ce que** le groupe $R_5$ est positionné en méta.

6. Composés de type azapeptide ou azapeptidomimétique de formule (IA) selon la revendication 3 ou 4, **caractérisés en ce que** le groupe $R_5$ est positionné en para.

**7.** Composés de type azapeptide ou azapeptidomimétique de formule (IA) selon l'une des revendications 3 à 6, **caractérisés en ce que** $X_1$ représente -N- et $X_2$ représente -CH-.

**8.** Composés de type azapeptide ou azapeptidomimétique de formule (IA) selon l'une des revendications 3 à 6, **caractérisés en ce que** $X_1$ et $X_2$ représentent N.

**9.** Composés de type azapeptide ou azapeptidomimétique de formule (IA) selon l'une des revendications 3 à 8, **caractérisés en ce que** Y représente -C(O)-.

**10.** Composés de type azapeptide ou azapeptidomimétique de formule (IA) selon l'une des revendications 3 à 9, **caractérisés en ce que**- $X_3$- $X_4$ représente un groupe- $(CH_2)_n$- $NHR_4$ avec n égal à 4 ou 6 et $R_4$ tel que défini pour (I) à la revendication 1.

**11.** Composés de type azapeptide ou azapeptidomimétique selon la revendication 1 de formule (IB), sous la forme d'isomère pur ou de mélange d'isomères, ainsi que leurs sels solvats ou hydrates :

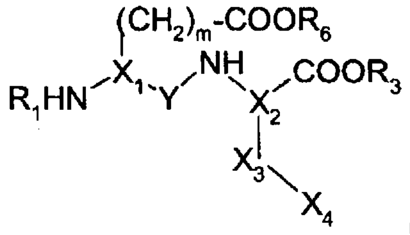

(IB)

dans laquelle m est égal à 1, 2 ou 3, $R_6$ représente un groupe alkyle de 1 à 12 atomes de carbone, ou un groupe- $(CH_2)_{m6}R'_6$ avec $R'_6$ qui représente un groupe cycloalkyle ou aryle, les dits groupes cycloalkyle et aryle pouvant être non- substitués ou substitués par un ou plusieurs groupes choisis parmi :- $CH_3$, -$CF_3$, -COOH, -$NO_2$, -Cl et $NH_2$ et m6 qui est égal à 0, 1, 2 ou 3, $R_1$, $X_1$, Y, $X_2$, $X_3$, $X_4$ et $R_3$ étant tels que définis pour (I) à la revendication 1.

**12.** Composés de type azapeptide ou azapeptidomimétique de formule (IB) selon la revendication 11, **caractérisés en ce que** m est égal à 1 ou 2.

**13.** Composés de type azapeptide ou azapeptidomimétique de formule (IB) selon la revendication 11 ou 12, **caractérisés en ce que** $R_6$ représente un groupe éthyle, benzyle, ou- CH [CH $(CH_3)_2]_2$.

**14.** Composés de type azapeptide ou azapeptidomimétique de formule (IB) selon l'une des revendications 11 à 13, **caractérisés en ce que** $X_1$ représente -CH-.

**15.** Composés de type azapeptide ou azapeptidomimétique de formule (IB) selon l'une des revendications 11 à 14, **caractérisés en ce que** $X_2$ représente -N-.

**16.** Composés de type azapeptide ou azapeptidomimétique de formule (IB) selon l'une des revendications 11 à 15, **caractérisés en ce que**- $X_3$- $X_4$ représente un groupe- $(CH_2)_n$- $NHR_4$ avec n égal à 6 et $R_4$ tel que défini pour (I) à la revendication 1.

**17.** Composés de type azapeptide ou azapeptidomimétique de formule (I), (IA) ou (IB) selon l'une des revendications 1 à 16, **caractérisés en ce que** $R_1$ représente un groupe -COOR'$_1$ et R'$_1$ représente un groupe tert-butyle ou benzyle.

**18.** Composés de type azapeptide ou azapeptidomimétique de formule (I), (IA) ou (IB) selon l'une des revendications 1 à 17, **caractérisés en ce que** $R_3$ représente un groupe tert-butyle ou benzyle.

**19.** Composés de type azapeptide ou azapeptidomimétique de formule (I), (IA) ou (IB) selon l'une des revendications 1 à 18, **caractérisés en ce que** $R_4$ représente -COOR'$_4$ avec R'$_4$ qui représente un groupe benzyle ou *tert*-butyle.

**20.** Composés de type azapeptide ou azapeptidomimétique selon la revendication 1 choisis parmi :

- Ester *tert*- butylique de l'acide (2S)- 6- (benzyloxycarbonylamino)- 2- [1- (2'- (benzyloxycarbonyl) phényl)- 2- (*tert*- butoxycarbonyl)- hydrazinocarbonylamino]- hexanoïque (IA. 1)
- Ester tert- butylique de l'acide (2S)- 6- (benzyloxycarbonylamino)- 2- [1- (3'- (benzyloxycarbonyl) phényl)- 2- *(tert*- butoxycarbonyl)- hydrazinocarbonylamino]- hexanoïque (IA. 2)
- Ester *tert*- butylique de l'acide (2S)- 6- (benzyloxycarbonylamino)- 2- [1- (4'- (benzyloxycarbonyl) phényl)- 2- (*tert*- butoxycarbonyl)- hydrazinocarbonylamino]- hexanoïque (IA. 3)
- Ester benzylique de l'acide (2R)- 6- (benzyloxycarbonylamino)- 2- [1- (3'- (benzyloxycarbonyl) phényl)- 2- (*tert*-butoxycarbonyl)- hydrazinocarbonylamino]- hexanoïque (IA. 4)
- Ester benzylique de l'acide (2S)- 6- (benzyloxycarbonylamino)- 2- [1- (3'- (benzyloxycarbonyl) phényl)- 2- (*tert*-butoxycarbonyl)- hydrazinocarbonylamino]- hexanoïque (IA. 5)
- Ester *tert*- butylique de l'acide (2S)- 6- (benzyloxycarbonylamino)- 2- [1- (3'- (phényloxycarbonyl) phényl)- 2- (*tert*- butoxycarbonyl)- hydrazinocarbonylamino]- hexanoïque (IA. 6)
- Ester benzylique de l'acide 2- [1- (3'- (benzyloxycarbonyl) phényl)- 2- (*tert*- butoxycarbonyl)- hydrazinocarbonyl]- 1- (6- benzyloxycarbonylamino- hexyl)- hydrazinocarboxylique (IA. 7)
- Ester benzylique de l'acide 2- [1- (3'- (benzyloxycarbonyl) phényl)- 2- (*tert*- butoxycarbonyl)- hydrazinocarbonyl]- 1- (6- *tert*- butyloxycarbonylamino- hexyl)- hydrazinocarboxylique (IA. 8)
- Ester benzylique de l'acide (3S)- 4- [2- (6- benzyloxycarbonylamino- hexyl)- 2- *tert*- butoxycarbonyl- hydrazino]- 3- *tert*- butoxycarbonylamino- butyrique (IB. 1)
- Ester benzylique de l'acide (3S)- 4- [2- (3- benzyloxycarbonylamino- propyl)- 2- *tert*- butoxycarbonyl- hydrazino]- 3- *tert*- butoxycarbonylamino- 4- oxo- butyrique (IB. 2)
- Ester benzylique de l'acide (3S)- 4- [2- (4- benzyloxycarbonylamino- phényl)- 2- tert- butoxycarbonyl- hydrazino]- 3- *tert*- butoxycarbonylamino- 4- oxo- butyrique (IB. 3)
- Ester benzylique de l'acide (4S)- 5- [2- (4- benzyloxycarbonylamino- phényl)- 2- *tert*- butoxycarbonyl- hydrazino]- 4- *tert*- butoxycarbonylamino- 5- oxo- pentanoïque (IB. 4)
- Ester benzylique de l'acide (3S)- 4- [2- (5- benzyloxycarbonylamino- pentyl)- 2- *tert*- butoxycarbonyl- hydrazino]- 3- *tert*- butoxycarbonylamino- 4- oxo- butyrique (IB. 5)
- Ester benzylique de l'acide 4- [2- ((2S)- 3- benzyloxycarbonyl- 2- *tert*- butoxycarbonylamino- propyl)- 1- *tert*-butoxycarbonyl- hydrazinométhyl]- pipéridine- 1- carboxylique (IB. 6)
- Ester benzylique de l'acide 4- [2- ((2S)- 3- benzyloxycarbonyl- 2- *tert*- butoxycarbonylamino- propionyl)- 1- *tert*- butoxycarbonyl- hydrazinométhyl]- pipéridine- 1- carboxylique (IB. 7)
- Ester benzylique de l'acide (3S)- 4- [2- (5- benzyloxycarbonylamino- pentyl)- 2- *tert*- butoxycarbonyl- hydrazino]- 3- *tert*- butoxycarbonylamino- butyrique (IB. 8)
- Ester benzylique de l'acide (3S)- 4- [2- (6- benzyloxycarbonylamino- hexyl)- 2- *tert*- butoxycarbonyl- hydrazino]- 3- *tert*- butoxycarbonylamino- 4- oxo- butyrique (IB. 9)
- Ester benzylique de l'acide (3S)- 3- *tert*- butoxycarbonylamino- 4- [2- *tert*- butoxycarbonyl- 2- (6- *tert*- butoxycarbonylamino- hexyl)- hydrazino]- butyrique (IB. 10)
- Ester benzylique de l'acide (3S)- 4- [2- benzyloxycarbonyl- 2- (6- benzyloxycarbonylamino- hexyl)- hydrazino]- 3- *tert*- butoxycarbonylamino- butyrique (IB. 11)
- Ester benzylique de l'acide (3S)- 3- benzyloxycarbonylamino- 4- [2- benzyloxycarbonyl- 2- (6- benzyloxycarbonylamino- hexyl)- hydrazino]- butyrique (IB. 12)
- Ester benzylique de l'acide (3S)- 4- [2- benzyloxycarbonyl- 2- (6- *tert* butoxycarbonylamino- hexyl)- hydrazino]- 3- *tert*- butoxycarbonylamino- butyrique (IB. 13)
- Ester *tert*- butylique de l'acide (2S)- 6- (benzyloxycarbonylamino)- 2- [1- (benzyloxycarbonylméthyl)- 2- (*tert*-butoxycarbonyl)- hydrazinocarbonylamino]- hexanoïque (IB. 14),

ainsi que leurs sels, solvats et/ou hydrates, et notamment ceux pharmaceutiquement acceptables.

**21.** Composé de type azapeptide ou azapeptidomimétique selon l'une des revendications précédentes en tant qu'adjuvant d'un traitement thérapeutique administré à un patient, pour lequel l'organisme du patient traité développe une résistance, destiné à restaurer l'activité du traitement thérapeutique.

**22.** Composé de type azapeptide ou azapeptidomimétique selon l'une des revendications précédentes en tant qu'adjuvant d'un médicament anticancéreux ou anti-infectieux.

**23.** Composé de type azapeptide ou azapeptidomimétique selon l'une des revendications précédentes en tant qu'adjuvant d'un médicament choisi parmi les anthracyclines, les inhibiteurs de topoisomérase, les antimétaboliques (antifolates), les inhibiteurs de tyrosine kinase, les antiviraux (inhibiteurs de reverse transcriptase), antiparasitaires.

**24.** Composé de type azapeptide ou azapeptidomimétique selon l'une des revendications précédentes en tant qu'adjuvant d'un médicament choisi parmi la daunorubicine, la doxorubicine, la mitoxantrone, la camptotécine et ses dérivés, l'irinotécan, le topotécan, les indolocarbazoles, le méthotrexate, l'imatinib, le gefitinib, la zidovudine, la lamivudine, l'abacavir, l'ivermectine, l'albendazole, l'oxfendazole, le kétoconazole.

## Patentansprüche

**1.** Verbindungen, vom Typ Azapeptid oder azapeptidomimetischen Typ, der Formel (I) :

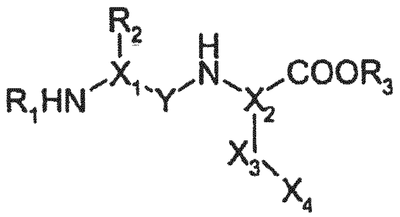

(I)

worin:

- $R_1$ eine- C (O) $OR'_1$- Gruppe darstellt, wobei $R'_1$ eine Alkyl- Gruppe mit 1 bis 12 Kohlenstoffatomen darstellt, oder eine- $(CH_2)_{m1}R''_1$- Gruppe darstellt, wobei $R''_1$ eine Aryl-, Cycloalkyl- oder Fluorenyl- Gruppe darstellt und m1 gleich 0, 1, 2 oder 3 ist,
- $X_1$ und $X_2$ gleich oder verschieden sind und -N- oder -CH- darstellt, wobei wenigstens eines -N- darstellt,
- $R_2$ eine Seitenkette, eventuell in geschützter Form, einer Aminosäure oder eines Aminosäure-Analogs, oder eine Aryl-Gruppe, eventuell substituiert, oder eine Heteroaryl-Gruppe, eventuell substituiert, darstellt,
- Y $-CH_2$- oder -C(O)- darstellt,
-- $X_3$- $X_4$ entweder eine- $(CH_2)_n$- $NHR_4$- Gruppe, mit n gleich 3, 4, 5 oder 6, oder eine Gruppe darstellt, die ausgewählt ist aus:

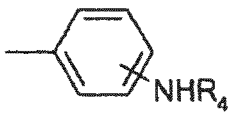

und

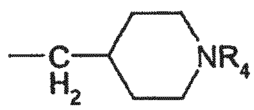

wobei $R_4$ eine Schutzgruppe einer Aminogruppe darstellt, vorzugsweise eine- $COOR'_4$- Gruppe, wobei $R'_4$ eine Alkyl- Gruppe mit 1 bis 12 Kohlenstoffatomen darstellt, oder eine- $(CH_2)_{m4}R''_4$- Gruppe, wobei $R''_4$ eine Aryl- oder Cycloalkyl- Gruppe darstellt und m4 gleich 0, 1, 2 oder 3 ist,
- $R_3$ eine Alkyl- Gruppe mit 1 bis 12 Kohlenstoffatomen oder eine- $(CH_2)_{m3}R''_3$- Gruppe darstellt, wobei $R''_3$ eine Cycloalkyl- oder Aryl- Gruppe darstellt, wobei die Cycloalkyl- und Aryl- Gruppen unsubstituiert oder durch eine oder mehrere Gruppen, ausgewählt aus- $CH_3$, $-CF_3$, -COOH, $-NO_2$, -Cl und $NH_2$ substituiert sein können, und m3 gleich 0, 1, 2 oder 3 ist,

in Form eines reinen optischen Isomers oder einer Mischung optischer Isomere, sowie deren Salze, Solvate oder Hydrate.

**2.** Verbindungen vom Typ Azapeptid oder azapeptidomimetischen Typ nach Anspruch 1, **dadurch gekennzeichnet, daß** $R_2$ eine- L- $COOR'_2$- Gruppe darstellt, wobei L eine Aryl- Gruppe, vorzugsweise Phenyl- Gruppe, oder eine- (CH) $m_2$- Kette darstellt, wobei $m_2$ gleich 1, 2 oder 3 ist, und wobei $R'_2$ eine Alkyl- Gruppe mit 1 bis 12 Kohlenstoffatomen oder eine- $(CH_2)_{m'2}R''_2$- Gruppe darstellt, wobei $R''_2$ eine Aryl- oder Cycloalkyl- Gruppe, eventuell substituiert, darstellt und m'2 gleich 0, 1, 2 oder 3 ist.

**3.** Verbindungen vom Typ Azapeptid oder azapeptidomimetischen Typ nach Anspruch 1, der Formel (IA), in Form eines reinen Isomers oder einer Mischung von Isomeren, sowie deren Salze, Solvate oder Hydrate:

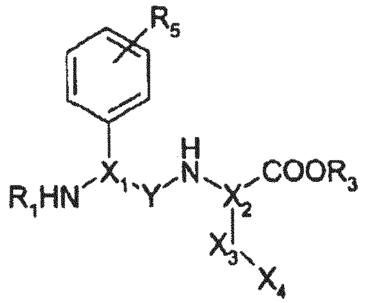

(IA)

worin $R_5$ eine Gruppe- $NH_2$, $-NO_2$, -OH, -O- Alkyl mit 1 bis 8 Kohlenstoffatomen, O- $(CH_2)_{m5}R'_5$ darstellt, wobei $R'_5$ eine Aryl- Gruppe darstellt und m5 gleich 0 oder 1 ist, oder aber $R_5$ eine- $COOR''_5$- Gruppe darstellt, wobei $R''_5$ eine Alkyl- Gruppe mit 1 bis 12 Kohlenstoffatomen oder eine- $(CH_2)_{m'5}R'''_5$- Gruppe darstellt, wobei $R'''_5$ eine Aryl- oder Cycloalkyl- Gruppe darstellt und m'5 gleich 0, 1, 2 oder 3 ist, wobei $R_1$, $X_1$, Y, $X_2$, $X_3$, $X_4$ und $R_3$ so wie in Anspruch 1 für (I) definiert sind.

4. Verbindungen vom Typ Azapeptid oder azapeptidomimetischen Typ der Formel (IA) nach Anspruch 3, **dadurch gekennzeichnet, daß** $R_5$ eine- $COOR''_5$- Gruppe darstellt, wobei $R''_5$ eine Ethyl-, Benzyl- oder- $CH[CH(CH_3)_2]_2$- Gruppe darstellt.

5. Verbindungen vom Typ Azapeptid oder azapeptidomimetischen Typ der Formel (IA) nach Anspruch 3 oder 4, **dadurch gekennzeichnet, daß** die $R_5$-Gruppe sich in meta-Stellung befindet.

6. Verbindungen vom Typ Azapeptid oder azapeptidomimetischen Typ der Formel (IA) nach Anspruch 3 oder 4, **dadurch gekennzeichnet, daß** die $R_5$-Gruppe sich in para-Stellung befindet.

7. Verbindungen vom Typ Azapeptid oder azapeptidomimetischen Typ der Formel (IA) nach einem der Ansprüche 3 bis 6, **dadurch gekennzeichnet, daß** $X_1$ -N- darstellt und $X_2$ -CH- darstellt.

8. Verbindungen vom Typ Azapeptid oder azapeptidomimetischen Typ der Formel (IA) nach einem der Ansprüche 3 bis 6, **dadurch gekennzeichnet, daß** $X_1$ und $X_2$ N darstellen.

9. Verbindungen vom Typ Azapeptid oder azapeptidomimetischen Typ der Formel (IA) nach einem der Ansprüche 3 bis 8, **dadurch gekennzeichnet, daß** Y- C (O)- darstellt.

10. Verbindungen vom Typ Azapeptid oder azapeptidomimetischen Typ der Formel (IA) nach einem der Ansprüche 3 bis 9, **dadurch gekennzeichnet, daß**- $X_3$- $X_4$ eine- $(CH_2)_n$- $NHR_4$- Gruppe darstellt, mit n gleich 4 oder 6 und $R_4$ so wie in Anspruch 1 für (I) definiert.

11. Verbindungen vom Typ Azapeptid oder azapeptidomimetischen Typ nach Anspruch 1, der Formel (IB), in Form eines reinen Isomers oder einer Mischung von Isomeren, sowie deren Salze, Solvate oder Hydrate:

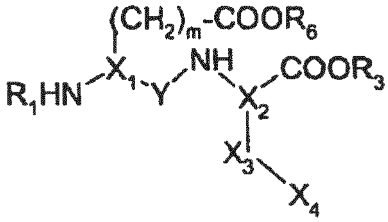

(IB)

worin m gleich 1, 2 oder 3 ist, $R_6$ eine Alkyl- Gruppe mit 1 bis 12 Kohlenstoffatomen darstellt, oder eine- $(CH_2)_{m6}R'_6$- Gruppe, wobei $R'_6$ eine Cycloalkyl- oder Aryl- Gruppe darstellt, wobei die Cycloalkyl- und Aryl- Gruppen unsubstituiert oder durch eine oder mehrere Gruppen, ausgewählt aus- $CH_3$, $-CF_3$, -COOH, $-NO_2$, -Cl und $NH_2$ substituiert sein können, und m6 gleich 0, 1, 2 oder 3 ist, wobei $R_1$, $X_1$, Y, $X_2$, $X_3$, $X_4$ und $R_3$ so wie in Anspruch 1 für (I) definiert sind.

12. Verbindungen vom Typ Azapeptid oder azapeptidomimetischen Typ der Formel (IB) nach Anspruch 11, **dadurch gekennzeichnet, daß** m gleich 1 oder 2 ist.

13. Verbindungen vom Typ Azapeptid oder azapeptidomimetischen Typ der Formel (IB) nach Anspruch 11 oder 12, **dadurch gekennzeichnet, daß** $R_6$ eine Ethyl-, Benzyl- oder- CH [CH $(CH_3)_2]_2$- Gruppe darstellt.

14. Verbindungen vom Typ Azapeptid oder azapeptidomimetischen Typ der Formel (IB) nach einem der Ansprüche 11 bis 13, **dadurch gekennzeichnet, daß** $X_1$ -CHdarstellt.

15. Verbindungen vom Typ Azapeptid oder azapeptidomimetischen Typ der Formel (IB) nach einem der Ansprüche 11 bis 14, **dadurch gekennzeichnet, daß** $X_2$ -N-darstellt.

16. Verbindungen vom Typ Azapeptid oder azapeptidomimetischen Typ der Formel (IB) nach einem der Ansprüche 11 bis 15, **dadurch gekennzeichnet, daß**- $X_3$- $X_4$ eine- $(CH_2)_n$- $NHR_4$- Gruppe darstellt, mit n gleich 6 und $R_4$ so wie in Anspruch 1 für (I) definiert.

17. Verbindungen vom Typ Azapeptid oder azapeptidomimetischen Typ der Formel (I), (IA) oder (IB) nach einem der Ansprüche 1 bis 16, **dadurch gekennzeichnet, daß** $R_1$ eine- $COOR'_1$- Gruppe darstellt und $R'_1$ eine tert- Butyl- oder Benzylgruppe darstellt.

18. Verbindungen vom Typ Azapeptid oder azapeptidomimetischen Typ der Formel (I), (IA) oder (IB) nach einem der Ansprüche 1 bis 17, **dadurch gekennzeichnet, daß** $R_3$ eine tert-Butyl- oder Benzylgruppe darstellt.

19. Verbindungen vom Typ Azapeptid oder azapeptidomimetischen Typ der Formel (I), (IA) oder (IB) nach einem der Ansprüche 1 bis 18, **dadurch gekennzeichnet, daß** $R_4$ $-COOR'_4$ darstellt, wobei $R'_4$ eine Benzyl- oder tert-Butylgruppe darstellt.

20. Verbindungen vom Typ Azapeptid oder azapeptidomimetischen Typ nach Anspruch 1, die ausgewählt sind aus:

- (2S)- 6- (Benzyloxycarbonylamino)- 2- [1- (2'- (benzyloxycarbonyl) phenyl)- 2- (tert- butoxycarbonyl)- hydrazinocarbonylamino]- hexansäure- tert- butylester (IA. 1)
- (2S)- 6- (Benzyloxycarbonylamino)- 2- [1- (3'- (benzyloxycarbonyl) phenyl)- 2- (tert- butoxycarbonyl)- hydrazinocarbonylamino]- hexansäure- tert- butylester (IA. 2)
- (2S)- 6- (Benzyloxycarbonylamino)- 2- [1- (4'- (benzyloxycarbonyl) phenyl)- 2- (tert- butoxycarbonyl)- hydrazinocarbonylamino]- hexansäure- tert- butylester (IA. 3)
- (2R)- 6- (Benzyloxycarbonylamino)- 2- [1- (3'- (benzyloxycarbonyl) phenyl)- 2- (tert- butoxycarbonyl)- hydrazinocarbonylamino]- hexansäure- benzylester (IA. 4)
- (2S)- 6- (Benzyloxycarbonylamino)- 2- [1- (3'- (benzyloxycarbonyl) phenyl)- 2- (tert- butoxycarbonyl)- hydrazinocarbonylamino]- hexansäure- benzylester (IA. 5)
- (2S)- 6- (Benzyloxycarbonylamino)- 2- [1- (3'- (phenyloxycarbonyl) phenyl)- 2- (tert- butoxycarbonyl)- hydrazinocarbonylamino]- hexansäure- tert- butylester (IA. 6)
- 2- [1- (3'- (Benzyloxycarbonyl) phenyl)- 2- (tert- butoxycarbonyl)- hydrazinocarbonyl]- 1- (6- benzyloxycarbonylamino- hexyl) hydrazincarbonsäure- benzylester (IA. 7)
- 2- [1- (3'- (Benzyloxycarbonyl) phenyl)- 2- (tert- butoxycarbonyl)- hydrazinocarbonyl]- 1- (6- tert- butyloxycarbonylamino- hexyl)- hydrazincarbonsäure- benzylester (IA. 8)
- (3S)- 4- [2- (6- Benzyloxycarbonylamino- hexyl)- 2- tert- butoxycarbonyl- hydrazino]- 3- tert- butoxycarbonylamino- buttersäure- benzylester (IB. 1)
- (3S)- 4- [2- (3- Benzyloxycarbonylamino- propyl)- 2- tert- butoxycarbonyl- hydrazino]- 3- tert- butoxycarbonylamino- 4- oxo- buttersäure- benzylester (IB. 2)
- (3S)- 4- [2- (4- Benzyloxycarbonylamino- phenyl)- 2- tert- butoxycarbonyl- hydrazino]- 3- tert- butoxycarbonylamino- 4- oxo- buttersäure- benzylester (IB. 3)
- (4S)- 5- [2- (4- Benzyloxycarbonylamino- phenyl)- 2- tert- butoxycarbonyl- hydrazino]- 4- tert- butoxycarbonylamino- 5- oxo- pentansäure- benzylester (IB. 4)
- (3S)- 4- [2- (5- Benzyloxycarbonylamino- pentyl)- 2- tert- butoxycarbonyl- hydrazino]- 3- tert- butoxycarbonylamino- 4- oxo- buttersäure- benzylester (IB. 5)
- 4- [2- ((2S)- 3- Benzyloxycarbonyl- 2- tert- butoxycarbonylamino- propyl)- 1- tert- butoxycarbonyl- hydrazinomethyl]- piperidin- 1- carbonsäure- benzylester (IB. 6)
- 4- [2- ((2S)- 3- Benzyloxycarbonyl- 2- tert- butoxycarbonylamino- propionyl)- 1- tert- butoxycarbonyl- hydrazinomethyl]- piperidin- 1- carbonsäure- benzylester (IB. 7)
- (3S)- 4- [2- (5- Benzyloxycarbonylamino- pentyl)- 2- tert- butoxycarbonyl- hydrazino]- 3- tert- butoxycarbonylamino- buttersäure- benzylester (IB. 8)
- (3S)- 4- [2- (6- Benzyloxycarbonylamino- hexyl)- 2- tert- butoxycarbonyl- hydrazino]- 3- tert- butoxycarbonylamino- 4- oxo- buttersäure- benzylester (IB. 9)
- (3S)- 3- tert- Butoxycarbonylamino- 4- [2- tert- butoxycarbonyl- 2- (6- tert- butoxycarbonylamino- hexyl)- hydrazino]- buttersäure- benzylester (IB. 10)
- (3S)- 4- [2- Benzyloxycarbonyl- 2- (6- benzyloxycarbonylamino- hexyl)- hydrazino]- 3- tert- butoxycarbonylamino- buttersäure- benzylester (IB. 11)
- (3S)- 3- Benzyloxycarbonylamino- 4- [2- benzyloxycarbonyl- 2- (6- benzyloxycarbonylamino- hexyl)- hydrazino]- buttersäure- benzylester (IB. 12)
- (3S)- 4- [2- Benzyloxycarbonyl- 2- (6- tert- butoxycarbonylamino- hexyl)- hydrazino]- 3- tert- butoxycarbonylamino- buttersäure- benzylester (IB. 13)
- (2S)- 6- (Benzyloxycarbonylamino)- 2- [1- (benzyloxycarbonylmethyl)- 2- (tert- butoxycarbonyl)- hydrazinocarbonylamino]- hexansäure- tert- butylester (IB. 14),

sowie ihren Salzen, Solvaten und/oder Hydraten, und insbesondere den pharmazeutisch akzeptablen.

**21.** Verbindung vom Typ Azapeptid oder azapeptidomimetischen Typ nach einem der vorhergehenden Ansprüche, als Zusatzmittel einer einem Patienten verabreichten therapeutischen Behandlung, für die der Organismus des behandelten Patienten eine Resistenz entwickelt, zur Wiederherstellung der Aktivität der therapeutischen Behandlung.

**22.** Verbindung vom Typ Azapeptid oder azapeptidomimetischen Typ nach einem der vorhergehenden Ansprüche, als Zusatzmittel eines Antitumor-Medikaments oder Antiinfektivums.

**23.** Verbindung vom Typ Azapeptid oder azapeptidomimetischen Typ nach einem der vorhergehenden Ansprüche, als Zusatzmittel eines Medikaments, ausgewählt aus den Anthracyclinen, den Topoisomerasehemmern, den Antimetaboliten (Antifolaten), den Tyrosinkinasehemmern, den antiviralen Medikamenten (Reverse- Transkriptase- Inhibitoren), Antiparasitika.

**24.** Verbindung vom Typ Azapeptid oder azapeptidomimetischen Typ nach einem der vorhergehenden Ansprüche, als Zusatzmittel eines Medikaments, ausgewählt aus Daunorubicin, Doxorubicin, Mitoxantron, Camptothecin und seinen Derivaten, Irinotecan, Topotecan, den Indolocarbazolen, Methotrexat, Imatinib, Gefitinib, Zidovudin, Lamivudin, Abacavir, Ivermectin, Albendazol, Oxfendazol, Ketoconazol.

## Claims

**1.** Compounds, of azapeptide or azapeptidomimetic type, of formula (I) :

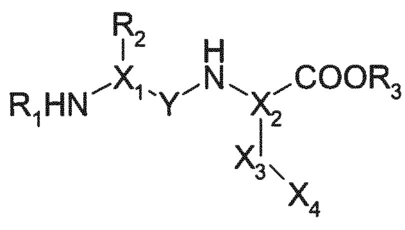

(I)

in which:

- $R_1$ represents a group- C (O) OR'$_1$ with R'$_1$ which represents an alkyl group of 1 to 12 carbon atoms or a group- $(CH_2)_{m1}R''_1$ with R"$_1$ which represents an aryl, cycloalkyl or fluorenyl group and m1 which is equal to 0, 1, 2 or 3,
- $X_1$ and $X_2$ are identical or different and represent -N- or -CH-, it being understood that at least one represents -N-,
- $R_2$ represents a side chain optionally in protected form of an amino acid or an amino acid analog, or an optionally substituted aryl group or an optionally substituted heteroaryl group,
- Y represents $-CH_2-$ or -C(O)-,

-- $X_3$- $X_4$ represents either a group- $(CH_2)_n$- $NHR_4$ with n equal to 3, 4, 5 or 6, or a group chosen from:

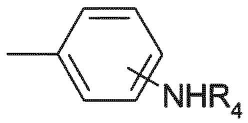

and

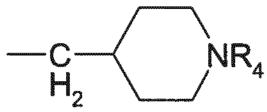

with $R_4$ which represents a protecting group for an amino group, preferably a group- $COOR'_4$ with $R'_4$ which represents an alkyl group of 1 to 12 carbon atoms or a group- $(CH_2)_{m4}R''_4$ with $R''_4$ which represents an aryl or cycloalkyl group and m4 which is equal to 0, 1, 2 or 3,

- $R_3$ represents an alkyl group of 1 to 12 carbon atoms, or a group- $(CH_2)_{m3}R''_3$ with $R''_3$ which represents a cycloalkyl or aryl group, said cycloalkyl and aryl groups possibly being unsubstituted or substituted with one or more groups chosen from:- $CH_3$, $-CF_3$, -COOH, $-NO_2$, -Cl and $NH_2$ and m3 which is equal to 0, 1, 2 or 3,

in the form of a pure optical isomer or a mixture of optical isomers, and also the salts, solvates or hydrates thereof.

2. Compounds of azapeptide or azapeptidomimetic type as claimed in claim 1, **characterized in that** $R_2$ represents a group- L- $COOR'_2$ with L which represents an aryl group, preferably phenyl, or a chain- $(CH_2)$ $m_2$- with $m_2$ which is equal to 1, 2 or 3 and $R'_2$ which represents an alkyl group of 1 to 12 carbon atoms or a group- $(CH_2)_{m'2}R''_2$ with $R''_2$ which represents an aryl group or cycloalkyl, optionally substituted and m'2 which is equal to 0, 1, 2 or 3.

3. Compounds of azapeptide or azapeptidomimetic type as claimed in claim 1, of formula (IA), in the form of a pure isomer or a mixture of isomers, and also the salts, solvates or hydrates thereof:

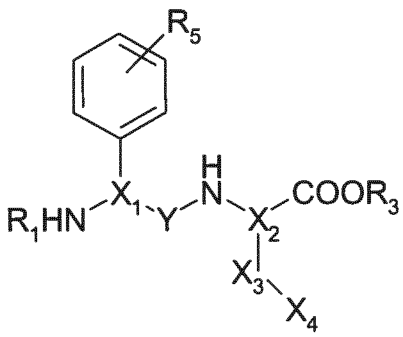

(IA)

in which $R_5$ represents a group- $NH_2$, $-NO_2$, -OH, -O- alkyl of 1 to 8 carbon atoms, O- $(CH_2)_{m5}R'_5$ with $R'_5$ which represents an aryl group and m5 which is equal to 0 or 1 or alternatively $R_5$ represents a group- $COOR''_5$ with $R''_5$ which represents an alkyl group of 1 to 12 carbon atoms or a group- $(CH_2)_{m'5}R'''_5$ with $R'''_5$ which represents an aryl or cycloalkyl group and m'5 which is equal to 0, 1, 2 or 3, $R_1$, $X_1$, Y, $X_2$, $X_3$, $X_4$ and $R_3$ being as defined for (I) in claim 1.

4. Compounds of azapeptide or azapeptidomimetic type of formula (IA) as claimed in claim 3, **characterized in that** $R_5$ represents a group- $COOR''_5$ with $R''_5$ which represents an ethyl, benzyl or- CH $[CH (CH_3)_2]_2$ group.

5. Compounds of azapeptide or azapeptidomimetic type of formula (IA) as claimed in claim 3 or 4, **characterized in that** the group $R_5$ is in the meta position.

6. Compounds of azapeptide or azapeptidomimetic type of formula (IA) as claimed in claim 3 or 4, **characterized in that** the group $R_5$ is in the para position.

7. Compounds of azapeptide or azapeptidomimetic type of formula (IA) as claimed in one of claims 3 to 6, **characterized in that** $X_1$ represents -N- and $X_2$ represents -CH-.

8. Compounds of azapeptide or azapeptidomimetic type of formula (IA) as claimed in one of claims 3 to 6, **characterized in that** $X_1$ and $X_2$ represent N.

**9.** Compounds of azapeptide or azapeptidomimetic type of formula (IA) as claimed in one of claims 3 to 8, **characterized in that** Y represents -C(O)-.

**10.** Compounds of azapeptide or azapeptidomimetic type of formula (IA) as claimed in one of claims 3 to 9, **characterized in that**- $X_3$- $X_4$ represents a group- $(CH_2)_n$- $NHR_4$ with n equal to 4 or 6 and $R_4$ as defined for (I) in claim 1.

**11.** Compounds of azapeptide or azapeptidomimetic type as claimed in claim 1 of formula (IB), in the form of a pure isomer or a mixture of isomers, and also the salts, solvates or hydrates thereof:

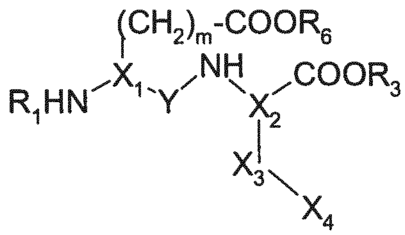

(IB)

in which m is equal to 1, 2 or 3, $R_6$ represents an alkyl group of 1 to 12 carbon atoms, or a group- $(CH_2)_{m6}R'_6$ with $R'_6$ which represents a cycloalkyl or aryl group, said cycloalkyl and aryl groups possibly being unsubstituted or substituted with one or more groups chosen from:- $CH_3$, -$CF_3$, -COOH, -$NO_2$, -Cl and $NH_2$ and m6 which is equal to 0, 1, 2 or 3, $R_1$, $X_1$, Y, $X_2$, $X_3$, $X_4$ and $R_3$ being as defined for (I) in claim 1.

**12.** Compounds of azapeptide or azapeptidomimetic type of formula (IB) as claimed in claim 11, **characterized in that** m is equal to 1 or 2.

**13.** Compounds of azapeptide or azapeptidomimetic type of formula (IB) as claimed in claim 11 or 12, **characterized in that** $R_6$ represents an ethyl, benzyl, or- CH [CH $(CH_3)_2]_2$ group.

**14.** Compounds of azapeptide or azapeptidomimetic type of formula (IB) as claimed in one of claims 11 to 13, **characterized in that** $X_1$ represents -CH-.

**15.** Compounds of azapeptide or azapeptidomimetic type of formula (IB) as claimed in one of claims 11 to 14, **characterized in that** $X_2$ represents -N-.

**16.** Compounds of azapeptide or azapeptidomimetic type of formula (IB) as claimed in one of claims 11 to 15, **characterized in that**- $X_3$- $X_4$ represents a group- $(CH_2)_n$- $NHR_4$ with n equal to 6 and $R_4$ as defined for (I) in claim 1.

**17.** Compounds of azapeptide or azapeptidomimetic type of formula (I), (IA) or (IB) as claimed in one of claims 1 to 16, **characterized in that** $R_1$ represents a group -COOR'$_1$ and R'$_1$ represents a tert-butyl or benzyl group.

**18.** Compounds of azapeptide or azapeptidomimetic type of formula (I), (IA) or (IB) as claimed in one of claims 1 to 17, **characterized in that** $R_3$ represents a *tert-butyl* or benzyl group.

**19.** Compounds of azapeptide or azapeptidomimetic type of formula (I), (IA) or (IB) as claimed in one of claims 1 to 18, **characterized in that** $R_4$ represents - COOR'$_4$ with R'$_4$ which represents a benzyl or tert-butyl group.

**20.** Compounds of azapeptide or azapeptidomimetic type as claimed in claim 1, chosen from:

- *tert*- butyl ester of (2S)- 6- (benzyloxycarbonylamino)- 2- [1- (2'- (benzyloxycarbonyl) phenyl)- 2- (*tert*- butoxycarbonyl) hydrazinocarbonylamino] hexanoic acid (IA. 1)
- *tert*- butyl ester of (2S)- 6- (benzyloxycarbonylamino)- 2- [1- (3'- (benzyloxycarbonyl) phenyl)- 2- (*tert*- butoxycarbonyl) hydrazinocarbonylamino] hexanoic acid (IA. 2)
- *tert*- butyl ester of (2S)- 6- (benzyloxycarbonylamino)- 2- [1- (4'- (benzyloxycarbonyl) phenyl)- 2- (*tert*- butoxycarbonyl) hydrazinocarbonylamino] hexanoic acid (IA. 3)
- benzyl ester of (2R)- 6- (benzyloxycarbonylamino)- 2- [1- (3'- (benzyloxycarbonyl) phenyl)- 2- (*tert*- butoxycarbonyl) hydrazinocarbonylamino] hexanoic acid (IA. 4)

- benzyl ester of (2S)- 6- (benzyloxycarbonylamino)- 2- [1- (3'- (benzyloxycarbonyl) phenyl)- 2- (*tert*- butoxycarbonyl) hydrazinocarbonylamino] hexanoic acid (IA. 5)
- *tert- butyl* ester of (2S)- 6- (benzyloxycarbonylamino)- 2- [1- (3'- (phenyloxycarbonyl) phenyl)- 2- (*tert*- butoxycarbonyl) hydrazinocarbonylamino]- hexanoic acid (IA. 6)
- benzyl ester of 2- [1- (3'- (benzyloxycarbonyl) phenyl)- 2- (*tert*- butoxycarbonyl) hydrazinocarbonyl]- 1- (6-benzyloxycarbonylaminohexyl)- hydrazinocarboxylic acid (IA. 7)
- benzyl ester of 2- [1- (3'- (benzyloxycarbonyl) phenyl)- 2- (*tert*- butoxycarbonyl) hydrazinocarbonyl]- 1- (6- *tert*-butyloxycarbonylaminohexyl)- hydrazinocarboxylic acid (IA. 8)
- benzyl ester of (3S)- 4- [2- (6- benzyloxycarbonylaminohexyl)- 2- *tert*- butoxycarbonylhydrazino]- 3- *tert*- butoxycarbonylaminobutyric acid (IB. 1)
- benzyl ester of (3S)- 4- [2- (3- benzyloxycarbonylaminopropyl)- 2- *tert*- butoxycarbonylhydrazino]- 3- *tert*-butoxycarbonylamino- 4- oxobutyric acid (IB. 2)
- benzyl ester of (3S)- 4- [2- (4- benzyloxycarbonylaminophenyl)- 2- *tert*- butoxycarbonylhydrazino]- 3- *tert*-butoxycarbonylamino- 4- oxobutyric acid (IB. 3)
- benzyl ester of (4S)- 5- [2- (4- benzyloxycarbonylaminophenyl)- 2- *tert*- butoxycarbonylhydrazino]- 4- *tert*-butoxycarbonylamino- 5- oxopentanoic acid (IB. 4)
- benzyl ester of (3S)- 4- [2- (5- benzyloxycarbonylaminopentyl)- 2- *tert*- butoxycarbonylhydrazino]- 3- *tert*-butoxycarbonylamino- 4- oxobutyric acid (IB. 5)
- benzyl ester of 4- [2- ((2S)- 3- benzyloxycarbonyl- 2- *tert*- butoxycarbonylaminopropyl)- 1- *tert*- butoxycarbonylhydrazinomethyl] piperidine- 1- carboxylic acid (IB. 6)
- benzyl ester of 4- [2- ((2S)- 3- benzyloxycarbonyl- 2- *tert*- butoxycarbonyl- aminopropionyl)- 1- *tert*- butoxycarbonylhydrazinomethyl] piperidine- 1- carboxylic acid (IB. 7)
- benzyl ester of (3S)- 4- [2- (5- benzyloxycarbonylaminopentyl)- 2- *tert*- butoxycarbonylhydrazino]- 3- *tert*-butoxycarbonylaminobutyric acid (IB. 8)
- benzyl ester of (3S)- 4- [2- (6- benzyloxycarbonylaminohexyl)- 2- *tert*- butoxycarbonylhydrazino]- 3- *tert*- butoxycarbonylamino- 4- oxobutyric acid (IB. 9)
- benzyl ester of (3S)- 3- *tert*- butoxycarbonylamino- 4- [2- *tert*- butoxycarbonyl- 2- (6- *tert*- butoxycarbonylaminohexyl) hydrazino] butyric acid (IB. 10)
- benzyl ester of (3S)- 4- [2- benzyloxycarbonyl- 2- (6- benzyloxycarbonyl- aminohexyl) hydrazino]- 3- *tert*-butoxycarbonylaminobutyric acid (IB. 11)
- benzyl ester of (3S)- 3- benzyloxycarbonylamino- 4- [2- benzyloxycarbonyl- 2- (6- benzyloxycarbonylaminohexyl) hydrazino] butyric acid (IB. 12)
- benzyl ester of (3S)- 4- [2- benzyloxycarbonyl- 2- (6- *tert*- butoxycarbonyl- aminohexyl) hydrazino]- 3- *tert*-butoxycarbonylaminobutyric acid (IB. 13)
- *tert*- butyl ester of (2S)- 6- (benzyloxycarbonylamino)- 2- [1- (benzyloxycarbonylmethyl)- 2- (*tert*- butoxycarbonyl) hydrazinocarbonylamino] hexanoic acid (IB. 14),

and also salts, solvates and/or hydrates thereof, and especially the pharmaceutically acceptable forms thereof.

**21.** The compound of azapeptide or azapeptidomimetic type as claimed in one of the preceding claims, as an adjuvant for a therapeutic treatment administered to a patient, for which the body of the treated patient develops a resistance, which is intended to restore the activity of the therapeutic treatment.

**22.** The compound of azapeptide or azapeptidomimetic type as claimed in one of the preceding claims, as an adjuvant for an anticancer or anti-infectious medicament.

**23.** The compound of azapeptide or azapeptidomimetic type as claimed in one of the preceding claims, as an adjuvant for a medicament chosen from anthracyclines, topoisomerase inhibitors, antimetabolic agents (antifolates), tyrosine kinase inhibitors, antiviral agents (reverse transcriptase inhibitors) and antiparasitic agents.

**24.** The compound of azapeptide or azapeptidomimetic type as claimed in one of the preceding claims, as an adjuvant for a medicament chosen from daunorubicin, doxorubicin, mitoxantrone, camptothecin and derivatives thereof, irinotecan, topotecan, indolocarbazoles, methotrexate, imatinib, gefitinib, zidovudine, lamivudine, abacavir, ivermectin, albendazole, oxfendazole and ketoconazole.

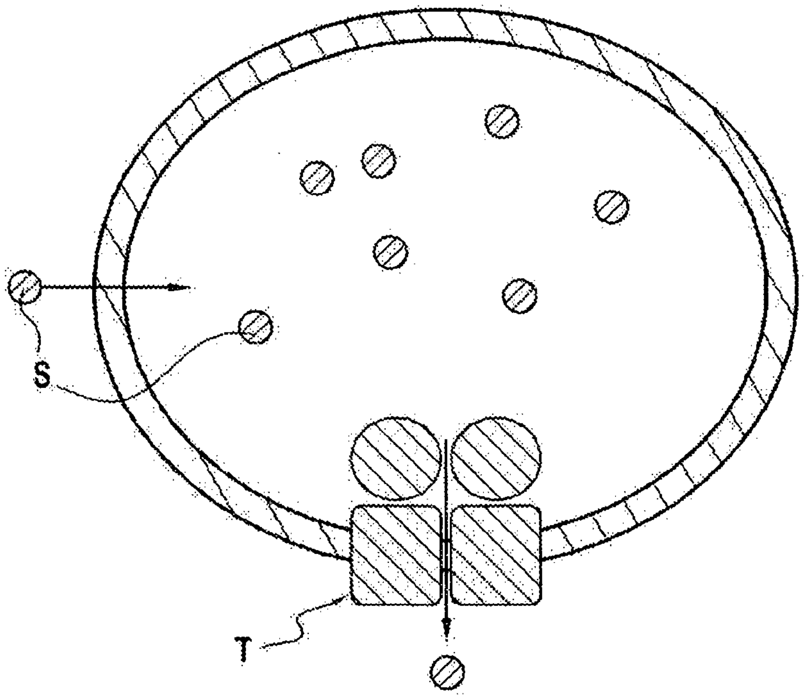


FIG.1

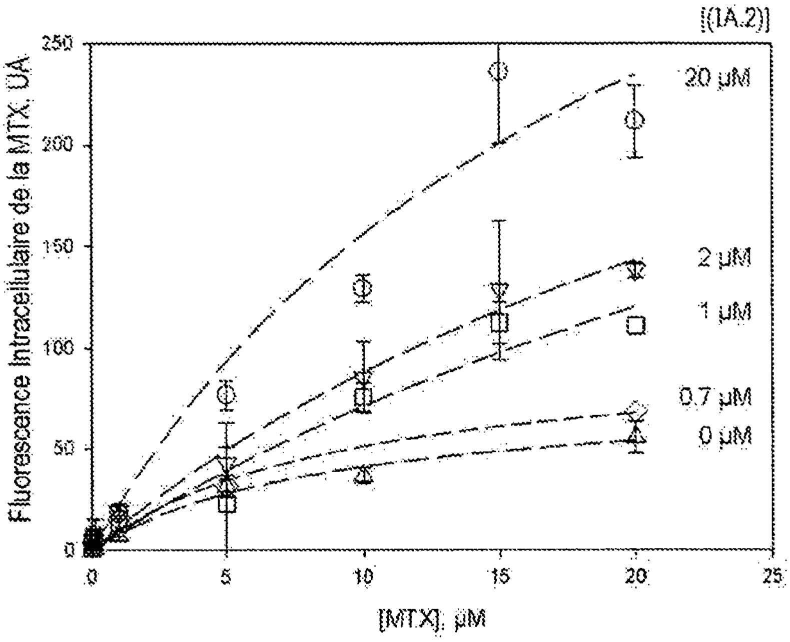
[(IA.2)]
20 µM
2 µM
1 µM
0,7 µM
0 µM
Fluorescence Intracellulaire de la MTX, UA
[MTX], µM
0
50
100
150
200
250
0
5
10
15
20
25

FIG.2A

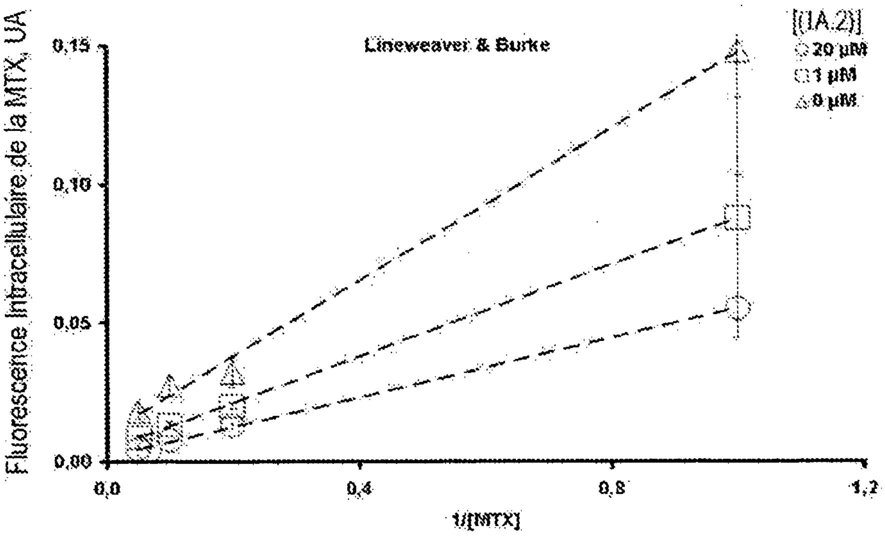
Lineweaver & Burke
[(IA.2)]
20 µM
1 µM
0 µM
Fluorescence Intracellulaire de la MTX, UA
1/[MTX]
0,00
0,05
0,10
0,15
0,0
0,4
0,8
1,2

FIG.2B

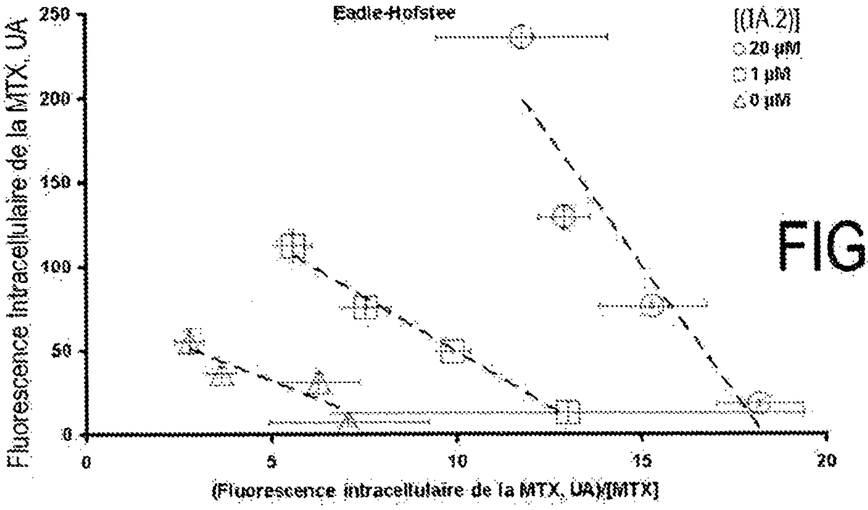


FIG.2C

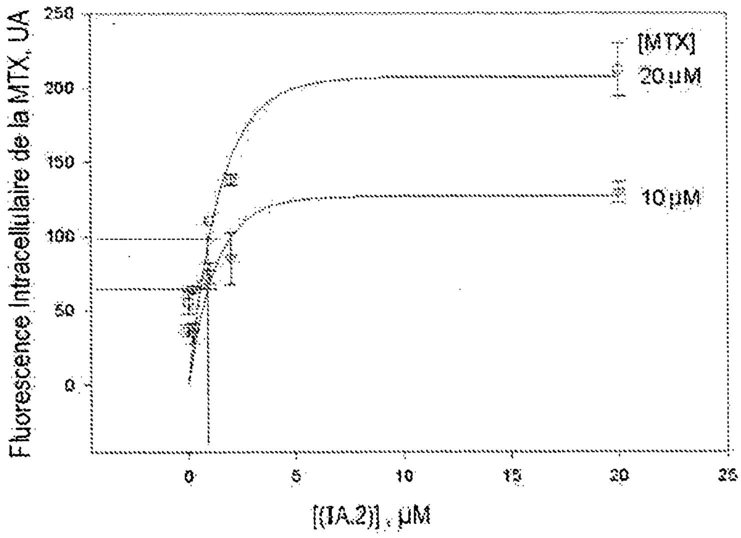


FIG.2D

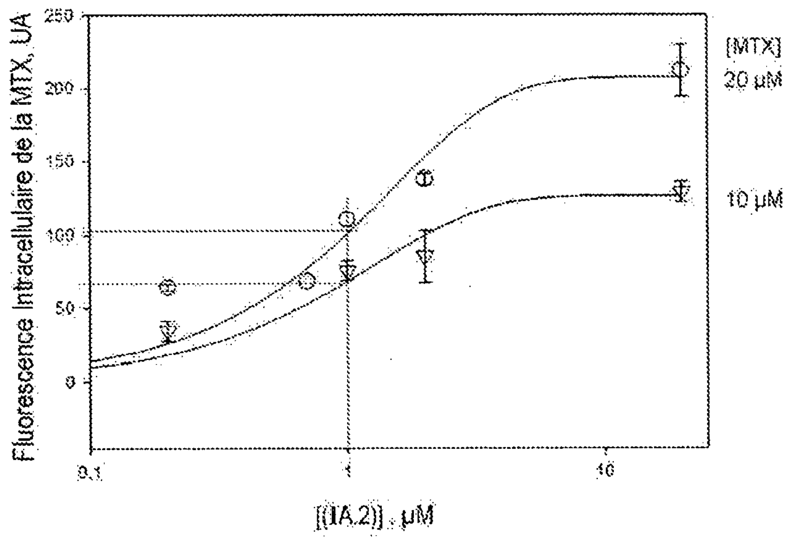


FIG.2E

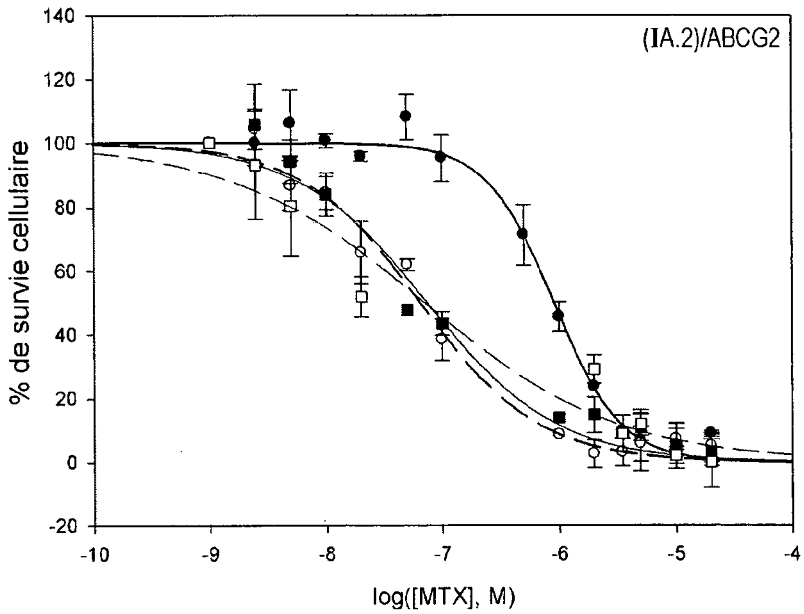
(IA.2)/ABCG2
% de survie cellulaire
log([MTX], M)
140
120
100
80
60
40
20
0
-20
-10
-9
-8
-7
-6
-5
-4

FIG.3

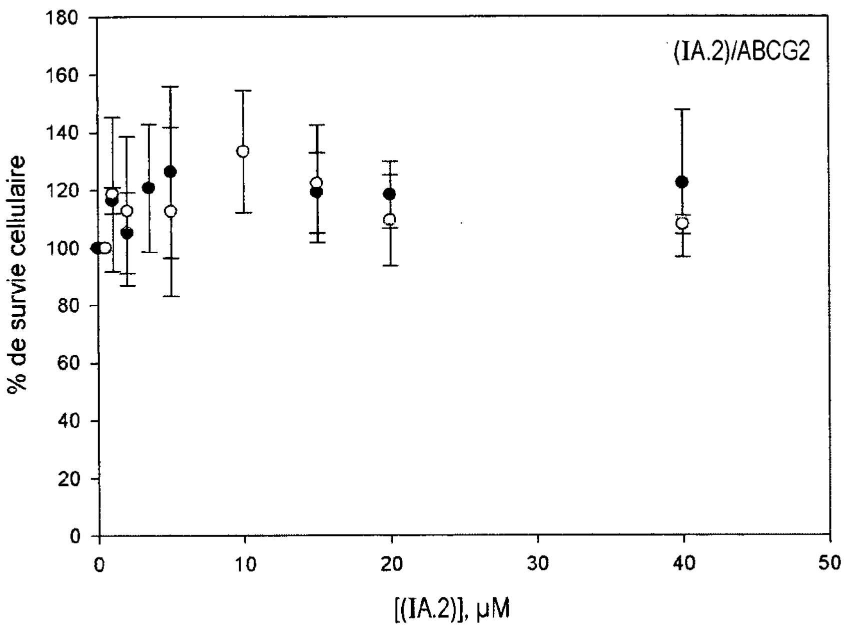
(IA.2)/ABCG2
% de survie cellulaire
[(IA.2)], μM
180
160
140
120
100
80
60
40
20
0
0
10
20
30
40
50

FIG.4

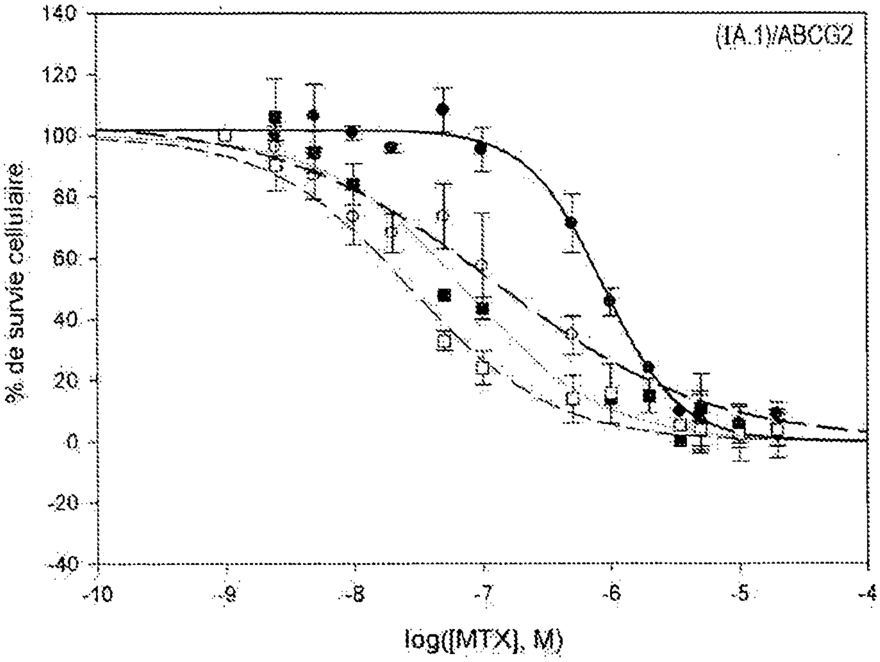
140
120
100
80
60
40
20
0
-20
-40
-10
-9
-8
-7
-6
-5
-4
% de survie cellulaire
log([MTX], M)
(IA.1)/ABCG2

FIG.5

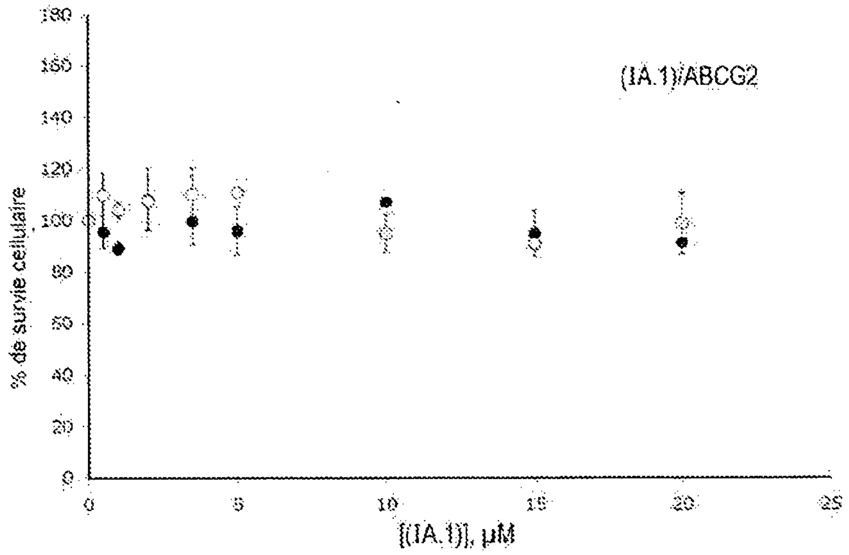
180
160
140
120
100
80
60
40
20
0
0
5
10
15
20
25
% de survie cellulaire
[(IA.1)], µM
(IA.1)/ABCG2

FIG.6

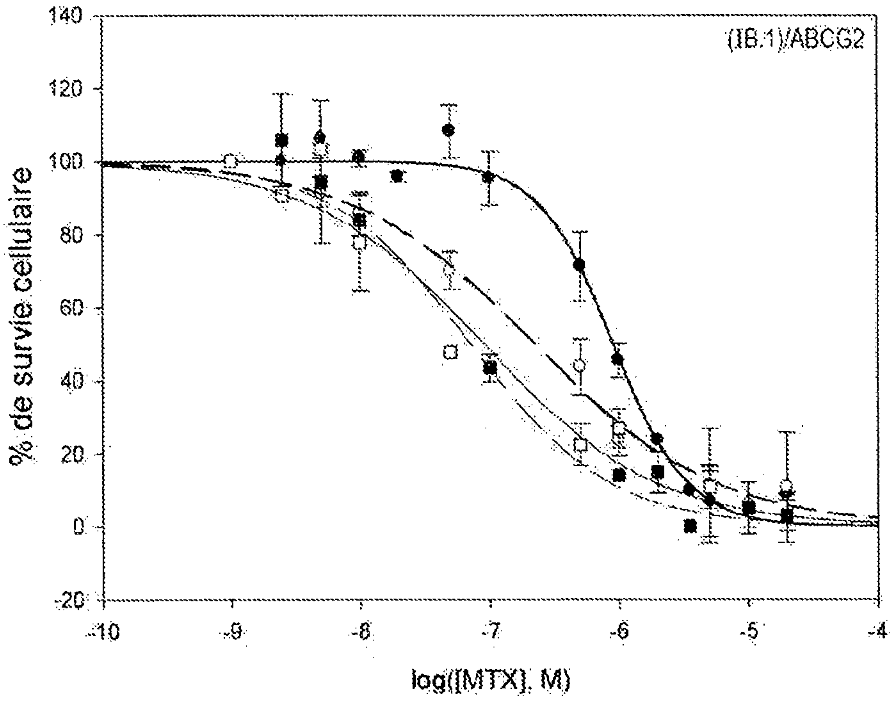
(IB.1)/ABCG2
% de survie cellulaire
log([MTX], M)
140
120
100
80
60
40
20
0
-20
-10
-9
-8
-7
-6
-5
-4

FIG.7

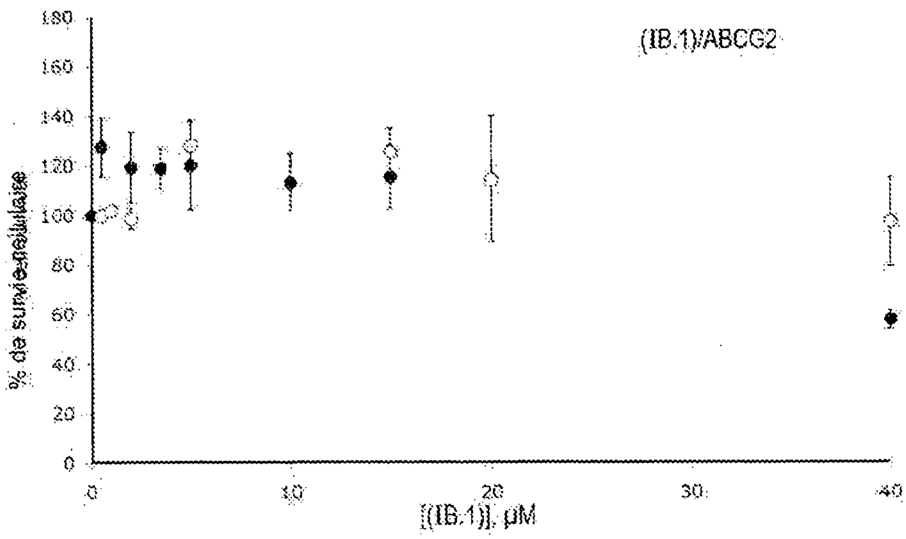
(IB.1)/ABCG2
% de survie cellulaire
[(IB.1)], μM
180
160
140
120
100
80
60
40
20
0
0
10
20
30
40

FIG.8

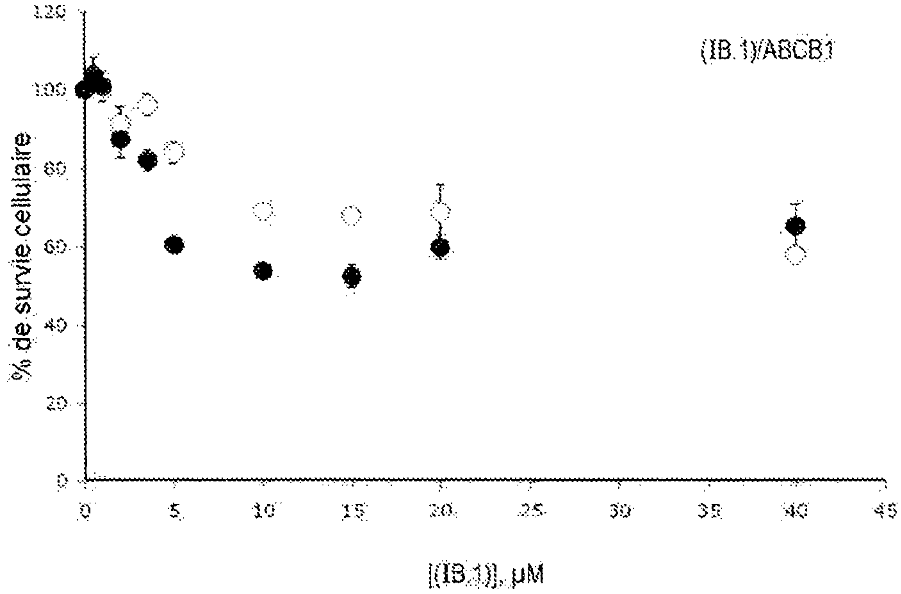
(IB.1)/ABCB1
% de survie cellulaire
120
100
80
60
40
20
0
0
5
10
15
20
25
30
35
40
45
[(IB.1)], µM

FIG.9

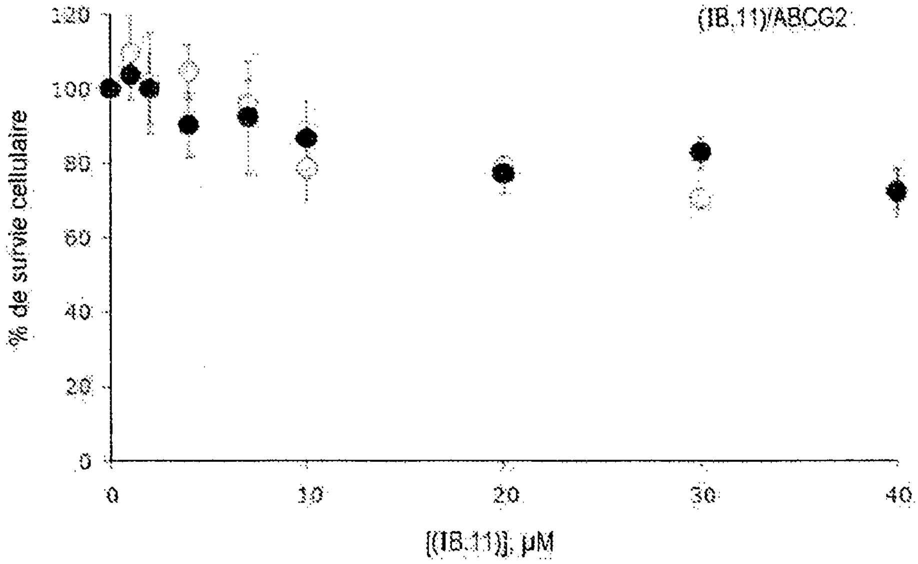
(IB.11)/ABCG2
% de survie cellulaire
120
100
80
60
40
20
0
0
10
20
30
40
[(IB.11)], µM

FIG.10

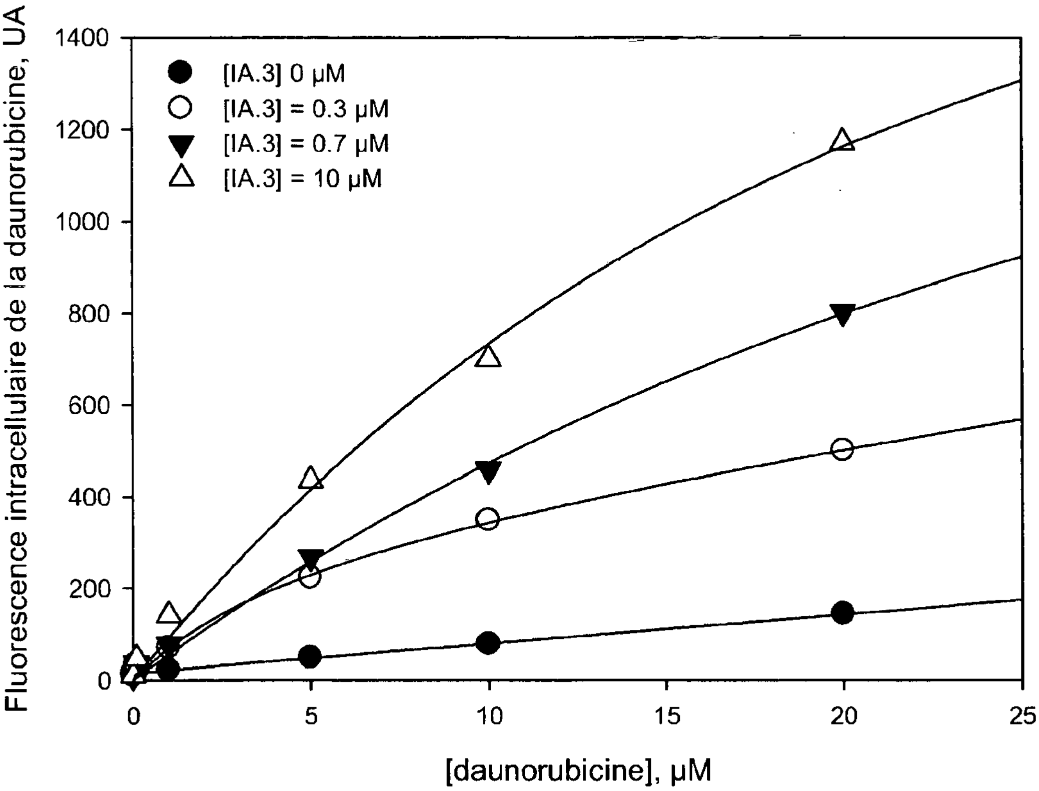
Fluorescence intracellulaire de la daunorubicine, UA
1400
1200
1000
800
600
400
200
0
[IA.3] 0 µM
[IA.3] = 0.3 µM
[IA.3] = 0.7 µM
[IA.3] = 10 µM
0
5
10
15
20
25
[daunorubicine], µM

FIG.11

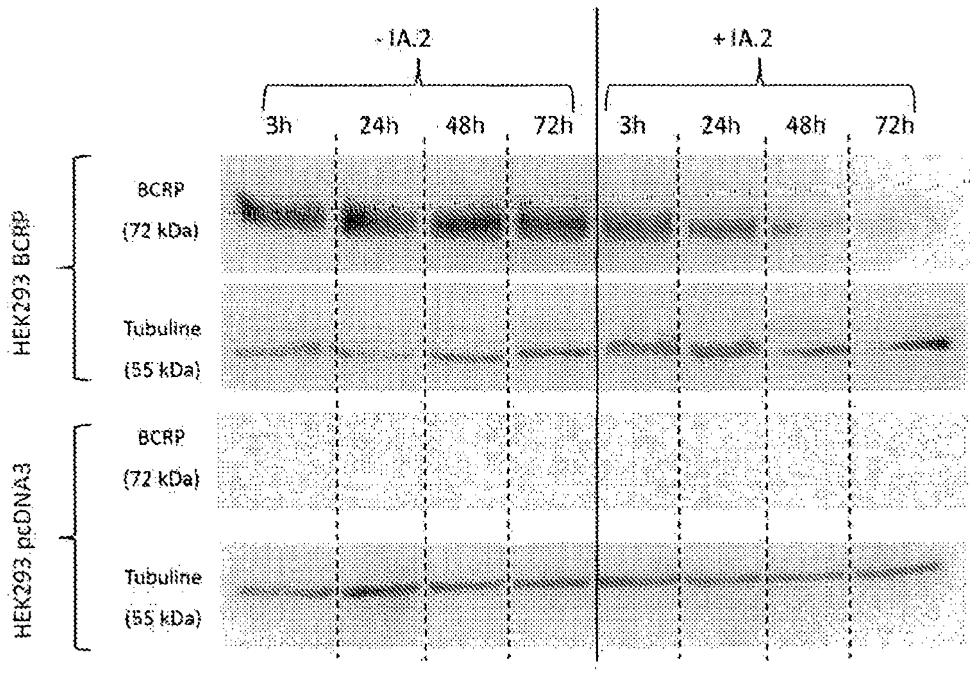
- IA.2
+ IA.2
3h
24h
48h
72h
3h
24h
48h
72h
HEK293 BCRP
BCRP
(72 kDa)
Tubuline
(55 kDa)
HEK293 pcDNA3
BCRP
(72 kDa)
Tubuline
(55 kDa)

FIG.12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2008086008 A


### Littérature non-brevet citée dans la description


- **JULIANO ; LING.** *Biochim Biophys Acta,* 1976, vol. 455, 152-162
- *Science,* 1992, vol. 258, 1650-1654
- **ROSS et al.** *J Natl Cancer Inst,* 1999, vol. 91, 429-433
- **BATES et al.** *J Cell Sci,* 2000, vol. 113, 2011-2021
- **DAWSON ; LOCHER.** *Nature,* 2006, vol. 443 (7108), 180-5
- **WARD et al.** *Proc. Natl. Acad. Sci. USA,* 2007, vol. 104 (48), 19005-10
- **KIM.** *Top HIV Med,* 2003, vol. 11, 136-139
- **WANG et al.** *Mol. Pharmacol.,* 2003, vol. 63, 65-72
- **WEISS et al.** *J. Antimicrob. Chemother.,* 2007, dkl474
- *Current Opinion in Pharmacology,* 2006, vol. 6, 350-354
- *Chem. Rev.,* 2009, vol. 109, 2455-2504
- Protective Groups in Organic Synthesis. John Wiley et Sons, 2006
- Protecting Groups. Georg Thieme Verlag, 1994
- *Journal of Peptide Science,* 1997, vol. 3, 429-441
- *J Med Chem,* 1996, vol. 39, 1172-1188
- **WANG.** *J. Org. Chem.,* 1977, vol. 42, 1286-1290
- **K. MEYER.** *Synlett,* 2004, 2355-2356
- **BUCHWALD.** *Org. Lett.,* 2001, vol. 3, 3803-3805
- **PATI.** *Synthetic communications,* 2004, vol. 34, 933-40
- *Tetrahedron,* 1989, vol. 45, 6319-6330
- *J Org Chem,* 2001, vol. 66, 2869-2873
- *Tetrahedron Asymmetry,* 2003, vol. 14, 139-143
- **Z. GUO et al.** *Bioorg. Med. Chem.,* 2001, vol. 9, 99-106
- **BOUILLON et al.** *Tetrahedron,* 2007, vol. 63, 223-2234
- **KOCIENSKI P.J.** Protecting Groups. Georg Thieme Verlag, 1994
- **ROBEY, R. W. ; HONJO, Y. ; MORISAKI, K. ; NADJEM, T. A. ; RUNGE, S. ; RISBOOD, M. ; PORUCHYNSKY, M. S. ; BATES, S. E.** *Br. J. Cancer,* 2003, vol. 89 (10), 1971-1978
- **CHANG ; XIU-BAO ; HOU ; YUE-XIAN ; RIORDAN ; JOHN R.** *J. Biol. Chem.,* 1997, vol. 272 (49), 30962-30968
- **CARDARELLI ; CAROL O. ; AKSENTIJEVICH ; IVAN ; PASTAN ; IRA ; GOTTESMAN ; MICHAEL M.** *Cancer Res,* 1995, vol. 55, 1086-1091
- **ROBEY, R. W. ; HONJO, Y. ; MORISAKI, K. ; ADJEM, T. A. ; RUNGE, S. ; RISBOOD, M. ; PORUCHYNSKY, M. S. ; BATES, S. E.** *Br. J. Cancer,* 2003, vol. 89 (10), 1971-1978
- **CHANG ; XIU-BAO, HOU ; YUE-XIAN ; RIORDAN ; JOHN R.** *J. Biol. Chem.,* 1997, vol. 272 (49), 30962-30968
- **U. K. LAEMMLI.** *Nature,* 1970, vol. 227, 680